(19) Europäisches Patentamt
European Patent Office
Office européen des brevets

(11) Publication number: **0 352 909**
**A2**

(12) # EUROPEAN PATENT APPLICATION

(21) Application number: **89306483.2**

(22) Date of filing: **26.06.89**

(51) Int. Cl.[4]: **C07D 413/14 , A61K 31/42 , C07D 405/06**

Claims for the following Contracting State: ES.

The title of the invention has been amended (Guidelines for Examination in the EPO, A-III, 7.3).

(30) Priority: **30.06.88 GB 8815626**

(43) Date of publication of application: **31.01.90 Bulletin 90/05**

(84) Designated Contracting States: **AT BE CH DE ES FR GB GR IT LI LU NL SE**

(71) Applicant: **BEECHAM GROUP PLC**
**Beecham House Great West Road**
**Brentford Middlesex TW8 9BD(GB)**

(72) Inventor: **O'Hanlon, Peter John**
**Beecham Pharmaceuticals Brockham Park**
**Betchworth Surrey RH3 7AJ(GB)**
Inventor: **Pearson, Neil David**
**Beecham Pharmaceuticals Brockham Park**
**Betchworth Surrey RH3 7AJ(GB)**

(74) Representative: **Dayneswood, Trevor et al**
**Beecham Pharmaceuticals Great Burgh Yew Tree Bottom Road**
**Epsom Surrey KT18 5XQ(GB)**

(54) **Monic acid derivatives.**

(57) Compounds of formula (I):

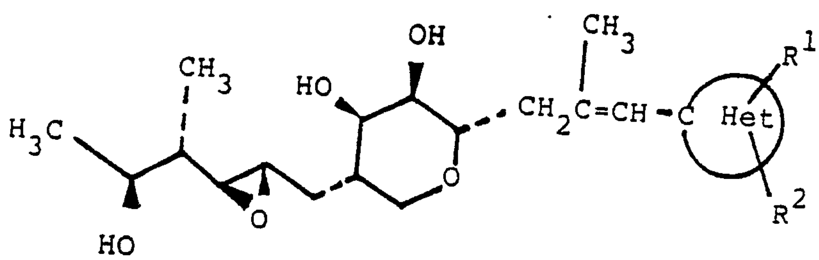

(I)

wherein

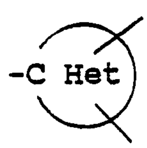

is a divalent or a trivalent, 5-membered heterocyclic group having a 6-$\pi$ electron system, and containing from 1 to 4 heteroatoms, each selected from oxygen, nitrogen and sulphur;
$R^1$ is a substituted $C_{3-7}$ cycloalkyl group attached to a carbon or nitrogen of

EP 0 352 909 A2

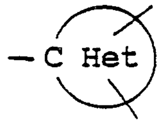 ;

and where appropriate, $R^2$ is a group attached to a carbon or nitrogen of

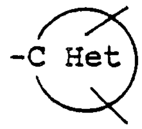 ,

and, when present, is selected from optionally substituted $C_{1-20}$ alkyl, optionally substituted $C_{3-7}$ cycloalkyl, optionally substituted $C_{2-8}$ alkenyl, aryl, aralkyl, heterocyclyl and hydrogen;
and where the trisubstituted carbon-carbon double bond preferably has the E- configuration;
have anti-bacterial activity.

## NOVEL COMPOUNDS

The present invention relates to novel derivatives of monic and isomonic acids having antibacterial activity, to processes for their preparation and to their use in human and veterinary medicine.

EP-A-0 087 953 (Beecham Group plc ) and

EP-A-0 123 378 (Beecham Group plc ) disclose compounds of the general formula (A):

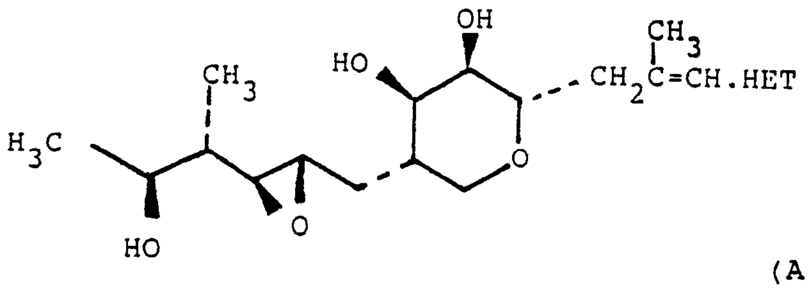

(A)

wherein HET is an optionally substituted 5-membered heteroaryl ring containing from 1 to 4 heteroatoms, each selected from oxygen, nitrogen and sulphur; with suitable substituents for HET including $C_{1-20}$ alkyl, $C_{2-8}$ alkenyl, each of which may optionally be substituted; aryl; aralkyl; heterocyclyl; and $C_{3-7}$ cycloalkyl.

These compounds are derivatives of monic or isomonic acid, the carboxylic acid group having been replaced by HET, and have antibacterial and antimycoplasmal activity.

Surprisingly, it has now been found that there exists another class of substituents for HET, giving further derivatives of monic and isomonic acids which have enhanced biological activity.

Accordingly, the present invention provides a compound of formula (I):

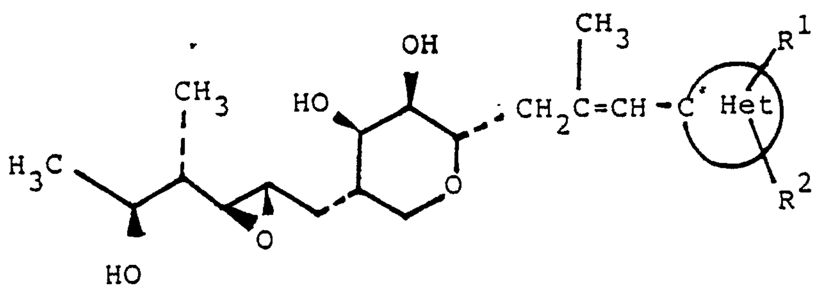

(I)

wherein

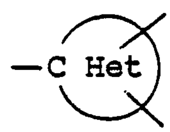

is a divalent or a trivalent, 5-membered heterocyclic group having a 6-$\pi$ electron system, and containing from 1 to 4 heteroatoms, each selected from oxygen, nitrogen and sulphur;

$R^1$ is a substituted $C_{3-7}$ cycloalkyl group attached to a carbon or nitrogen of

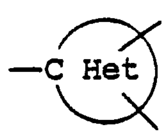 ;

and, where appropriate, $R^2$ is a group attached to a carbon or nitrogen of

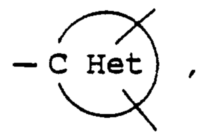

and, when present is selected from optionally substituted $C_{1-20}$ alkyl, optionally substituted $C_{3-7}$ cycloalkyl, optionally substituted $C_{2-8}$ alkenyl, aryl, aralkyl, heterocyclyl and hydrogen.

When used herein the term 'aryl' includes phenyl and naphthyl optionally substituted with up to five, preferably up to three, groups selected from halogen, cyano, $C_{1-6}$ alkyl, phenyl, $C_{1-6}$ alkoxy, halo($C_{1-6}$) alkyl, hydroxy, amino, mono- or di-($C_{1-6}$)alkylamino, $C_{1-6}$ acylamino, nitro, carboxy, $C_{1-6}$ alkanoyl, $C_{1-6}$ alkoxycarbonyl, $C_{1-6}$ alkoxycarbonyl ($C_{1-6}$)alkyl, $C_{1-6}$ alkylcarbonyloxy, $C_{1-6}$ alkylthio, $C_{1-6}$ alkanesulphinyl, $C_{1-6}$ alkanesulphonyl, sulphamoyl, mono- and di-($C_{1-6}$)alkylsulphamoyl, carbamoyl and mono- and di-($C_{1-6}$)alkylcarbamoyl.

When used herein, the term 'aralkyl' includes groups wherein the aryl moiety is a phenyl group which may be optionally substituted as herein before defined for aryl and wherein the alkylene radical has from 1 to 4. carbon atoms and may be optionally substituted.

When used herein, the term 'heterocyclyl' includes single or fused rings comprising up to four hetero atoms in the ring selected from oxygen, nitrogen and sulphur and optionally substituted with up to three groups selected from halogen, $C_{1-6}$ alkyl, $C_{1-6}$ alkoxy, halo ($C_{1-6}$) alkyl, hydroxy, amino, mono- or di-($C_{1-6}$)alkylamino, carboxy, $C_{1-6}$ alkoxycarbcnyl, $C_{1-6}$ alkoxycarbonyl($C_{1-6}$) alkyl, aryl or oxo.

Suitably the heterocyclic ring comprises from 3 to 7 ring atoms, preferably 5 to 6 atoms.

Suitable substituents for an alkyl, cycloalkyl and alkenyl group and alkylene radical include halogen, cyano, azido, nitro, carboxy, $C_{1-6}$ alkoxycarbonyl, carbamoyl, mono- or di-($C_{1-6}$)alkylcarbamoyl, sulphono, sulphamoyl, mono- and di-($C_{1-6}$)alkylsulphamoyl, amino, mono- and di-($C_{1-6}$)alkylamino, $C_{1-6}$ acylamino, ureido, $C_{1-6}$ alkoxycarbonylamino, 2,2,2-trichloroethoxycarbonylamino, aryl, heterocyclyl, hydroxy, $C_{1-6}$ alkoxy, $C_{1-6}$ acyloxy, oxo, aroyl, heterocycloyl, 2-thenoyl, $C_{1-6}$ alkylthio, $C_{1-6}$ alkanesulphinyl, $C_{1-6}$ alkanesulphonyl, hydroxyimino, alkoxyimino, hydrazino, hydrazono, benzohydroximoyl, 2-thiophenecarbohydroximoyl, guanidino, amidino, and iminoalkylamino.

Suitable substituents for a $C_{3-7}$ cycloalkyl group also include $C_{1-20}$ alkyl, $C_{2-8}$ alkenyl and $C_{2-8}$ alkynyl, each of which may be optionally substituted with a substituent suitable for an alkyl, cycloalkyl and alkenyl group and alkylene radical, as hereinbefore defined.

When the alkyl, cycloalkyl or alkenyl group or alkylene radical has more than one substituent, these may be the same or different.

Preferably $R^1$ has two substituents, which may be the same or different. Advantageously $R^1$ has a single substituent.

Compounds of formula (I) have a tri-substituted carbon-carbon double bond and this may be in either the E configuration [formula (I-E)]:

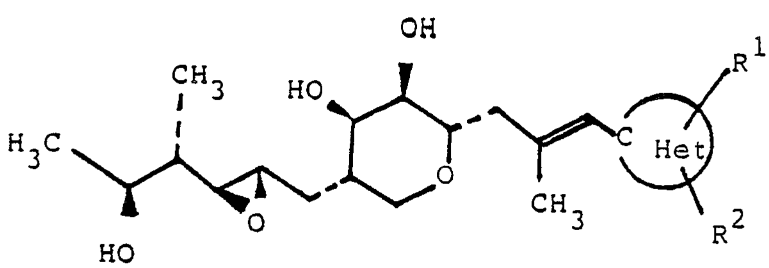

(I-E)

wherein

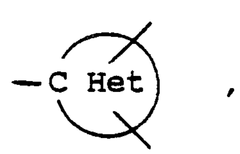

$R^1$ and $R^2$ are as hereinbefore defined with respect to formula (I);

or the Z configuration [formula (I-Z)]:

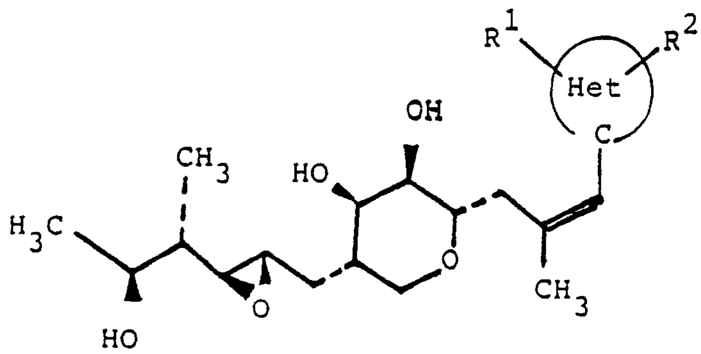

(I-Z)

wherein

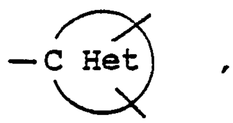

,

$R^1$ and $R^2$ are as hereinbefore defined with respect to formula (I); giving rise to two geometrically isomeric forms. The present invention encompasses both such isomers individually and admixed in any proportions. In general, greater biological activity is associated with the E isomer and for this reason the E isomer is preferred.

Compounds of formula (I-E) have been named '1-normon-2-yl-heterocycles'. The absolute stereochemistry of the 1-normon-2-yl radical is as shown in formula (I-E).

It will be appreciated that in compounds of formula (I), $R^1$ and/or $R^2$ may contain one or more chiral centres. The present invention encompasses all such resultant isomeric possibilities.

It will also be appreciated that

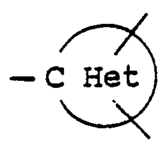

represents a variety of related heterocyclic systems including pyrroles, furans and thiophenes; oxazoles, thiazoles, isoxazoles, isothiazoles and diazoles; oxadiazoles, thiadiazoles and triazoles; and tetrazoles and that for the tetrazoles, oxa- and thiadiazoles, it is not possible for there to be a group $R^2$.

Preferably the moiety (X):

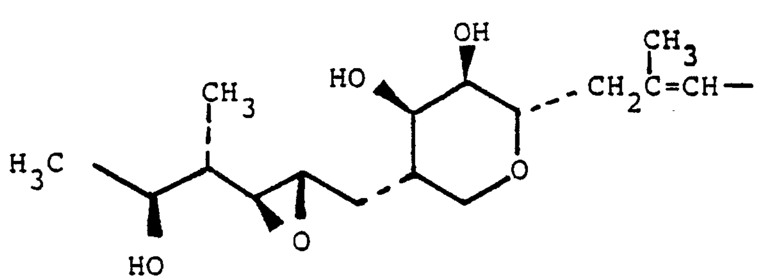

(X)

is bonded to a carbon atom which itself is bonded to a heteroatom of

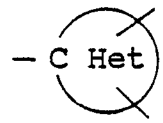 .

Preferably the heteroatom is nitrogen. More preferably the carbon atom is bonded to two heteroatoms, one of which is nitrogen.

Preferably R' is bonded to an atom located in the β-position in relation to the carbon atom of

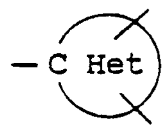

to which the moiety (X) is bonded.

Advantageously

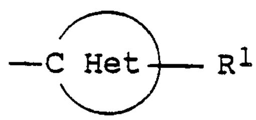

is a substituted oxazolyl moiety, especially a 1,3 oxazol-2-yl moiety.

A class of compounds within formula (I) comprises compounds of formula (IA):

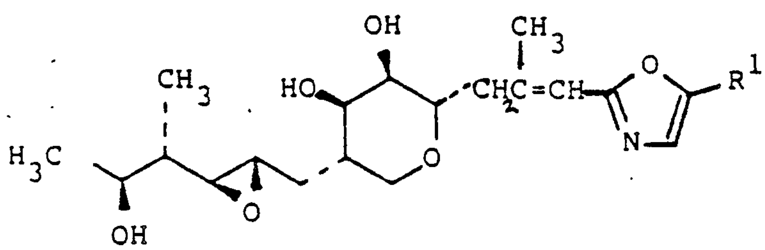

(IA)

wherein R' is as defined in relation to compounds of formula (I); of which a preferred subgroup are compounds of formula (IB):

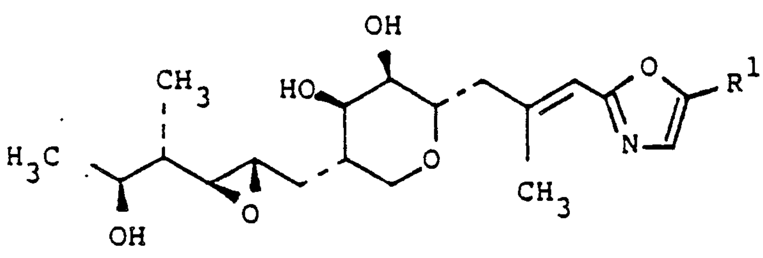

(IB)

wherein R' is as defined in relation to compounds of formula (I).

Compounds of formula (I) can be prepared by analogy with the methods described in EP-A-0 087 953 and EP-A-0 123 378.

In particular, the present invention provides a process for producing a compound of formula (I) which process comprises reacting a compound of formula (II):

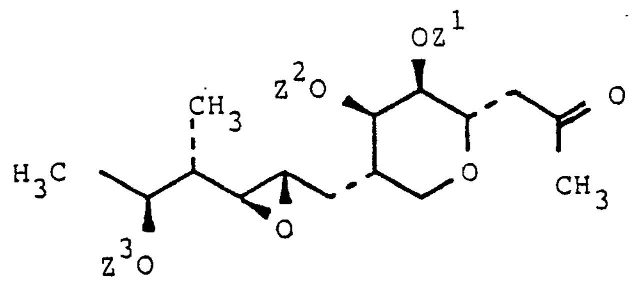

(II)

wherein $Z^1$, $Z^2$ and $Z^3$ are the same or different and each is hydrogen or a hydroxyl-protecting group, with a compound of formula (III):

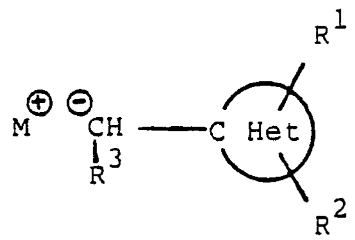

(III)

wherein:

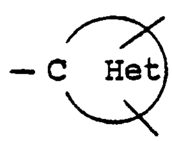

,

$R^1$ and $R^2$ are as defined in relation to formula (I); $M^+$ is a metal cation, preferably an alkali metal cation, most preferably a lithium or sodium cation, and $R^3$ is an anion-stabilising group, preferably a trialkylsilyl or a dialkylphosphonate group, most preferably trimethylsilyl or diethylphosphonate, which group will spontaneously eliminate with a $\beta$-hydroxyl group to produce an olefin, and, where necessary, removing any hydroxyl-protecting groups, and, if desired, converting one compound of formula (I) into a further compound of formula (I).

When used herein, the term 'hydroxyl-protecting group' refers to any such group known in the art which may be removed without disruption of the remainder of the molecule. Suitable hydroxyl protecting groups include those described in 'Protective Groups in Organic Synthesis', T.W. Greene, Wiley-Interscience, New York, 1981.

The reaction of a compound of formula (II) with a compound of formula (III) may conveniently be effected in an organic solvent, such as tetrahydrofuran, diethyl ether or dimethyl sulphoxide, at from reduced to elevated temperature, such as from -80° to 100°C.

The compound of formula (II) wherein $Z^1$, $Z^2$ and $Z^3$ are hydrogen, and processes for its production, are described in GB 1 587 060 (Beecham Group Ltd.). Derivatives thereof wherein $Z^1$, $Z^2$ and $Z^3$ are hydroxyl-protecting groups may be produced by conventional methods such as those mentioned below. When this compound is produced with hydroxyl-protecting groups already in place it may be used directly or even *in situ* in the above reaction or it may be optionally deprotected and/or isolated.

The compounds of formula (III) may be produced by conventional processes such as described in EP-A-0 123 378 (Beecham Group plc) and shown in Scheme I.

Scheme I

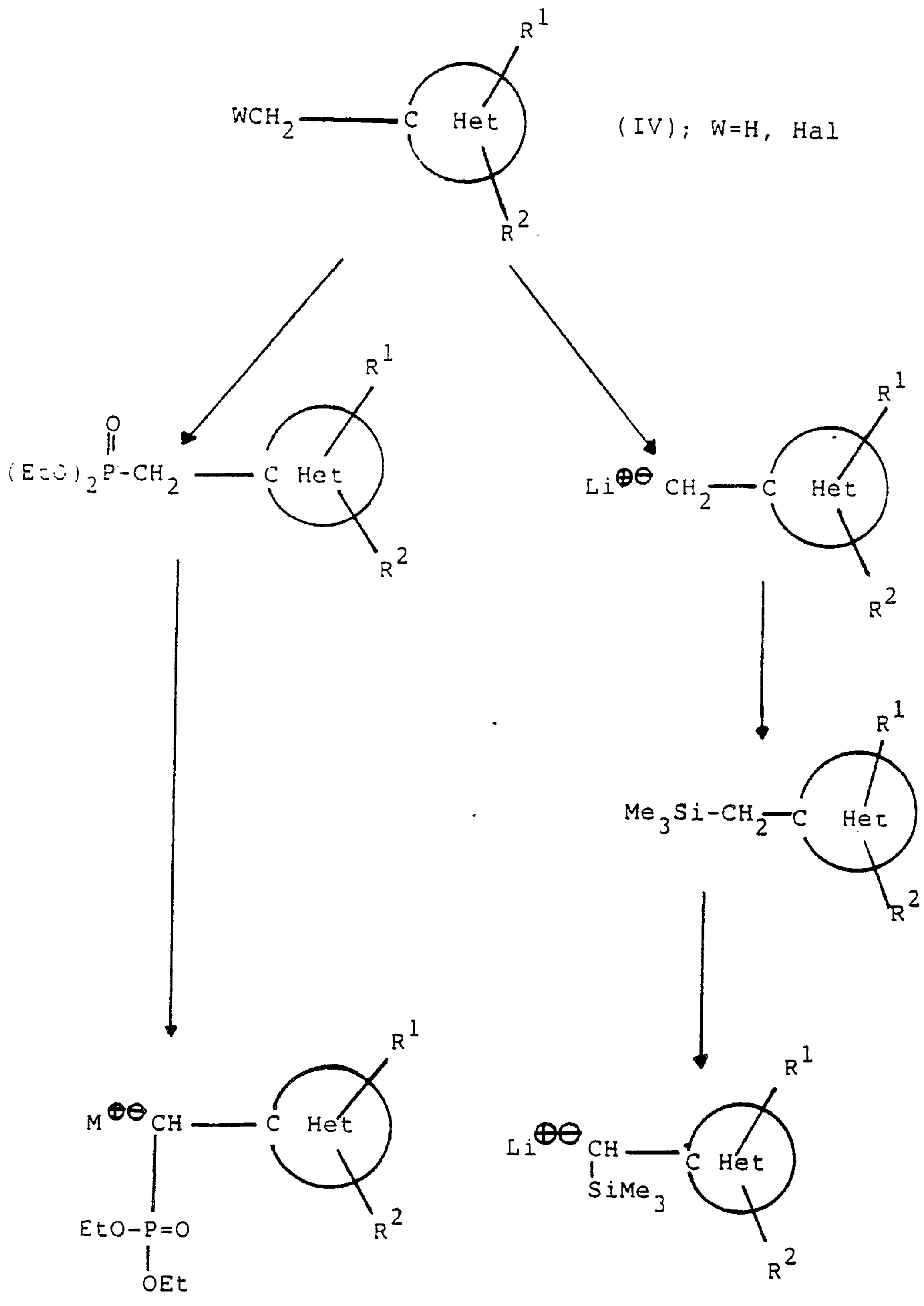

(IV); W=H, Hal

(IIIa); $R^3$ = $PO(OEt)_2$    (IIIb); M=Li, $R^3$=$SiMe_3$

The starting materials utilised in Scheme I, the compounds of formula (IV), are either well known and readily available or may be produced by analogy with conventional processes, for instance as described in 'Comprehensive Heterocyclic Chemistry', ed. Katritzky and Rees, 4, Chapter 3.03; 5, Chapter 4.03 and 6.

More particularly, methods are described for the preparation of (a) oxazoles by R.A. Jeffreys, J. Chem.

Soc., 1952, 4823; J.W. and R.H. Cornforth, J. Chem. Soc., 1947, 96 and in 'Heterocyclic Compounds', Vol. 5, Chapter 5, ed. Elderfield; (b) thiadiazoles in 'Heterocyclic Compounds', vol 7, Chapter 6, ed. Elderfield and (c) oxadiazoles by C. Ainsworth, J. Amer. Chem. Soc. 1955, 77, 1148.

Suitable reaction conditions for the preparation of compounds of general formula (IIIa) and (IIIb) are described by W.S. Wadsworth Jr, Organic Reactions, 1977, 25, 73 and by E.J. Corey and D.L. Boger, Tet. Letters, 1978, 5; T.H. Chan,, Acc. Chem. Res. 1977, 10, 442 and B.H. Lipshutz and K.W. Hungate, J. Org. Chem., 1981, 46, 1410; respectively. When

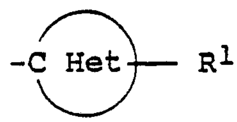

is a substituted 1,3-oxazol-2-yl moiety, the corresponding compounds of formula (IV) may be produced from compounds of formula (V):

$R^1COCH_2\hat{N}H_3Cl^-$ (V)

wherein $R^1$ is as defined in relation to formula (I), for instance by methodology described by J.L. La Mattina, J. Org. Chem., 1980, 45, 2261.

Compounds of formula (V) are prepared by analogy with methods described in Organic Synthesis, Coll. Vol. V, 909, using as starting materials compounds which are either well known and readily available or which may be produced from such well known and readily available starting materials by conventional manipulation thereof.

It will be appreciated that the reaction of a compound of formula (II) with a compound of formula (III) may produce a mixture of the E- and Z- isomeric products, the compounds of formulae (I-E) and (I-Z) respectively. These isomers may be separated by conventional procedures such as chromatography and/or fractional crystallisation.

The present invention also provides a process for producing a compound of formula (IA) which process comprises cyclising a compound of formula (VI):

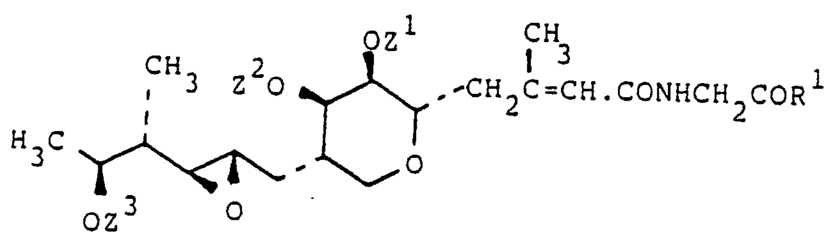

(VI)

wherein:

$R^1$ is as defined in relation to formula (I); and $Z^1$, $Z^2$ and $Z^3$ are the same or different and each is hydrogen or a hydroxyl-protecting group;

to form a compound of formula (IA) and, where necessary, removing any hydroxyl-protecting groups, and, if desired, converting one compound of formula (IA) into a further compound of formula (IA).

The cyclisation of a compound of formula (VI) is suitably effected using a chlorinating agent such as phosphorus oxychloride, phosgene, thionyl chloride or phosphorus pentachloride in the presence of a tertiary amine, such as triethylamine or pyridine. Such reactions are conveniently effected in an organic solvent, for instance dichloromethane or tetrahydrofuran, at from reduced to elevated temperature, for instance -80° to 100°C, over a period of several hours to a few days. Preferably phosgene or phosphorus oxychloride is used, at temperatures of from 0° to 20°C.

Alternatively, cyclisation may be effected using triphenylphosphine and carbon tetrachloride as the chlorinating reagent, in the presence of a tertiary amine, for instance triethylamine, in an inert solvent such as acetonitrile or acetonitrile-pyridine. This type of process is described by H. Vorbruggen and K. Krolikiewicz in Tet. Letters., 1981, 4471: it is particularly advantageous in that the production of compounds of formula (VI) and cyclisation of these to compounds of formula (IA) may be conducted in situ.

Compounds of formula (VI) may also be cyclised using a carboxylic anhydride or mixed anhydride or acid chloride, such as trifluoroacetic anhydride or trichloroacetic anhydride or trichloroacetyl chloride which

latter is used in the presence of pyridine and 4-dimethylaminopyridine. In this reaction the hydroxy groups of the 1-normon-2-yl moiety become acylated and must subsequently be deprotected. Trihaloacetyl groups formed during cyclisation may be removed using potassium carbonate in solvents such as water, alkanols or admixtures thereof. Appropriate deprotecting conditions for removing other acyl residues will be readily apparent to the skilled person. Alternatively the hydroxy groups of the moiety (X) may be protected, prior to cyclising with a carboxylic anhydride, and deprotected by conventional methods such as described below.

Conversion of one compound of formula (I) to another compound of formula (I) may be effected by conventional methods. Thus, for instance, substituents on the groups $R^1$ and $R^2$ may be modified or additional substituents may be inserted. Included within modification of the groups $R^1$ and $R^2$ are salification and esterification of a carboxy substituent, trans- and de-esterification of an ester-containing substituent, reduction of an alkoxycarbonyl substituent and formation of the free carboxy group from a carboxylate salt. Another example of such conversion is the formation of alkanesulphinyl and alkanesulphonyl compounds from the corresponding alkylthio compound of formula (I). This latter conversion may be achieved using conventional oxidising agents such as percarboxylic acids, for instance m-chloroperbenzoic acid, in a suitable solvent.

Compounds of formula (VI) as defined above are novel and useful as chemical intermediates in the aforementioned process.

Accordingly, the present invention also provides a compound of formula (VI), as hereinbefore defined. Compounds of formula (VI) may be produced according to the reaction sequence shown in Scheme II below. This sequence was previously described in EP 0 087 953 (Beecham Group plc.) for the particular instance of compounds of formula (VI) in which the trisubstituted carbon-carbon double bond was of the E-configuration, in which case the starting material was monic acid. Compounds of formula (IA) in which the trisubstituted carbon-carbon double bond has the Z- configuration may be obtained by a similar sequence to that of scheme II, but using isomonic acid, rather than monic acid, as the starting material.

Scheme II

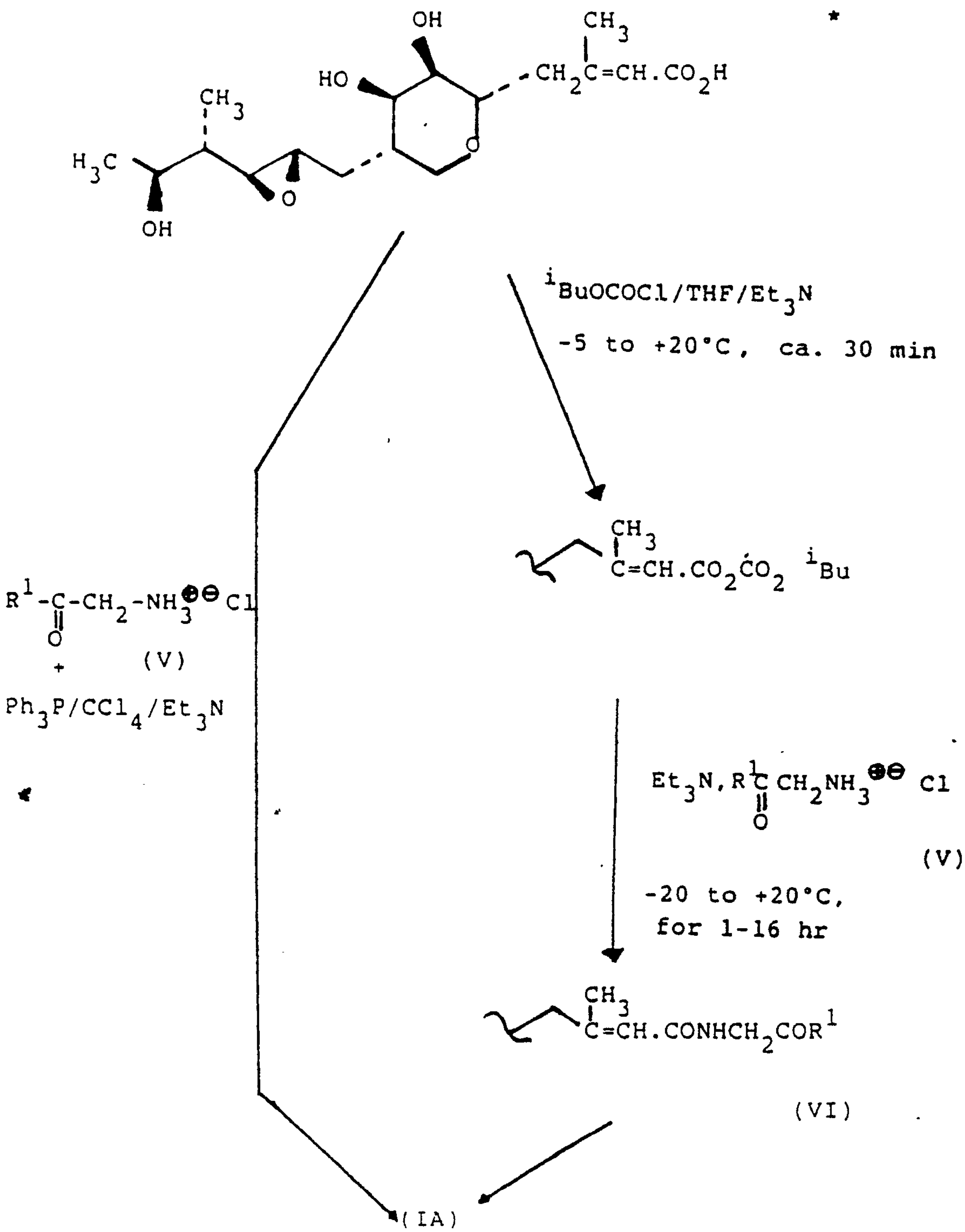


* When trisubstituted carbon-carbon double bond has E-configuration, compound is MONIC ACID

In compounds of formula (I), the configuration of the trisubstituted carbon-carbon double bond may be changed, for instance by photolysis, allowing compounds of formula (I-Z) to be obtained from compounds of formula (I-E).

The hydroxyl groups of monic acid, and compounds of formulae (II) and (VI) may be protected at any

stage of the above processes, using conventional methods.

Particularly suitable protecting groups are silyl groups since these are readily removed under mild conditions. Such groups are introduced using conventional silylating agents, including halosilanes and silazanes, of the formulae below:

$L_3SiX$

$L_2SiX_2$

$L_3SiNL_2$

$L_3SiNHSiL_3$

$L_3SiNHCOL$

$L_3SiNHCONHSiL_3$

$LNHCONHSiL_3$

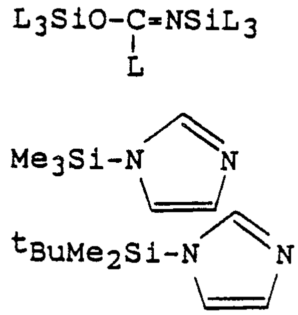

$^tBuMe_2Si-O-SO_2-CF_3$

wherein X is halogen and each group L is independently selected from hydrogen, alkyl, alkoxy, aryl or aralkyl. A preferred silyating agent is trimethylsilyl chloride. Particularly suitable protecting groups are trimethylsilyl, t-butyldimethylsilyl and t-butyldiphenylsilyl groups. Preferred protecting groups are trimethylsilyl groups because of their ease of removal.

The glycol function of monic or isomonic acid and the compounds of formulae (II) and (VI) may be protected by forming a cyclic derivative using a compound of formula (VII):

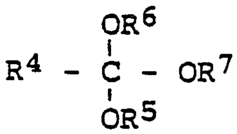

(VII)

wherein $R^4$ is hydrogen or $C_{1-6}$ alkyl and each of $R^5$, $R^6$ and $R^7$ is $C_{1-6}$ alkyl. In the cyclic derivative $Z^1$ and $Z^2$ together are a moiety:

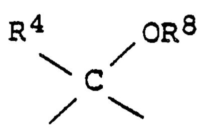

wherein $R^8$ is $C_{1-6}$ alkyl.

Suitably $R^4$ is hydrogen, methyl, ethyl n- or iso-propyl; most suitably it is hydrogen. The groups $R^5$, $R^6$ and $R^7$ are suitably methyl, ethyl, n- or iso-propyl, n-, iso-, sec- or t-butyl; most suitably methyl.

Similarly the hydroxyl groups of a compound of formula (I) may be protected prior to conversion to a further compound of formula (I) as described above.

In each case the protecting groups described above may be removed by mild acid hydrolysis followed by alkaline hydrolysis, for instance, as described by J.P. Clayton, K. Luk and N.H. Rogers, in 'Chemistry of Pseudomonic Acid, Part II', J.C.S. Perkin Trans. I, 1979, 308.

The compounds of this invention are useful for the treatment of bacterial infections in animals, including man, such as respiratory tract infections, otitis, meningitis, and skin and soft tissue infections in man, and mastitis in cattle and respiratory infections in animals such as pigs and cattle.

The compounds of this invention are active against both Gram negative and Gram positive organisms, including Haemophilus, for instance H.influenzae Q1; Branhamella, for instance B.Catarrhalis 1502; Streptococci, for instance S.pyogenes CN10 and S.pneumonia PU7; and Staphylococci, for instance S.aureus Oxford.

This invention also provides a pharmaceutical or veterinary composition which comprises a compound of formula (I) (hereinafter referred to as the 'drug') together with a pharmaceutically or veterinarily acceptable carrier or excipient.

The compositions may be formulated for administration by any route, and would depend on the disease being treated. The compositions may be in the form of tablets, capsules, powders, granules, lozenges, liquid or gel preparations, such as oral, topical or sterile parenteral suspensions.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin, hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For topical application to the skin the drug may be made up into a cream, lotion or ointment. Cream or ointment formulations that may be used for the drug are conventional formulations well known in the art, for example, as described in standard text books of pharmaceutics and cosmetics, such as 'Harry's Cosmeticology' ed. J.B.Wilkinson and R.J.Moore, 7th Edn., George Goodwin, London, and the British Pharmacopoeia.

Suppositories will contain conventional suppository bases, e.g. cocoa-butters or other glyceride.

For parenteral administration, fluid unit dosage forms may be prepared utilizing the drug and a sterile vehicle. The drug, depending on the vehicle and concentration used, can be suspended in the vehicle. Advantageously, adjuvants such as a local anaesthetic, preservative and buffering agents can be dissolved in the vehicle. To enhance the stability the composition can be frozen after filling into the vial and water removed under vacuum. The dry lypophilized powder is then sealed in the vial. The drug can be sterilised by exposure to ethylene oxide before suspending in the sterile vehicle. Advantageously, a surfactant or wetting agent is included in the composition to facilitate uniform distribution of the drug.

For topical application to the ear, the drug may be made up into a suspension in a suitable liquid carrier, such as water, glycerol, diluted ethanol, propylene glycol, polyethylene glycol or fixed oils.

For topical application to the eye, the drug may be formulated as a suspension in a suitable, sterile aqueous or non-aqueous vehicle. Additives, for instance buffers such as sodium metabisulphite or disodium edetate; preservatives including bactericidal and fungicidal agents, such as phenylmercuric acetate or nitrate, benzalkonium chloride or chlorhexidine; and thickening agents such as hypromellose may also be included.

The dosage employed for compositions administered topically will, of course, depend on the size of the area being treated. For the ears and eyes each dose will typically be in the range from 10 to 100 mg of the drug.

Veterinary compositions for intramammary treatment of mammary disorders in animals, especially bovine mastitis, will generally contain a suspension of the drug in an oily vehicle.

The compositions may contain from 0.1% to 99% by weight, preferably from 10-60% by weight, of the drug, depending on the method of administration. Where the compositions are in unit dose form, each dosage unit will preferably contain from 50-500 mg, of the drug. The dosage as employed for adult human treatment will preferably range from 100 mg to 3 g, per day, for instance 250 mg to 2 g of the drug per day, depending on the route and frequency of administration.

Alternatively, especially in the case of animals, the drug may be administered as part of the total dietary intake. In this case the amount of drug employed may be less than 1% by weight of the diet and is preferably no more than 0.5% by weight. The diet for animals may consist of normal foodstuffs to which the drug may be added or the drug may be included in a premix for admixture with the foodstuff.

A suitable method of administration of the drug to animals is to add it to the animals' drinking water. In this case a concentration of the drug in the drinking water of about 5-500 $\mu$g/ml, for example 5-200 $\mu$g/ml, is suitable.

The present invention further provides a method for treating the human, or non-human animal which method comprises administering a compound of formula (I) to a human or non-human in need of such therapy

Alternatively, a pharmaceutical or a veterinary composition as hereinbefore described may be employed

in the treatment.

In particular aspects of the treatment, there are provided methods for treating bacterial infections of human or non-human animals, especially, respiratory infections in humans and in animals.

The following Examples illustrate the invention, but are not intended to limit the scope in any way.

The following abbreviations have been used in the Examples:

| | |
|---|---|
| DMF | N,N-Dimethylformamide |
| DMSO | Dimethyl sulphoxide |
| THF | Tetrahydrofuran |

$MgSO_4$ Anhydrous magnesium sulphate

AMBERLYST 15 (Trade Mark) is a macroreticular, strongly acid cation exchanger resin.

AMBERLITE IR 120 (Trade Mark) is a cross linked polystyrene, sulphonic acid cation exchanger resin.

Chromatography and flash chromatography were executed using silica gel as the adsorbent.

HP20SS is a grade of DIAION HP20 (Trade Mark) styrene-divinylbenzene copolymer resin.

Monic acid A is obtained from pseudomonic acid according to the process described in GB 1 587 058 (Beecham Group Ltd.).

General method of preparation of monamides

To a solution of monic acid A in dry THF (5 ml/mmol) at 0°C were added triethylamine (1.1 equiv.) and isobutyl chloroformate (1 equiv.). After 0.5 h at 0°C the appropriate ammonium salt (1 equiv.) and triethyl amine (1 equiv.) were added and the reaction mixture stirred at 0°C for 3 h. Ethyl acetate was added and the solution washed with aqueous sodium hydrogen carbonate and brine, then dried ($MgSO_4$) and evaporated under reduced pressure. The resulting residue was purified by chromatography (silica gel, eluting with methanol in dichloromethane) to yield pure amide.

General method of preparation of oxazoles via monamides

Trichloroacetyl chloride (9 equiv.) was added to a solution of monamide, 4-dimethylaminopyridine (few crystals/mmol) and pyridine (20 equiv.) in dichloromethane (10 ml/mmol), cooled in an ice bath. After 0.5 h the solution was washed with aqueous sodium-hydrogen carbonate solution and then evaporated under reduced presssure. The resulting residue was dissolved in methanol (5 ml/mmol) and the solution cooled to 0°C before addition of potassium carbonate (3 equiv.). After 15 min at 0°C brine and ethyl acetate were added and the organic layer separated. The aqueous layer was further extracted with ethyl acetate and the combined extracts washed with brine, dried ($MgSO_4$) then evaporated under reduced pressure. The resulting residue was chromatographed on silica (methanol in dichloromethane) to give pure oxazole.

6,7,13-tris-t-butyldimethylsilylmonic acid

Monic acid (11.41g, 33.2mmol), t-butyldimethylsilylchloride (25.0g, 166mmol) and imidazole (22.55g, 332mmol) in N,N-dimethylformamide (50ml) were heated to 70°C for 40h. water (200ml) was added and the solution extracted with ethyl acetate (3 x 200ml) and the extracts dried ($MgSO_4$). After evaporation to dryness under reduced pressure the crude products were dissolved in methanol (360ml), THF (125ml) and water (125ml). Potassium carbonate (14.68g, 106mmol) was added and the solution stirred at room temperature for 1h. The products were evaporated to low volume under reduced pressure, water (200ml) added and 5N hydrochloric acid added until pH 3 was reached. Extraction with dichloromethane (3 x 750ml), drying ($MgSO_4$) and purification by flash chromatography using 0-15% ethyl acetate in hexane gave the title compound (14.16g, 62%) as a white solid; $\nu_{max}$ (KBr) 3420, 3200-2200 (Br), 2929, 2857, 1693, 1638, 1254, 1122, 1085, 837cm$^{-1}$; $\delta_H$ ($CDCl_3$) 0.04-0.10 (18H, m, 6 x $SiCH_3$), 0.89-0.93 (30H, m, 17-$H_3$ and 3 x $^tBu$), 1.19 (3H, d, J 6.3Hz, 14-$H_3$), 1.29-1.43 (1H, m, 12-H), 1.73-1.97 (3H, m, 8-H and 9-$H_2$), 1.98-2.10 (1H, m, 4-H), 2.64-2.77 (2H, m, 10 and 11-H), 3.38 (1H, dd, J 9.1 and 1.8Hz, 6-H), 3.55 (1H, d, J 11.3Hz, 16-H), 3.82-3.95 (4H, m, 5,7,13 and 16-H), 5.76 (1H, s, 2-H); $\delta_C$ ($CDCl_3$), -4.9 ($SiCH_3$). -4.6 ($SiCH_3$), -4.5 ($SiCH_3$),

-4.3 ($SiCH_3$), -4.2 ($SiCH_3$), -3.3 ($SiCH_3$), 12.7 (C-17), 18.0 [$\underline{C}(CH_3)_3$], 18.1 [$\underline{C}(CH_3)_3$], 18.1 [$\underline{C}(CH_3)_3$], 19.1 (C-15), 20.9 (C-14), 25.7 [$C(\underline{C}H_3)_3$], 25.9 [$C(\underline{C}H_3)_3$], 26.1 [$C(\overline{C}H_3)_3$]. 31.9 (C-9), 42.1 (C-8), 43.0 (C-12), 43.5 (C-4), 55.5 (C-10), 59.1 (C-11), 65.4 (C-16), 70.3 (C-6), 70.8 (C-7), 73.4 (C-13), 74.1 (C-5), 116.6 (C-2), 160.5 (C-3), 171.7 (C-1); m/z (FAB 3-NOBA/Na), 731 [$M-H + 2Na)^+$, 100%], 709 [$(MNa)^+$, 27%].

The following Table lists the Examples, the preparation of which is described thereafter.

Table

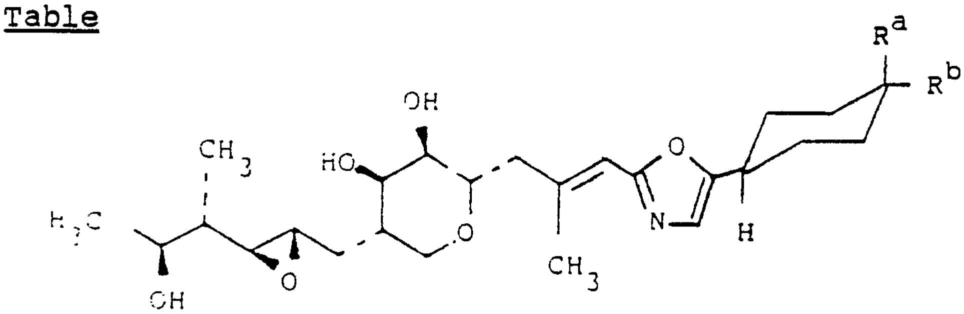

| Example No. | $R^a$ | $R^b$ |
|---|---|---|
| 1 | H | $CO_2Me$ |
| 2 | H | $CH_2OH$ |
| 3 | H | $CO_2Na$ |
| 4 | OH | H |
| 5 | H | OH |
| 6 | H | -CN |
| 7 | H | $-C(OH)Me_2$ |
| 8 | H | 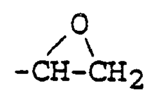 |
| 9 | H | $-CH(OH)CH_2N_3$ |
| 10 | H | 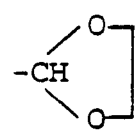 |
| 11 | = O | |
| 12 | H | $-CH(OH)CH_2OEt$ |
| 13 | $= CHCO_2Et$ | |
| 14 | $= CHCH_2OH$ | |

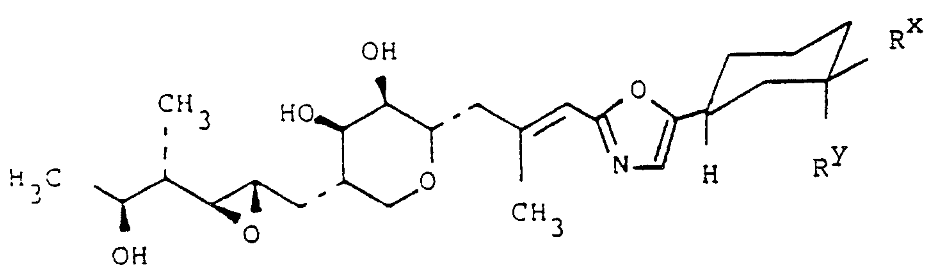

| Example No. | $R^x$ | $R^y$ |
|---|---|---|
| 15 | H | OH |
| 16 | OH | H |

Example 1

5-[trans-(4-Methoxycarbonyl)cyclohexyl]-2-(1-normon-2-yl)oxazole

a) trans-1-Bromoacetyl-4-(methoxycarbonyl)cyclohexane

Oxalyl chloride (2.23 ml. 25.54 mmol) in dichloromethane (20 ml) was added dropwise over 1 h to a solution of trans-1,4-cyclohexanedicarboxylic acid monomethyl ester (4.29 g, 23.06 mmol) in dichloromethane (50 ml) and 2 drops of DMF at room temperature. After a further 0.5 h the reaction products were evaporated to dryness under reduced pressure to give crude trans-1-chlorocarbonyl-4-(methoxycarbonyl) cyclohexane (4.72 g, 100%) as a pale yellow oil, $\nu$ max (liquid film) 2960, 2870, 1800, 1730, 1450, 1440, 1000, 940, 900, 800, 775, 740 $cm^{-1}$; $\delta_H$ ($CDCl_3$) (60M Hz) 1.00-3.00 $\delta$ (10H, multiplet), 3.60 (3H, s, $OCH_3$). The crude acid chloride (4.72 g, 23.06 mmol) in ether (50 ml) was added dropwise over 50 mins to diazomethane (ca. 71 mmol) in ether (ca. 200 ml) at 0°C. After 30 mins at that temperature hydrobromic acid (48%) (16 ml) was added dropwise over 30 mins with rapid stirring. After a further 30 mins the organic layer was separated and washed to neutrality with saturated aqueous sodium hydrogen carbonate. Drying ($MgSO_4$), evaporation to dryness under reduced pressure followed by recrystallisation from light petroleum (b.p. 60-80°C) gave the title compound (5.64 g, 89%) as white needles, m.p. 68-69°C, $\nu$ max (KBr) 2995, 2936, 2859, 1722, 1237, 1021, 650 $cm^{-1}$; $\delta_H$ ($CDCl_3$) 1.38-1.57 (4H, m, cyclohexyl), 1.99-2.11 (4H, m, cycylohexyl), 2.25-2.32 (1H, m, 4-H), 2.66-2.77 (1H, m, 1-H), 3.68 (3H, s, $OCH_3$), 3.96 (2H, s, $CH_2Br$); $\delta_C$ ($CDCl_3$) 204.7 ($COCH_2Br$), 175.7 ($CO_2CH_3$), 51.7 ($OCH_3$), 47.0, 33.2 ($CH_2Br$), 28.0, 27.8; m/z 262 (10.3%, $M^+$) and 141 (100%). (Found: 262.0206. $C_{10}H_{15}O_3Br$ requires 262.0205).

b) N-[2-Oxo-2-(trans-4-methoxycarbonylcyclohexyl)ethyl]monamide

Hexamethylenetetramine (2.82 g, 20.15 mmol) was added to a solution of $\alpha$-bromoketone (Example 1a) (5.30 g, 20.15 mmol) in dichloromethane (100 ml) and the reaction mixture stirred at room temperature for 16 h. Evaporation to dryness under reduced pressure gave the crude hexamethylenetetramine salt as a white solid (8.12 g, 100%), $\delta_H$ ($d_6$-DMSO) 1.20-1.40 (4H, m, cyclohexyl), 1.91-1.99 (4H, m, cyclohexyl), 2.25-2.33 (1H, m, $CHCO_2CH_3$), 2.41-2.51 (1H, m, $CHCOCH_2$), 3.59 (3H, s, $OCH_3$), 4.13 (2H, s, $CH_2Br$), 4.48-4.67 (6H, m, 3 x $NCH_2$), 5.25 (6H, s, 3 x $N^+CH_2$).

The crude hexamethylenetetramine salt (8.12 g, 20.15 mmol) was dissolved in methanol (13 ml) and concentrated hydrochloric acid (6.5 ml) was added. After stirring at room temperature for 16 h the reaction products were evaporated to dryness under reduced pressure to give the crude amine hydrochloride (4.75 g, 100%) as a buff powder, $\delta_H$ ($d_6$-DMSO) 1.14-1.44 (4H, m, cyclohexyl), 1.85-2.05 (4H, m, Cyclohexyl), 2.24-2.35 (1H, m, $CHCO_2CH_3$), 2.48-2.57 (1H, m, $CHCOCH_2$-), 3.59 (3H, s, $OCH_3$). 3.94-4.06 (2H, m, $COCH_2$). The monamide (13b) was prepared according to the general method from the above crude amine hydrochloride (4.50 g, 19.1 mmol) in THf:water (3:1, 200 ml) and isolated after chromatography as a white foam (3.30 g, 33%), $\nu_{max}$ (KBr) 3405, 2932, 2865, 1724, 1660, 1630, 1524, 1450, 1246, 1114, 1049, 1024 $cm^{-1}$, $\lambda$ max (EtOH) 221 nm ($\epsilon_m$ 15,065); $\delta_H$ ($CDCl_3$) 0.93 (3H, d, J 7.1Hz, 17-$H_3$), 1.22 (3H, d, J 6.2Hz, 14-$H_3$), 1.25-1.60 (5H, m, 12-H and 4 x cyclohexyl), 1.90-2.19 (4H, m, cyclohexyl). 2.19 (3H, s, 15-$H_3$), 2.19-2.62 (5H, m, 2 x 4-H, 1″-H, 3″-H and OH), 3.67 (3H, s, $OCH_3$), 4.24 (2H, d, J 4.5Hz, $CH_2NH$), 5.73 (1H, s, 2-H), 6.22 (1H, t, J 4.5 Hz, NH); $\delta_C$ ($CDCl_3$) 208.4 (C-2′), 175.8 ($CO_2$), 167.2 (C-1), 152.2 (C-3), 119.5 (C-2), 74.9 (C-5), 71.2 (C-13), 70.4 (C-7), 68.8 (C-6), 65.4 (C-16), 61.2 (C-11), 55.6 (C-10), 51.7 (OMe), 47.7 (C-1″), 47.5 (C-1′), 42.8 (C-12), 42.6 (C-4), 42.3 (C-4″), 39.5 (C-8), 31.7 (C-9), 28.0 (C-3″), 27.5 (C-2″), 20.9 (C-14), 18.9 (C-15), 12.7 (C-17); m/z (E.I.) 525 (16%, $M^+$), 281 (100%). (Found: 525.2929. $C_{27}H_{43}NO_9$ requires 525.2938).

c) 5-[trans-(4-Methoxycarbonyl)cyclohexyl)-2-(1-normon-2-yl)oxazole

The monamide (Example 1b) (1.44 g, 2.74 mol) was cyclised according to the general procedure to give the oxazole (0.66 g, 47%) as a white foam, $\nu_{max}$ (KBr) 3425, 2934, 2864, 1732, 1654, 1450, 1109, 1044 $cm^{-1}$; $\lambda_{max}$ (EtOH) 264 nm ($\epsilon_m$ 15,510); $\delta_H$ ($CDCl_3$) 0.93 (3H, d, J 7.1Hz, 17-$H_3$), 1.22 (3H, d, J 6.3Hz, 14-$H_3$), 1.25-1.60 (5H, m, 12-H and 4 x cyclohexyl), 1.90-2.24. (5H, m, 8-H and 4 x cyclohexyl), 2.24 (3H, s, 15-$H_3$), 2.24-2.42 (2H, m, 4-H and 4″-H), 2.50-2.64 (3H, m, 4-H, 11-H and 1″-H), 3.68 (3H, s, $OCH_3$), 6.17 (1H, s, 2-H), 6.72 (1H, s, 4′-H); $\delta_C$ ($CDCl_3$) 176.2 ($CO_2$), 160.6 (C-1), 155.2 (C-3), 145.7 (C-5′), 121.0 (C-4′), 113.3 (C-2), 75.3 (C-5), 71.2 (C-13), 70.4 (C-7), 69.0 (C-6), 65.6 (C-16), 61.3 (C-11), 55.7 (C-10), 51.7 (OMe), 42.8

(C-12), 42.7 (C-4″), 42.7 (C-4), 39.6 (C-8), 34.6 (C-1″), 31.8 (C-9), 30.1 (C-3′), 28.4 (C-2″), 20.9 (C-14), 19.5 (C-15), 12.8 (C-17); m/z (E.I.) 507 ($M^+$, 23%), 263 (100%). (Found; 479.2879. $C_{26}H_{41}NO_7$ requires 479.2883).

Example 2

5-[trans-4-(Hydroxymethylcyclohexyl)]-2-normon-2-yl)oxazole

To a solution of the cyclohexyloxazole (Example 1) (0.147 g, 0.29 mmol) and triethylamine (127 µl, 0.90 mmol) in THF (10 ml) was added trimethylsilyl chloride (114 µl, 0.90 mmol) and 4-dimethylaminopyridine (catalytic amount). The mixture was stirred at room temperature for 3.0 h, the mixture was filtered, evaporated to dryness under reduced pressure, dissolved in ether (5 ml), filtered and evaporated to dryness. The products were dissolved in dichloromethane (10 ml), cooled to 0°C, and di-isobutylaluminium hydride (1M in toluene, 0.42 ml, 0.42 mmol) was added. After 3 h at 0°C, methanol (3 ml) was added and the products filtered, and the filtrate evaporated to dryness under reduced pressure. The products were dissolved in THF:$H_2O$ (4:1) (20 ml) and added concentrated hydrochloric acid (4 drops) was added. The reaction mixture was stirred at room temperature for 5 mins and saturated sodium hydrogen carbonate solution was added until pH 8.0 was reached. The solution was extracted with ethyl acetate, dried ($MgSO_4$) and evaporated to dryness under reduced pressure. Purification by flash chromatography using 2-10% methanol in dichloromethane gave the title compound (0.065 g, 47%) as a white foam, $\nu_{max}$ ($CHCl_3$) 3450, 2940, 2860, 1660, 1450, 1360, 1110 $cm^{-1}$; $\lambda_{max}$ (EtOH) 265 nm ($\epsilon_m$ 14,698); $\delta_H$ ($CDCl_3$) 0.93 (3H, d, J 7.1Hz, 17-$H_3$), 1.20 (3H, d, J 6.4Hz, 14-$H_3$), 1.00-1.60 (5H, m, cyclohexyl), 1.83-2.24 (5H, m, 8-H and 4 x cyclohexyl), 2.50-2.64 (3H, m, 4-H, 11-H and 1″-H), 3.75 (2H, t, J 6.8Hz, $CH_2OH$), 6.17 (1H, s, 2-H). 6.70 (1H, s, 4′-H); $\delta_C$ ($CDCl_3$) 160.3 (C-1), 155.7 (C-3), 145.0 (C-5′), 120.9 (C-4′), 113.5 (C-2), 75.2 (C-5), 71.4 (C-14), 70.4 (C-7), 68.9 (C-6), 68.3 ($CH_2OH$), 65.4 (C-16), 61.3 (C-11), 55.5 (C-10), 42.8 (C-12), 42.7 (C-4), 40.0 (C-4″), 39.5 (C-8), 35.4 (C-11″), 31.7 (C-9), 28.8 (C-3″), 25.6 (C-2″), 20.8 (C-14), 19.3 (C-15), 12.7 (C-17); m/z (E.I.) 479 ($M^+$, 12%), 235 (100%); (Found 479.2879. $C_{26}H_{41}NO_7$ requires 479.2883).

Example 3

Sodium 5-(4-Carboxylatocyclohexyl)-2-(1-normon-2-yl) oxazole

The cyclohexyloxazole (Example 1) (0.150 g, 0.29 mmol) was dissolved in trimethylorthoformate (3.0 ml) and treated with p-toluenesulphonic acid (few crystals). After 0.25 h, the mixture was diluted with ethyl acetate, washed with sodium hydrogen carbonate solution and brine, dried and evaporated to dryness under reduced pressure to yield a colourless oil. This was dissolved in THF (3 ml) and 1M sodium hydroxide (3 ml) added. After heating to reflux for 2 h the mixture was cooled, adjusted to pH 2.0 and stirred for 0.5 h. Brine was added and the solution extracted with ethyl acetate. The combined extracts were dried ($MgSO_4$) and evaporated to dryness under reduced pressure. Methanol (3 ml) was added to the crude products and potassium carbonate (6.20 g) added. After stirring for 0.5 h the mixture was diluted with water, adjusted to pH 2.0, and extracted with ethyl acetate. Drying ($MgSO_4$) and evaporated to dryness under reduced pressure gave a white foam. This foam was dissolved in sodium hydrogen carbonate solution and purified by HP20SS chromatography, eluting with 0-9% THF in water, to give the title compound (12d) (0.100 g, 66%) as a white powder, $\nu_{max}$ (KBr) 3409, 2935, 1627, 1420, 1383, 1112, 873 $cm^{-1}$; $\nu_{max}$ (EtOH) 265 nm ($\epsilon_m$ 2,014); $\delta_H$ ($D_2O$) 0.95 (3H, d, J 7.1Hz, 17-$H_3$), 1.19 (3H, d, J 6.5Hz, 14-$H_3$), 1.35-1.56 (5H, m, cyclohexyl), 2.05-2.20 (4H, m, cyclohexyl), 2.60-2.80 (2H, m, 4-H and 1″-H), 3.80-4.00 (4H, m, 5-H, 7-H, 13-H, 16-H), 6.15 (1H, s, 2-H), 6.80 (1H, s, 4′-H); $\delta_C$ ($D_2O/d_6$-DMSO) 186. 8 ($CO_2Na$), 158.4 (C-1), 148.0 (C-3 and C-5′), 121.7 (C-4′), 114.2 (C-2), 76.2 (C-5), 71.1 (C-13), 71.1 (C-7), 70.1 (C-6), 66.6 (C-16), 62.7 (C-11), 58.2 (C-10), 47.8 (C-21), 43.5 (C-12), 43.2 (C-4), 40.6 (C-8), 35.8 (C-1″), 32.7 (C-3″), 31.5 (C-9), 30.6 (C-2″), 20.6 (C-14), 19.9 (C-15), 12.9 (17); m/z (F.A.B.) 516 ($MH^+$, 26%), 532 ($MNH_4^+$, 7%), 538 ($MNa^+$, 3%) and 237 (100%).

Example 4

5-(cis-4-Hydroxycyclohexyl)-2-(1-normon-2-yl)oxazole

a) 2-Oxabicyclo-(2,2,2)-octan-3-one

p-Hydroxybenzoic acid (100.00 g, 0.73 mmol) was hydrogenated over 5% Ru on C (4.00 g) at 1500 p.s.i. in glacial acetic acid (200 ml) at 100°C for 24 h. The crude products were evaporated to dryness under reduced pressure to give a brown oil. This oil was heated to reflux in acetic anhydride (10 ml/g) in two batches. Water (9 ml/g) was added and the resulting solution extracted with light petroleum (b.p. 60-80°C) (4 x 250 ml). The aqueous phase was adjusted to pH 8 with sodium hydrogen carbonate solution and extracted with dichloromethane (4 x 250 ml). The dichloromethane extracts were dried ($MgSO_4$) and evaporated to dryness under reduced pressure to give a brown semi-solid. This semi-solid was sublimed twice at a bath temperature of 110-115°C at 0.1 mmHg to give a white crystalline solid (12.70 g). This solid was soxhlet extracted with light petroleum (b.p. 40-60°C) and recrystallised twice from light petroleum (b.p. 60-80°C) to give the title compound (8.80 g, 10%) as white needles m.p. 126-127°C (lit. 127-128°C), $\nu_{max}$ ($CH_2Cl_2$), 2960, 2840, 1740, 1065, 1000, 955, 878, 770 $cm^{-1}$; $\delta_H$ ($CDCl_3$) 1.65-2.25 (8H, m, cyclohexyl), 2.64-2.71 (1H, m, 4-H), 4.68-4.78 (1H, m, 1-H).

b) cis-4-Methoxycarbonylcyclohexanol

The lactone (Example 4a) (4.0 g, 34.9 mmol) was dissolved in methanol (300 ml) and Amberlyst 15 resin (11.00 g) was added. After stirring at room temperature for 16 h the mixture was filtered and evaporated to dryness under reduced pressure. Purification by flash chromatography using dichloromethane/ethyl acetate (3:1) as eluent gave the title compound (5.41 g, 98%) as a colourless liquid, $\nu_{max}$ (liquid film) 3450, 2940, 2860, 1725, 1440 $cm^{-1}$; $\delta_H$ ($CDCl_3$) 1.55-1.72 [6H, m, 4 x (2-H) and 2 x (3-H equatorial)], 1.89-2.03 [2H, m, 2 x (3-H axial)], 2.11 (1H, br. s, OH), 2.40 (1H, tt, J 8.8 and 3.5Hz, 4-H), 3.89 (1H, quintet, J 4.3Hz, 1-H); $\delta_C$ ($CDCl_3$)$^g$ 175.9 (C=O), 67.0 (C-1), 51.7 (C-6), 41.34 (C-4). 32.2 (C-2), 23.6 (C-3).

c) cis-1-t-Butyldiphenylsilyloxy-4-methoxycarbonylcyclohexane

t-Butylchlorodiphenylsilane (2.06 g, 1.95 ml, 7.5 mmol) and imidazole (1.02 g, 15 mmol) were added to the alcohol (Example 4b) (0.79 g, 5 mmol) in DMF (20 ml). The reaction mixture was stirred at 60°C for 16 h then partitioned between ether (50 ml) and water (50 ml). The ether layer was separated and the aqueous phase extracted with ether. The combined organic extracts were dried ($MgSO_4$), evaporated to dryness under reduced pressure and purified by flash chromatography using dichloromethane/hexane (1:1) as eluent to give the title compound (1.87g, 95%) as a colourless liquid, $\nu_{max}$ (liquid film) 2960, 2840, 1730, 1595, 1425, 1110, 1040, 820, 760 700 $cm^{-1}$; $\delta_H$ ($CDCl_3$),1.07 (9H, s, $C(CH_3)_3$), 1.25-1.43 (2H m, cyclohexyl), 1.63-1.72 (4H, m, cyclohexyl), 1.96-2.10 [2H, m, 2 x (3-H axial)], 2.30 (1H, tt, J 3.4 and 10.5Hz, 4-H), 3.69 (3H, s, $OCH_3$). 3.93-3.98 (1H, m, 1-H), 7.32-7.45 (6H, m, aromatic), 7.64-7.68 (4H, m, aromatic); $\delta_C$ ($CDCl_3$) 176.2 (C=O), 135.8, 134.5 (qu), 129.5, 127.5, 67.5 (C-1), 51.5 (C-6), 42.1 (C-4), 32.4 (2), 27.0 [($C(CH_3)_3$], 25.5 (C-3), 19.3 [$C(CH_3)_3$]; m/z (C.I.) 397 ($MH^+$); (Found: 339.1406. $C_{20}H_{23}O_3Si$ (M-$^t$Bu requires 339.1417.

d) cis-4-t-Butyldiphenylsilyloxycyclohexanecarboxylic acid

The methyl ester (Example 4c) (3.06 g, 7.72 mmol) in methanol (120 ml), THF (40 ml) and 1M lithium hydroxide (38.60 ml, 38.6 mmol) was stirred at room temperature for 16 h. The solution was adjusted to pH 7 with Amberlite IR 120 $H^+$ resin and filtered, the filtered resin washed with dichloromethane, and the filtrate evaporated to dryness under reduced pressure. Purification by flash chromatography using dichloromethane/acetic acid (25:1) as eluent gave the title compound (2.61 g, 88%) as white needles m.p. 119-120°C, $\nu_{max}$ (KBr) 3422, 3200-2500 (br.) 2930, 2860, 1700, 1426, 1112, 1048, 935, 740, 700 $cm^{-1}$; $\delta_H$ ($CDCl_3$) 1.07 (9H, s, $^t$Bu), 1.32-1.45 2 x (3-H axial)], 2.35 (1H, tt, J 10.4 and 3.1Hz, 4-H), 3.94 (1H, br.s, 1-H), 7.33-7.46 (6H, m, aromatic), 7.64-7.68 (4H, m, aromatic); $\delta_C$ ($CDCl_3$), 182.7 (C=O), 135.8, 134.5 (qu), 129.5, 127.5, 67.0 (C-4), 32.0 (C-3), 27.0 [$C(CH_3)_3$], 23.6 (C-2), 19.2 [$C(CH_3)_3$]; m/z (F.A.B.) 427 [$(M-H+2Na)^+$, 8%], 421 [$(M+K)^+$, 10%], 405 [(M+Na), 12%], [Found: C, 72.0; H, 8.0. $C_{23}H_{30}O_3Si$ requires C, 72.2; H,

7.9%).

e) cis- and trans-4-t-Butyldiphenylsilyloxy-1-chloroacetylcycloxhexane

Oxalyl chloride (0.66 ml, 78.46 mmol) was added dropwise over 0.5 h to a solution of the cyclohexyl carboxylic acid (Example 4d) (2.59 g, 6.78 mmol) in dichloromethane (30 ml) and DMF (2 drops) at 0°C. After a further 0.5 h at room temperature the products were evaporated to dryness under reduced pressure to give the crude acid chloride as a pale yellow oil, $\nu_{max}$ (liquid film) 2940, 2860, 1800, 1420, 1110, 920, 780, 760, 700 cm$^{-1}$; $\delta_H$ (90M Hz, $CDCl_3$) 1.05 (9H, s, $^tBu$), 1.40-3.00 (9H, m, cyclohexyl). 3.80-4.10 (1H, m, 4-H), 7.25-7.45 (6H, m, aromatic), 7.44-7.70 (4H, m, aromatic).
The crude acid chloride in dichloromethane (20 ml) was added dropwise to diazomethane (ca. 11 mmol) in ether (ca. 60 ml) with rapid stirring at 0°C. After 0.5 h at 0°C hydrogen chloride gas was bubbled through the solution for 1 h. The solution was neutralised with sodium hydrogen carbonate solution and extracted with dichlromethane. Drying ($MgSO_4$), evaporation to dryness under reduced pressure and purification by flash chromatography using hexane/dichloromethane (2:1) as eluent gave firstly the cis- title compound (1.74 g, 62%) as a colourless liquid. $\nu_{max}$ (liquid film) 2960, 2840, 1725, 1420, 1110, 1050, 820, 760, 700 cm$^{-1}$; $\delta_H$ ($CDCl_3$), 1.07 (9H, s, $^tBu$), 1.32-1.43 (2H, m, cyclohexyl), 1.60-1.76 (4H, m, cyclohexyl), 1.93-2.08 [2H, m, 2 x (3-H axial)], 2.61 (1H, tt, J 3.6 and 11.2Hz, 1-H), 4.00 (1H, br, m, 4-H), 4.20 (2H, s, $CH_2Cl$), 7.33-7.46 (6H, m, aromatic), 7.63-7.66 (4H, m, aromatic); $\delta_C$ ($CDCl_3$) 204.6 (C=O), 135.8, 134.4 (qu), 129.6, 127.5, 66.9 (C-4), 47.2 (C-1), 47.0 ($CH_2Cl$), 32.3 (C-3), 27.0 [$C(CH_3)_3$], 22.7 (C-2), 19.3 [$C(CH_3)_3$]; m/z (C.I.) 415 ($MH^+$, 67%), 357 (100%); (Found: C, 69.6; H, 7.8. $C_{24}H_{31}O_2SiCl$ requires C, 69.5; H, 7.67%) followed by the trans-title compound (0.63 g, 22%) as a colourless oil, $\nu_{max}$ (liquid film) 2960, 2840, 1725, 1420, 1110, 1050, 820, 760 and 700 cm$^{-1}$; $\delta_H$ ($CDCl_3$) 1.05 (9H, s, $^tBu$), 1.18-1.49 (4H, m, cyclohexyl), 1.79-1.91 (4H, m, cyclohexyl), 2.57 (1H, tt, J 3.4 and 11.3Hz, 1-H), 3.57 (1H, tt, J 4.2 and 0.1Hz, 4-H), 4.10 (2H, s, $CH_2Cl$), 7.33-7.46 (6H, m, aromatic), 7.63-7.66 (4H, m, aromatic); $\delta_C$ ($CDCl_3$) 204.7 (C=O), 135.9, 135.1 (qu), 129.9, 127.8, 71.3 (C-4), 4.75 ($CH_2Cl$), 46.75 (C-1), 34.7 (C-3), 27.3 ($C(CH_3)_3$], 26.8 (C-2), 19.4 [$C(CH_3)_3$]; m/z (C.I.) 415 ($MH^+$, 90%), 337 (100%); (Found: C, 69.8; H, 8.0. $C_{24}H_{31}O_2SiCl$ requires C, 69.5; H. 7.55%).

f) cis-4-Chloroacetylcyclohexanol

The cis-isomer silyl ether (Example 4e) (1.73 g, 4.17 mmol) was dissolved in 3% methanolic hydrogen chloride (50 ml) and stirred at room temperature for 40 h. The solution was neutralised with sodium hydrogen carbonate solution, water (100 ml) added and the aquecus phase extracted with dichloromethane. Drying ($MgSO_4$), evaporation to dryness under reduced pressure and purification by flash chromatography using dichloromethane/ethyl acetate (3:1) as eluent gave the title compound (0.62 g, 82%) as white needles m.p. 51-52°C, $\nu_{max}$ ($CH_2Cl_2$) 3500, 3400, 2960, 2840, 1725, 1450, 1400, 1120, 1050, 965 cm$^{-1}$; $\delta_H$ ($CDCl_3$) 1.32 (1H, br.s, OH), 1.58-2.05 (8H, m, cyclohexyl), 2.70 (1H, tt J 3.6 and 9.8Hz, 4-H), 3.95-4.05 (1H, m, 1-H), 4.18 (2H, s, $CH_2Cl$); $\delta_C$ ($CDCl_3$) 204.71 (C=O), 65.9 (C-1), 47.2 (C-4), 46.49 ($CH_2Cl$), 31.8 (C-2), 22.7 (C-3); m/z C.I.) 177 [$(MH)^+$, 31%] and 159 (100%); (Found: C, 54.6; H, 7.3, Cl, 19.8. $C_8H_{13}O_2Cl$ requires C, 54.5; H, 7.4; Cl, 20.1%).

g) cis-4-Azidoacetylcyclohexanol

cis-4-Chloroacetylcyclohexanol (0.62 g, 3.5 mmol) in chloroform (25 ml) was treated with tetramethylguanidinium azide (0.66 g, 4.2 mmol) for 16 h at room temperature. Evaporation to dryness under reduced pressure and purification by flash chromatography using dichloromethane/ethyl acetate (3:1) as eluent gave the title compound (0.58 g, 90%) as white needles, m.p. 74-75°C, $\nu_{max}$ ($CH_2Cl_2$) 3420, 2940, 2100, 1725, 1270 cm$^{-1}$, $\delta_H$ ($CDCl_3$) 1.43 (1H, br.s, OH), 1.57-1.97 (8H, m, cyclohexyl), 2.47 (1H, tt, J 3.7Hz, and 9.9Hz, 4H), 3.95-4.05 (1H, m 1-H), 4.02 (2H, s, $CH_2Cl$); $\delta_C$ ($CDCl_3$) 205.9 (C=O), 65.9 (C-1), 55.8 (C-4), 47.1 ($CH_2Cl$), 31.7 (C-2), 22.6 (C-3).

h) N-[2-Oxo-2-(cis-4-hydroxycyclohexyl)ethyl]monamide

The azide (Example 4g) (0.57 g, 3.11 mmol)) in methanol (50 ml), water (25 ml) and concentrated hydrochloric acid (2.61 ml) was hydrogenated over 10% Pd on C (0.057 g) at 1 atm. for 2.5 h. The reaction mixture was filtered and the filtrate evaporated to dryness under reduced pressure to give the crude cis-4-aminoacetylcyclohexanol hydrochloride $\delta_H$ ($d_6$-DMSO) 1.43-1.81 (8H, m, cyclohexyl), 2.49-2.57 (1H, m, 4-H), 3.68 3.78 (1H, m, 1-H), 3.97-3.99 (2H, m, $CH_2N$.)

The monamide was prepared according to the general method from the crude amine hydrochloride (0.73 g, 3.77 mmol) in THF:water (1:1, 100 ml) and isolated after chromatography using 0-20% methanol in dichloromethane then rechromatographed using acetone/ethyl acetate (2:1) as a white foam (0.63 g, 35%), $\nu_{max}$ (KBr) 3407, 2934, 1717, 1658, 1629, 1448, 1262, 1052 $cm^{-1}$; $\lambda_{max}$ (EtOH) 220 nm ($\epsilon_m$ 15,464); $\delta_H$ - ($CDCl_3$) 0.93 (3H, d, J 7.0Hz, 17-$H_3$), 1.23 (3H, d, J 6.2Hz, 14-$H_3$), 1.63-2.08 (14H, m, 9-$H_2$, 8-H, 3 x OH and 8 x cyclohexyl), 2.38-2.55 (2H, m, 1″-H and OH), 3.99 (1H, m, 4″-OH), 4.25 (2H, d, J 4.5Hz, 1′-$H_2$), 5.75 (1H, s, 2-H), 6.29 (1H, t, J 4.5Hz, NH), $\delta_C$ ($CD_3OD$), 210.3 (C.2′), 169.7 (C-1), 153.1 (C-3), 120.7 (C-2), 76.2 (C-5), 71.6 (C-13), 70.7 (C-7), 70.0 (C-4″), 67.0 (C-6), 66.3 (C-16), 61.2 (C-11), 56.9 (C-10), 48.1 (C-1′), 47.8 (C-1″), 43.7 (C-12), 43.7 (C-4), 41.6 (C-8), 33.0 (C-3″), 32.7 (C-9), 23.9 (C-2″), 20.3 (C-14), 19.0 (C-15), 12.3 (C-17).

i) 5-[cis-4-Hydroxycyclohexyl]-2-(1-normon-2-yl)oxazole

The monamide (13e) (0.30 g, 0.62 mmol) was cyclised according to the general procedure to give the oxazole as a white foam (0.13 g, 45%),$\nu_{max}$ (EtOH) (KBr) 3399, 2933, 1717, 1653, 1450, 1379, 1110, 1050 and 960 $cm^{-1}$; $\lambda_{max}$ (EtOH) 265 nm, ($\epsilon$

m

12707); $\delta_H$ ($CDCl_3$) 0.95 (3H, d, J 6.9Hz, 17-$H_3$), 1.24 (3H, d, J 6.3Hz, 14-$H_3$), 1.60-2.10 (11H, m, 9-H2, 18-H and 8 x cyclohexyl), 2.28 (3H, s, 15-$H_3$), 2.55-2.89 (4H, m, 4-H, 16-H, 11-H and 4″-H), 6.32 (1H, s, 2-H), 6.90 (1H, s, 4′-H); $\delta_C$ ($CDCl_3$) 160.5 (C-1) 155.2 (C-3), 145.6 (C-5′), 121.4 (C-4′), 113.2 (C-2), 75.2 (C-5), 71.0 (C-13), 70.4 (C-7), 68.8 (C-6), 66.5 (C-4″), 65.5 (C-16), 61.2 (C-11), 55.6 (C-10), 42.7 (C-12), 42.7 (C-4), 39.6 (C-8), 33.6 (C-1″), 31.9 (C-9), 31.72 (C-3″), 25.3 (C-2″), 20.7 (C-14, 19.4 (C-15), 12.6 (C-17); m/z (E.I.) 465 ($M^+$, 15%) and 221 (100%); (Found: 465.2698. $C_{25}H_{39}NO_7$ requires, 465.2728).

Example 5

5-[trans-(4-Hydroxycyclohexyl)]-2-(1-normon-2-yl)oxazole

a) trans-4-Chloroacetylcyclohexanol

The trans-isomer (Example 4e) (0.97 g, 2.34 mmol) was dissolved in 3% methanolic hydrogen chloride (25 ml) and stirred at room temperature for 40 h. The solution was evaporated to dryness under reduced presssure and purified by flash chromatography using dichloromethane/ ethyl acetate (3:1) as eluent to give the title compound (0.32 g, 70%) as white needles m.p. 78-79°C, $\nu_{max}$ (KBr) 3414, 2938, 2860, 1723, 1455, 1395, 1075, 1055 $cm^{-1}$; $\delta_H$ ($CDCl_3$) 1.24-1.56 (4H, m, cyclohexyl), 1.60 (1H, br.s, OH), 1.92-2.10 (4H, m, cyclohexyl), 2.62 (1H, tt, J 11.4 and 3.6Hz, 5-H), 3.62 (1H, tt, J 10.4 and 3.4Hz, 1-H), 4.17 (2H, s, $CH_2Cl$); $\delta_C$ ($CDCl_3$) 204.8 (C O), 69.5 (C-1), 47.3 (C-4), 45.5 (C-6), 34.4 (C-2), 26.7 (C-3); m/z (C.I.), 177 ($MH^+$, 40% and 159 (100%); (Found: C, 54.5; H, 7.5; Cl, 19.8. $C_8H_{13}NO_2Cl$ requires C, 54.4; H, 7.4; Cl, 20.1%).

b) trans-4-Azidocycylohexanol

trans-4-Chloroacetylcyclohexanol (0.33 g, 1.86 mmol) in chloroform (20 ml) was treated with tetramethylguanidinum azide (0.35 g, 2.23 mmol) for 16 h at room temperature. Evaporation to dryness under reduced pressure and purification by flash chromatography using dichloromethane/ethyl acetate (3:1) as eluent gave the title compound (0.33 g, 97%) as a colourless oil which solidified on standing, $\nu_{max}$ (liquid film) 3420, 2940, 2100, 1725, 1270 $cm^{-1}$; $\delta_H$ ($CDCl_3$) 1.23-1.56 (5H, m, 4 x cyclohexyl and OH), 1.89-1.95 (2H, m, cyclohexyl), 2.03-2.11 (2H, m, cyclohexyl), 2.39 (1H, tt, J 11.5 and 4.4Hz, 4-H), 3.61 (1H, tt, J 10.4 and 4.3Hz), 4.01 [2H, s, ($CH_2N_3$)]; $\delta_C$ ($CDCl_3$) 206.97 (C=O), 69.5 (C-1), 56.1 ($CH_2N_3$), 47.1 (C-4). 34.3 (C-2), 31.8 (C-1); (Found: C, 54.8; H, 7.5; N, 22.5. $C_8H_{13}N_3O_2$ requires C, 54.4; H, 7.4; N, 22.9%).

c) N-[2-Oxo-2-(trans-4-hydroxycyclohexyl)ethyl] monamide

The azide (Example 5b) (0.325 g, 177 mmol) in methanol (30 ml), water (15 ml) and concentrated hydrochloric acid (1.49 ml) was hydrogenated over 10% Pd on C (0.0325 g) at 1 atm. for 2.5 h. The reaction mixture was filtered and the filtrate evaporated to dryness under reduced pressure to give the crude trans-4-aminoacetylcyclohexanol $\delta_H$ ($d_6$DMSO) 1.08-1.34 (4H, m, cyclohexyl), 1.77-1.90 (4H, m, cyclohexyl). 3.34 (1H, tt, J 10.1 and 3.7Hz, 4-H). 3.94-4.01 (2H, m, $CH_2N$), 8.22 (3H, br.s, $NH_3$).

The monamide was prepared according to the general method from the crude amine hydrochloride (0.343 g, 1.77 mmol) in THF:water (1:1, 50 ml) and isolated after chromatography using 10-20% methanol in dichloromethane then rechromatographed using acetone/ethyl acetate (2:1) as a white foam (0.26 g, 31%), $\nu_{max}$ (KBr) 3415, 2930, 1716, 1659, 1029, 1055 $cm^{-1}$; $\lambda_{max}$ (EtOH) 220 nm ($\epsilon_m$ 13,430); $\delta_H$ ($CD_3OD$) 0.93 (3H, d, J 7.0Hz, 17-$H_3$), 1.22 (3H, d, J 6.3Hz, 14-$H_3$), 1.25-1.54 (5H, m, 4 x cyclohexyl and 12-H), 1.88-2.08 (5H, m, 4 x cyclohexyl and 8-H), 2.41 (1H, tt, J 11.6 and 3.4Hz, 1''H), 3.84-3.90 (1H, m, 4''-H), 4.20 (2H, s, 1'-$H_2$), 5.75 (1H, s, 2-H), 6.75 (1H, t, J 4.7Hz, NH), $\delta_C$ ($CD_3OD$) 210.4 (C-2'), 169.6 (C-1), 153.1 (C-3). 120.6, 76.2 (C-5), 71.6 (C-13), 70.7 (C-7), 70.5 (C-4''), 70.0 (C-6), 66.3 (C-16), 61.2 (C-11), 56.8 (C-10), 56.0 (C-1'), 48.2 (C-1''), 43.7 (C-12) 43.7 (C-4), 41.6 (C-8), 35.3 (C-3''), 33.0 (C-9), 27.8 (C-2''), 20.3 (C-14), 19.0 (C-5), 12.3 (C-17); m/z (E.I.) 483 ($M^+$, 1%), 139 (100%); (Found; 483.2856. $C_{25}H_{41}NO_8$ requires 483.2832.

d) 5-[trans-(4-Hydroxylcyclohexyl)1-2-(1-normon-2-yl) oxazole

The monamide (Example 5c) (0.25 g, 0.54 mmol) was cyclised according to the general procedure to give the oxazole (0.070 g, 28%) as a white foam, $\nu_{max}$ (KBr) 3405, 2961, 1654, 1515, 1451, 1379, 1113, 1056 $cm^{-1}$; $\lambda_{max}$ (EtOH) 265 nm ($\epsilon_m$ 16560); $\delta_H$ ($CD_3OD$) 0.95 (3H, d, J 7.0Hz, 17-$H_3$), 1.24 (3H, d, J 6.5Hz, 14-$H_3$), 1.30-1.62 (5H, m, 12-OH and 4 x cyclohexyl), 1.78-2.28 (5H, m, 8-H and 4 x cyclohexyl), 2.28 (3H, s, 15-$H_3$), 2.60-2.75 (2H, m, 11H, and 1''H), 6.32 (1H, s, 2-H), 6.97 (1H, s, 4'-H), 6.97 (1H, s, 4'-H) $\delta_C$ ($CD_3OD$) 162.1 (C-1), 156.9 (C-3), 147.8 (C-5'), 121.7 (C-4'), 113.8 (C-2), 76.4 (C-5), 71.8 (C-13), 70.8 (C-7), 70.7 (C-6), 70.0 (C-4''), 66.3 (C-6), 61.3 (C-11), 56.9 (C-10), 43.7 (C-12), 43.7 (C-4), 41.5 (C-8), 35.6 (C-1''), 35.5 (C-3''), 33.0 (C-9), 30.3 (C-2''), 20.4 (C-14), 19.6 (C-15), 12.3 (C-17); m/z (E.I.) 465 ($M^+$, 7%), 221 (100%). (Found: 465.2735. $C_{25}H_{39}NO_7$ requires 465.2726).

## Example 6

### 5-[trans-4-Cyanocyclohexyl]-2-(1-normon-2-yl)oxazole

a) trans-1-Bromoacetyl-4-cyanocyclohexane

trans-4-Carboxamidocyclohexanecarboxylic acid (6.80g, 39.8mmol) in thionyl chloride (25ml) was heated to reflux for 4h. Evaporation to dryness under reduced pressure afforded crude trans-4-cyanocyclohexanecarbonyl chloride (6.82g, 100%) as a pale yellow solid; $\nu_{max}$ ($CH_2Cl_2$) 2250, 1800$cm^{-1}$. The crude acid chloride in dichloromethane (25ml) was added dropwise over 0.5h to diazomethane (ca.100mmol) in ether (ca.250ml) at 0°C. After 0.5h at that temperature excess diazomethane was removed by bubbling argon through the solution. Evaporation to dryness under reduced pressure gave the crude $\alpha$-diazoketone as a pale yellow solid which was dissolved in dichloromethane (150ml) and hydrogen bromide was bubbled into the solution for 10mins. The products were poured into a large excess of saturated sodium hydrogen carbonate solution and the aqueous phase extracted with chloroform (3 x 250ml). Evaporation to dryness under reduced pressure and purification by flash chromatography using dichloromethane as eluent gave the title compound (7.27g, 79%) as a white solid, $\nu_{max}$ ($CHCl_3$) 2950, 2860, 2250, 1725, and 1450$cm^{-1}$; $\delta_H$ - ($CDCl_3$) 1.39-1.63 (4H, m, cyclohexyl), 1.95-2.07 (2H, m, cyclohexyl), 2.13-2.27 (2H, m, cyclohexyl), 2.48 (1H, tt, J 11.3 and 3.8Hz, 4-H), 2.82 (1H, tt, J 11.0 and 3.4Hz, 1-H), and 3.94 (2H, s, $CH_2Br$); δC ($CDCl_3$) 26.9 (C-3), 27.5 (C-4). 28.5 (C-2), 32.9 ($CH_2Br$), 45.7 (C-1), 121.9 (CN), and 203.4 (C=O); m/z 231 ($M^+$, 2%), 229 ($M^+$, 2%), 136 (100%) (Found: $M^+$, 229.0134. $C_9H_{12}NOBr$ requires M, 229.0123.

b)trans-1-Azidoacetyl-4-cyanocyclohexane

The above α-bromoketone (1.00g, 4.35mmol) in chloroform (15ml) was treated with tetramethylguanidinium azide (0.72g, 4.56mmol) for 16h at room temperature. Evaporation to dryness under reduced pressure and purification by flash chromatography using dichloromethane as eluent gave the title compound (0.81g, 97%) as a white semi-solid, $\nu_{max}$ (liquid film) 2960, 2840, 2250, 2100, 1720, 1450, and 1280cm$^{-1}$; $\delta_H$ ($CDCl_3$) 1.47-1.73 (4H, m, cyclohexyl), 1.91-2.04 (2H, m, cyclohexyl), 2.25-2.33 (2H, m, cyclohexyl), 2.42-2.56 (2H, m, 1-H and 4-H), and 4.00 (2H, s, $CH_2N_3$); $\delta_C$ ($CDCl_3$) 26.3 (C-3), 27.5 (C-4), 28.5 (C-2), 46.0 (C-1), 56.0 ($CH_2N_3$), 121.8 (CN), and 205.8 (C=O).

c) N-(2-Oxo-2-(trans-4-cyanocyclohexyl)ethyl]monamide

The azide (5.23g, 27.24mmol) in methanol (200ml), water (100ml) and concentrated hydrochloric acid (23.0ml) was hydrogenated over 10% palladium on charcoal (0.26g) at 1 atm. for 30min. The products were filtered and evaporated to dryness under reduced pressure then partitioned between dichloromethane (250ml) and water (250ml). The aqueous layer was separated and evaporated to dryness to give the amine hydrochloride salt (4.56g, 88%) as a white solid, $\nu_{max}$ (KBr) 3405, 2950, 2241, 1719, and 1001cm$^{-1}$; $\delta_H$ - ($D_6$DMSO) 1.17-1.75 (4H, m, cyclohexyl), 1.75-2.15 (4H, m, cyclohexyl), 2.56 (1H, tt, J 11.5 and 3.5Hz, 4-H or 1-H), 2.71 (1H, tt, J 11.5 and 3.6Hz, 4-H or 1-H), 3.96-4.03 (2H, m, $CH_2N^+H_3$), and 8.26 (3H, br.s, $N^+H_3$).

The monamide was prepared according to the general method from the above crude amine hydrochloride (4.56g, 23-85mmol) in THF:water (1:1, 100ml) and isolated after chromatography using 0-6% methanol in dichloromethane as a white foam (4.71g, 40%), $\nu_{max}$ (KBr) 3417, 2937, 2241, 1719, 1630 and 1112cm$^{-1}$; $\nu_{max}$ (EtOH) 221nm, ($\epsilon_m$ 16,210); $\delta_H$ ($CDCl_3$) 0.93 (3H, d, J 7.0Hz, 17-$H_3$), 1.22 (3H, d, J 6.3Hz, 14-$H_3$), 1.25-1.75 (7H, m, 12-H, 9-$H_2$ and 4 x cyclohexyl), 1.94-2.09 (3H, m, 8-H and 2 x cyclohexyl), 2.18 (3H, s, 15-$H_3$), 2.16-2.27 (3H, m) 4-H and 2 x cyclohexyl), 2.40-2.59 (3H, m, 4-H, 1″-H and 4″-H), 2.72 (1H, dd, J 7.8 and 2.1Hz, 11-H), 2.78-2.55 (1H, m, 10-H), 4.22 (2H, d, J 4.6Hz, 1′-$H_2$), 5.73 (1H, s, 2-H), and 6.31 (1H, t, J 4.6Hz, NH); $\delta_C$ ($CDCl_3$) 12.7 (C-17), 18.9 (C-25), 20.8 (C-14), 26.5 (C-3″), 27.5 (C-4″), 28.5 (C-2″), 31.7 (c-9), 39.6 (C-8), 42.6 (C-4), 42.8 (C-12), 46.4 (C-1″), 47.5 (C-1″), 55.6 (C-10), 61.1 (C-11), 65.3 (C-16), 68.6 (C-6), 70.4 (C-7), 71.1 (C-13), 74.9 (C-5), 119.4 (C-2), 122.0 (C-5″), 152.3 (C-3), 167.2 (C-1), and 207.7 (C-2′); m/z ($M^+$, 7%), 111 (100%).

d) 5-[trans-4-Cyanocyclohexyl]-2-(1-normon-2-yl)oxazole

The monamide (5.00g, 10.16mmol) was cyclised according to the general procedure. Chromatography using 1-5% methanol in dichloromethane as eluent and rechromatography using methyl acetate as eluent gave the title compound (0.99g, 20%) as a white foam, $\nu_{max}$ (KBr) 3424, 2932, 2865, 2241, 1719, 1655, 1450, 1111, and 1050cm$^{-1}$; $\lambda_{max}$ (EtOH) 265nm, ($\epsilon_m$ 16,720); $\delta_H$ ($CD_3OD$) 0.94 (3H, d, J 17.1Hz, 17-$H_3$), 1.19 (3H, d, J 6.4Hz, 14-$H_3$), 1.35-1.85 (11H, m, 12-H, 9-Hz and 8 x cyclohexyl), 2.18 (3H, s, 15-$H_3$), 2.62-2.84 (5H, m, 1″-H, 4″-H, 11-H, 10-H and 4-H), 6.12 (1H, s, 2-H), and 6.82 (1H, s, 4′-H); $\delta_C$ ($CD_3OD$) 12.3 (C-17), 19.6 (C-15), 20.3 (C-14), 28.5 (C-3″), 30.0 (C-4″), 30.3 (C-2″), 33.0 (C-9), 34.8 (C-1″), 41.6 (C-8), 43.7 (C-4), 43.7 (C-12), 56.6 (C-10), 61.2 (C-11), 66.3 (C-16), 70.0 (C-6), 70.6 (C-7), 71.6 (C-13), 76.4 (C-5), 113.7 (C-2), 122.0 (C-4′), 123.5 (C-5″), 148.0 (C-5′), 156.2 (C-3), and 162.1 (C-1); m/z (FAB, thioglycerol) 475 [$(M+H^+)^+$, 100%].

Example 7

5-[trans-4-(2-Hydroxyprop-2-yl)cyclohexyl]-2-(1-normon-2-yl)oxazole

a) 5-[trans-4-Methoxycarbonylcyclohexyl]-2-(1-nor-6,7,13-tris-t-butyldimethylsilyloxymon-2-yl)oxazole

t-Butyldimethylsilyltrifluoromethane sulphonate (1.32g, 1.15ml, 5.00mmol) was added dropwise to a stirred solution of 5-[trans-4-methoxycarbonylcyclohexyl]-2-(1-normon-1-yl)oxazole (0.507g, 1.00mmol) and 2,6-lutidine (0.80g, 0.87ml, 7.48mmol) in dichloromethane (10ml) at -20°C. After a further 5h at -20°C,

methanol (15ml) was added and the solution warmed to room temperature. Brine (15ml) was added, and the organic layer separated. The aqueous phase was extracted with dichloromethane (3 x 20ml), the organic extracts combined, dried ($MgSO_4$) and evaporated to dryness under reduced pressure. Purification by flash chromatography using ether/hexane (1:1) as eluent gave the title compound (0.49g, 58%) as a white foam; $\nu_{max}$ (KBr) 2954, 2857, 1732, 1654, 1470, 1123, 1084, 837, 774cm$^{-1}$; $\delta_H$($CDCl_3$) 0.04-0.10 (18H, m, 6 x $SiCH_3$), 0.85-0.97 (30H, m, 17-$H_3$ and 3 x $^tBu$), 1.20 (3H, d, J 6.3Hz, 14-$H_3$), 2.21 (3H, s, 15-$H_3$), 3.39-3.42 (1H, m, 6-H), 3.53-3.58 (1H, m, 16-H), 6.20 (1H, s, 2-H), 6.74 (1H, s, 4′-H); $\delta^C$ ($CDCl_3$) -4.9 ($SiCH_3$), -4.6 ($SiCH_3$), -4.5 ($SiCH_3$), -4.3 ($SiCH_3$), -4.2 ($SiCH_3$), -3.2 ($SiCH_3$), 12.7 (C-17), 18.0 [$C(CH_3)_3$], 18.1 [$C(CH_3)_3$], 18.1 [$C(CH_3)_3$], 19.0 (C-15), 20.6 (C-14), 25.7 [$C(CH_3)_3$], 26.1 [$C(CH_3)_3$], 28.3 (C-2″), 30.1 (C-3″), 32.0 (C-9), 34.5 (C-1″), 42.2 (C-8), 43.0 (C-4″), 43.1 (C-12), 43.1 (C-4), 51.6 (C-6″), 55.5 (C-10), 58.9 (C-11), 65.5 (C-16), 70.4 (C-6), 70.9 (C-7), 73.5 (C-13), 74.4 (C-5), 113.0 (C-2), 121.3 (C-4′), 145.7 (C-5′), 154.5 (C-3), 160.6 (C-1), 175.9 (C-5″); m/z (FAB 3-NOBA/Na) 872 ([MNa]$^+$, 48%), 159 (100%).

b) 5-[trans-4-(2-Hydroxyprop-2-yl)cyclohexyl]-2-(1-nor-6,7,13-tris-t-butyldimethylsilyloxymon-2-yl)oxazole

Methylmagnesium iodide (3.0M in ether) (1.00ml, 3.00mmol) was added dropwise to a solution of example 7a in THF (10ml) at -20°C. Allowed to warm to room temperature over 1h and stirred for 16h. The products were neutralised with pH 7 buffer solution and extracted with dichloromethane (3 x 20ml). Drying ($MgSO_4$), evaporation to dryness under reduced pressure and purification by flash chromatography using hexane/ether (3:2) as eluent gave the title compound (0.121g, 60%) as a white foam; $\nu_{max}$ (KBr) 3425, 2930, 2859, 1655, 1450, 1111, 1050cm$^{-1}$; $\delta_H$ ($CDCl_3$) 0.04-0.10 (18H, m, 6 x $SiCH_3$), 0.89-0.96 (30H, m, 17-$H_3$ and 3 x $^tBu$), 1.20 (6H, s, 2 x 6″-H), 2.20 (3H, s, 15-$H_3$), 3.38-3.42 (1H, m, 6-H), 3.53-3.58 (1H, m, 16-H), 6.15 (1H, s, 2-H), 6.69 (1H, s, 4′-H); δC ($CDCl_3$) -5.0 ($SiCH_3$), -4.7 ($SiCH_3$), -4.6 ($SiCH_3$), -4.4 ($SiCH_3$), -4.3 ($SiCH_3$), -3.3 ($SiCH_3$), 12.6 (C-17), 17.9 [$C(CH_3)_3$], 18.0 ([$C(CH_3)_3$], 18.1 [$C(CH_3)_3$], 18.9 (C-15), 20.7 (C-14), 25.8 [$C(CH_3)_3$], 26.0 [$C(CH_3)_3$], 26.7 (C-2″), 27.0 (C-6″), 31.0 (C-3″), 31.6 (C-9), 35.1 (C-1″), 42.1 (C-8), 42.7 (C-12), 43.0 (C-4), 48.4 (C-4″), 55.4 (C-10), 59.0 (C-11), 65.3 (C-16), 70.2 (C-6), 70.8 (C-7), 72.6 (C-5″), 73.3 (C-13), 74.3 (C-5), 112.9 (C-2), 120.7 (C-4′), 145.4 (C-5′), 155.2 (C-3), 160.4, (C-1); m/z (FAB 3-NOBA/Na) 872 [(MNa)$^+$, 42%)], 850 [(MH)$^+$, 100%].

c) 5-[trans-4-(2-Hydroxyprop-2-yl)cyclohexyl]-2-(1-normon-2-yl)oxazole

The above oxazole (0.180g, 0.212mmol) and tetra-n-butylammonium fluoride trihydrate (0.40g, 1.27mmol) in THF (20ml) were stirred at room temperature for 16h. Evaporation to dryness under reduced pressure and purification by flash chromatography using ethyl acetate/acetone (1:1) as eluent gave the title compound (0.100g, 93%) as a white foam; $\nu_{max}$ (KBr) 3419, 2926, 2859, 1655, 1450, 1380, 1111, 1050cm$^{-1}$; $\nu_{max}$ (EtOH), 206nm ($\epsilon_m$ 14,600); $\delta_H$ ($CD_3OD$) 0.91 (3H, d, J 6.3Hz, 17-$H_3$), 1.16 (6H, s, 2 x 6″-$H_3$), 1.20 (3H, d, J 6.4Hz, 14-$H_3$), 1.92-2.05 (3H, m, 2 x cyclohexyl- and 8-H), 2.10-2.19 (2H, m, cyclohexyl), 2.19 (3H, s, 15-$H_3$), 2.55-2.74 (4H, m, 4, 10, 11 and 14″-H), 6.13 (1H, s, 2-H), 6.75 (1H, s, 4′-H); $\delta_C$ ($CD_3OD$) 12.3 (C-17), 19.5 (C-15), 20.3 (C-14), 26.9 (C-6″), 27.9 (C-2″), 32.4 (C-3′), 33.0 (C-9), 36.5 (C-1″), 41.6 (C-8), 43.7 (C-4), 43.7 (C-12), 49.8 (C-4″), 56.6 (C-10), 61.2 (C-11), 66.3 (C-16), 70.0 (C-7), 71.6 (C-13), 73.1 (C-5″), 76.4 (C-5), 113.8 (C-2), 121.4 (C-4′), 147.6 (C-5′), 157.6 (C-3), 161.9 (C-1); m/z 507 (M$^+$, 18%), 245 (100%). (Found: 507.3224. $C_{28}H_{45}NO_7$ requires, 507.3196).

Example 8

5-[trans-4-(1,2-epoxyethyl)cyclohexyl]-2-(1-normon-2-yl)oxazole

a) trans-1-[(2-Bromo-1-hydroxy)ethyl]-4-methoxycarbonylcyclohexane

Sodium borohydride (0.31g, 8.12mmol) was added portionwise to a solution of trans-α-1-bromoacetyl-4-methoxycarbonylcyclohexane (15.24g, 19.9mmol) in methanol (80ml) at 0°C. After a further 15min at 0°C brine (150ml) was added and the solution extracted with dichloromethane (3 x 100ml). Drying ($MgSO_4$), evaporation to dryness under reduced pressure and purification by flash chromatography using

dichloromethane/ethyl acetate (10:1) as eluent gave the title compound (4.70g, 89%) as a colourless oil; $\nu_{max}$ (liquid film) 3500, 2960, 2850, 1720, 1430, 900cm$^{-1}$; $\delta_H$ ($CDCl_3$) 1.03-1.23 (2H, m, cyclohexyl), 1.32-1.55 (3H, m, OH and 2 x cyclohexyl), 1.70-1.93 (1H, m, 1-H), 1.95-2.17 (4H, m, cyclohexyl), 2.17-2.33 (1H, m, 4-H), 3.37-3.67 (3H, m, 2$''$-$H_2$ and 1$''$-H), 3.67 (3H, s, $CH_2$); δC ($CDCl_3$) 27.1 and 28.5 (C-2 and -6), 28.1 (C-3 and -5), 39.1 (C-2$''$), 40.4 (C-1), 43.0 (C-4), 51.6 ($CH_3$), 74.9 (C-1$''$), 176.2 (C=O); m/z ($NH_3$ C.I.) 267 ($M^+$, 60%), 265 ($M^+$, 63%), 81(100%).

b) trans-[(2-Bromo-1-t-butyldiphenylsilyloxy)ethyl]-4-methoxycarbonylcyclohexane and trans-[(2-t-butyldiphenylsilyloxy-1-chloro)ethyl]-4-methoxycarbonylcyclohexane

The above alcohol (9.80g, 37.0mmol), t-butyldiphenylsilylchloride (15.30g, 53.2mmol) and imidazole (7.50g, 110mmol) in DMF (100ml) were stirred at 60°C for 16h. Water (250ml) and ethyl acetate (250ml) were added, the organic layer separated, dried ($MgSO_4$) and evaporated to dryness under reduced pressure. Purification by flash chromatography using hexane/dichloromethane (3:2) as eluent gave the title compounds (16.66g, 90%) as a colourless oil containing a 1:1 mixture of the products; $\nu_{max}$ (liquid film) 2960, 2850, 1725, 1420, 1110, 820, 740, 700cm$^{-1}$; $\delta_H$ ($CDCl_3$) 1.11 (9H, s, $^tBu$), 1.05-1.48 (4H, m, cyclohexyl), 1.56-1.78 (2H, m, cyclohexyl), 1.79-1.90 (1H, m, 1-H), 1.92-2.08 (2H, m, cyclohexyl), 3.40 (1H, tt, J 3.4 and 11.9Hz, 4-H), 3.20-3.30 (2H, m, 2$''$-$CH_2Br$), 3.32-3.44 (2H, m, 2$''$-$CH_2Cl$), 3.64-3.69 (4H, m, 1$''$-H and $CH_3$), 7.32-7.46 (6H, m, aromatic), 7.62-7.72 (4H, m, aromatic); $\delta_C$ ($CDCl_3$) 19.6 [$C(CH_3)_3$], 25.9 and 28.1 (C-2 and -6), 27.1 [$C(CH_3)_3$], 28.7 and 28.8 (C-3 and -5), 35.0 [C-2$'$(Br)], 39.8 (C-4), 40.3 [C-1(Br)], 43.3 [C-1(Cl)], 45.7 [C-2$''$(Cl)], 51.5 ($CH_3$), 76.2 [C-1$'$(Br)], 76.7 [C-1$'$(Cl)], 127.6, 127.7, 129.8, 129.9, 133.3(q), 133.9(q), 136.0, 136.0, 176.3 (C=O); m/z (FAB 3-NOBA/Na), 525 [$(MNa)^+$, 10%(Br)], 481 [$(MNa)^+$, 100% (Cl)].

c) trans-4-[(2-Bromo-1-t-butyldiphenylsilyloxy) ethyl]cyclohexanecarboxylic acid and trans-4-[(1-t-butyldiphenylsilyloxy)-2-chloro)ethyl]cyclohexanecarboxylic acid

The above esters (5.03g, ca.10.5mmol) in lithium hydroxide (1.0M) (52.5ml, 52.5mmol), methanol (160ml) and THF (55ml) were stirred at room temperature for 16h. Water (100ml) was added and the solution evaporated to low volume under reduced pressure. The solution was brought to pH 4 using Amberlite IR 120$H^+$ resin, filtered and evaporated to dryness under reduced pressure to give the title compounds (4.68g, 95%) as a white solid; $\nu_{max}$ (KBr) 3210, 3100-2200(Br), 2930, 2854, 1699, 1114, 746, 704cm$^{-1}$; $\delta_H$ ($CDCl_3$) 1.07 (9H, s, $^tBu$), 1.07-1.47 (4H, m, cyclohexyl), 1.60-1.78 (2H, m, cyclohexyl), 1.82-1.94 (1H, m, 4-H), 1.84-2.11 (2H, m, cyclohexyl), 2.13-2.30 (1H, m, 1-H), 3.19-3.32 [2H, m, 2$'$-$H_2$(Br)], 3.32-3.44 [2H, m, 2$'$-$H_2$(Cl)], 3.62-3.69 1H, m, 1$''$-H), 7.36-7.48 (6H, m, aromatic), 7.66-7.69 (4H, m, aromatic); $\delta_C$ ($CDCl_3$) 19.6 [$C(CH_3)_3$], 25.6, 25.9, 27.1 [$C(CH_3)_3$] 28.0, 28.1, 28.4, 28.4. 28.5, 28.6, 35.0 [C-2$'$(Br)], 39.8 (C-1), 40.3 [C-4(Br)], 43.2 [C-4(Cl)], 45.7 [C2$'$(Cl)], 76.2 [C-1$'$(Br)]. 76.6 [C-1$'$(Cl)] 127.6, 127.7, 129.6, 129.9, 133.3(q), 133.9(q), 136.0, 162.3 (C=O); m/z ($CH_4$ C.I.) 489 [$(MH)^+$, 18%(Br)], 445 [$(MH)^+$, 32%(Cl)]. (Found: 387.1190. $C_{21}H_{24}O_3Si^{35}Cl$ requires, 387.1183). (Found: 431.0690. $C_{21}H_{24}O_3Si^{79}Br$ requires 431.0678).

d) trans-1-α-Bromoacetyl-4-((2-bromo-1-t-butyldiphenylsilyloxy)ethyl]cyclohexane and trans-1-α-bromoacetyl-4-[(1-t-butyldiphenylsilyloxy-2-chloro)ethyl]cyclohexane

Oxalylchloride (0.96ml, 10.94mmol) in dichloromethane (5ml) was added dropwise to a solution of the above acids in dichloromethane (30ml) over 30min. After a further 30min the solution was evaporated to dryness under reduced pressure to give a crude acid chloride as a pale yellow oil; $\nu_{max}$ (liquid film) 2950, 2560, 1800, 1425, 1105, 740, 700cm$^{-1}$.

The crude acid chloride in dichloromethane (50ml) was to diazomethane (ca.24.5mmol) in ether (ca.100ml) over 30min. After a further 1h hydrobromic acid (48%) (3.0ml) was added dropwise with rapid stirring. After 30min saturated sodium hydrogen carbonate solution (100ml) was added and the products extracted using dichloromethane (3 x 100ml). Drying ($MgSO_4$), evaporation to dryness under reduced pressure and purification by flash chromatography using hexane/ether (2:1) as eluent gave the title compounds (4.49g, ca.83%) as a colourless oil; $\nu_{max}$ (liquid film) 2940, 2860, 1720, 1425, 1105, 740, 700cm$^{-1}$; $\delta_H$ ($CDCl_3$) 1.07 (9H, s, $^tBu$), 1.07-1.47 (4H, m, cyclohexyl), 1.55-1.82 (2H, m, cyclohexyl), 1.82-1.04 (3H, m, 4-H and 2 x cyclohexyl), 2.54-2.72 (1H, m, 1-H), 3.18-3.32 [2H, m, 2$'$-$H_2$(Br)], 3.30-3.43 [2H, m,

2'-$H_2$(Cl)], 3.61-3.72 (1H, m, 1'-H), 3.94 (2H, s, $CH_2Br$), 7.34-7.50 (6H, m, aromatic), 7.63-7.66 (4H, m, aromatic); $\delta_C$ ($CDCl_3$) 19.6 [C($CH_3$)$_3$], 25.9, 26.0, 27.1 [C($CH_3$)$_3$], 28.0, 28.1, 28.3, 28.4, 28.4, 33.3 ($CH_2Br$), 34.9 [C-2'(Br)], 39.7 (C-1), 40.3 [C-4(Br)], 45.6 [C-4(Cl)], 48.0 [C-2'(Cl)], 76.0 [C-1'(Br)], 76.5 [C-1'(Cl)], 127.6, 127.7, 129.8, 129.9, 129.9, 133.2(q), 133.8(q), 136.0, 204.3 (C=O).

e) trans-1-α-Bromoacetyl-4-[(2-bromo-1-hydroxy)ethyl]cyclohexane and trans-1-α-bromoacetyl-4-[(2-chloro-1-hydroxy)ethyl]cyclohexane

The above silyl ethers (8.72g, ca.16 0mmol) were dissolved in methanol (170ml) and concentrated hydrochloric acid (10.3ml) and the solution stirred at 60°C for 60h. The solution was neutralised with saturated sodium hydrogen carbonate solution and extracted with dichloromethane (3 x 250ml). Drying ($MgSO_4$), evaporation to dryness under reduced pressure and purification by flash chromatography using dichloromethane/ethyl acetate (9:1) as eluent gave the title compounds (3.51g, ca.71%) as a white solid; $\nu_{max}$ (KBr) 3295, 2930, 2890, 2853, 1720, 1446, 1388, 763cm$^{-1}$; $\delta_H$ ($CDCl_3$) 1.05-1.73 (4H, m, OH and 3 x cyclohexyl), 1.73-1.90 (1H, m, cyclohexyl), 1.90-2.15 (3H, m, cyclohexyl), 2.15-2.27 (1H, m, 4-H), 2.62 (1H, tt, J 12.0 and 3.2Hz, 1-H), 3.44-3.72 (3H, m, 2'-$H_2$ and 1'-H), 4.17 (2H, s, $CH_2Br$); $\delta_C$ ($CDCl_3$) 27.9, 27.9, 28.0, 28.1, 39.0 [C-2'(Br)], 40.3 (C-1), 41.1 [C-4(Br)], 47.2 ($CH_2Br$), 47.4 [C-4(Cl)], 48.7 [C-2'(Cl)], 74.8 [C-1'-(Br)], 75.1 [C-1'(Cl)]; m/z ($NH_3$ C.I.) 344 [$M^+$, 2% ($Br_2$)], 258 [$M^+$, 98% (BrCl)], 258 [$M^+$, 62% (BrCl)], 107-(100%).

f) 1-α-Azidoacetyl-4-[(2-bromo-1-hydroxy)ethyl]cyclohexane and 1-α-azidoacetyl-4-[(2-chloro-1-hydroxy)-ethyl]cyclohexane

The above α-bromoketones (2.05g, ca.6.70mmol) and tetramethylguanidinium azide (1.06g, 6.70mmol) in chloroform (35ml) were stirred at room temperature for 16h. Evaporation to dryness under reduced pressure and purification by flash chromatography using dichloromethane/ethyl acetate (9:1) as eluent gave the title compounds (1.94g, 84%) as a colourless oil; $\nu_{max}$ (liquid film) 3350, 2940, 2800, 2210, 1710, 1280cm$^{-1}$; $\delta_H$ ($CDCl_3$) 1.07-1.61 (5H, m, OH and 4 x cyclohexyl), 1.73-1.90 (1H, m, cyclohexyl), 1.90-2.21 (4H, m, 4H and 3 x cyclohexyl), 2.38 (1H, tt, J 12.2 and 3.5Hz, 1-H), 3.43-3.74 (3H, m, 2'-$H_2$ and 1'-H), 4.00 (2H, s, $CH_2N_3$); $\delta_C$ ($CDCl_3$) 27.7, 27.7, 25.0, 28.1, 39.4 [C-2'(Br)], 40.3 (C-1), 41.1 [C-4(Br)], 48.0 [C-4(Cl)], 48.9 (C-2'(Cl)], 56.0 ($CH_2N_3$), 74.8 [C-1'(Br)], 75.1 [C-1'(Cl)], 207.0 (C=O); m/z ($NH_3$, C.I.) 309 [($MNH_4$)$^+$, 4%($^{81}$Br)], 307 [($MNH_4$)$^+$, 4%($^{79}$Br)], 218(100%).

g) N-[2-Oxo-2-[(trans-4-(2-bromo-1-hydroxy)ethyl)cyclohexyl]ethyl]-(6,7,13-tris-t-butyldimethylsilyloxy)-monamide and N-[2-oxo-2[(trans-4-(2-chloro-1-hydroxy)ethyl)-cyclohexyl]ethyl]-[6,7,13-tris-t-butyldimethyl-silyloxy)monamide

The above α-azidoketones (1.84g, ca.6.87mmol) in methanol (100ml), water (50ml) and concentrated hydrochloric acid (5.7ml) were hydrogenated at 1 atm. using 10% Pd on C (0.126g) as catalyst for 30min. Filtration and evaporation to dryness under reduced pressure gave the crude amine hydrochloride salts as a white foam. 6,7,13-tris-t-Butyldimethylsilyloxymonic acid (4.97g, 7.25mmol) in THF (50ml) was treated sequentially with i-butyl chloroformate (0.94ml, 7.25mmol) and triethylamine (1.11ml, 7.98mmol) at 0°C. After 1h at 0-5°C the crude amine hydrochloride salts in water (15ml) and THF (15ml) were added followed by triethylamine (1.11ml, 7.98mmol). After 2.5h at 0-5°C ethyl acetate (100ml) and brine (50ml) were added, the organic layer separated, the aqueous phase extracted with ethyl acetate (2 x 100ml) and the combined organic extracts dried ($MgSO_4$). Evaporation to dryness under reduced pressure and purification by flash chromatography using hexane/ethyl acetate (3:2) as eluent gave the title compounds (3.19g, 60%) as a white foam; $\nu_{max}$ (KBr) 3298, 2954, 2930, 2857, 1718, 1662, 1629, 1507, 1255, 1123, 837, 775cm$^{-1}$; $\delta_H$ -($CDCl_3$), 0.03-0.09 (18H, m, 6 x $SiCH_3$), 0.89-0.93 (30H, m, 17-$H_3$ and 3 x $^t$Bu), 1.19 (3H, d. J 6.3Hz, 14-$H_3$), 2.15 (3H, s, 15-$H_3$), 2.37 (1H, tt, J 12.0 and 3.1Hz, 1''-H), 3.34-3.38 (1H, m, 6H), 3.43-3.74 (4H, m, 6''-$H_2$), 16-H and 5''-H), 4.23 (2H, d, J 4.2Hz, 1'-$H_2$), 5.70 (1H, s, 2-H), 6.18 (1H, t, J 4.2Hz, NH); $\delta_C$ ($CDCl_3$), -4.9 ($SiCH_3$), -4.6 ($SiCH_3$), -4.5 ($SiCH_3$), -4.3 ($SiCH_3$), -4.2 ($SiCH_3$), -3.3 ($SiCH_3$), 12.7 (C-17), 18.0 [C($CH_3$)$_3$], 18.1 [C($CH_3$)$_3$], 18.3 (C-15), 20.8 (C-14), 25.9 [C($CH_3$)$_3$], 26.1 [C($CH_3$)$_3$], 27.9, 28.0, 28.1, 28.1, 31.9 (C-9), 38.8 [C-6''(Br)], 40.3 (C-1''), 41.1 [C-4''(Br)], 42.2 (C-8), 43.0 (C-12), 43.1 (C-4), 47.5 (C-1'), 48.5 [C-4''(C-1)], 48.7 [C-6''(C-1)], 55.5 (C-10), 59.0 (C-11), 65.4 (C-16), 70.3 (C-6), 70.8 (C-7), 73.4 (C-13), 74.0 (C-5), 74.7

[C-5''(Br)], 75.1 [C-5''Cl)], 119.2 (C-2), 152.8 (C-3), 166.9 (C-1), 208.3 (C-2'); m/z (FAB 3-NOBA/Na) 956 [-(MNa)$^+$, 7%(Br)], 910 [(MNa)$^+$, 12% (Cl)], 159(100%).

h) 5-[trans-4-(1,2-Epoxyethyl)cyclohexyl]-2-(1-nor-6,7,13-tris-t-butyldimethylsilyloxymo-2-yl)oxazole

Pyridine (3.44ml, 42.4mmol) and trichloroacetylchloride (3.80ml, 33.94mmol) were added sequentially to a solution of the monamides (7.72g, 8.48mmol) and N,N-dimethylaminopyridine (catalytic amount) in dichloromethane (60ml) at 0°C. After 1h at 0°C, dichloromethane (250ml) was added and the solution washed with water, saturated sodium hydrogencarbonate solution and brine. After drying ($MgSO_4$) and evaporation to dryness under reduced pressure the products were dissolved in methanol (40ml) and potassium carbonate (3.52g, 25.5mmol) was added. After stirring at room temperature for 16h pH 7 buffer solution (200ml) was added and the resulting solution extracted with dichloromethane (3 x 200ml). Drying ($MgSO_4$), evaporation to dryness under reduced pressure and purification by flash chromatography using ether/hexane (3:2) as eluent gave the title compound (2.95g, 42%) as a colourless foam; $\nu_{max}$ (KBr) 2955, 2931, 2857, 1657, 1124, 837, 775cm$^{-1}$; $\delta_H$ ($CDCl_3$) 0.04-0.08 (18H, m, 6 x $SiCH_3$), 0.83-0.93 (30H, m, 17-$H_3$ and 3 x $^t$Bu), 1.16 (3H, d, J 6.3Hz, 14-$H_3$), 2.15 (3H, s, 15-$H_3$), 3.31 (1H, dd, J 9.1 and 1.8Hz, 6-H). 3.47 (1H, d, J 11.0Hz, 16-H), 3.74-3.83 (4H, m, 5, 7, 13 and 16-H), 6.14 (1H, s, 2-H), 6.70 (1H, s, 4'-H); $\delta_C$ ($CDCl_3$), -4.9 ($SiCH_3$), -4.6 ($SiCH_3$), -4.5 ($SiCH_3$), -4.3 ($SiCH_3$), -4.2 ($SiCH_3$), -3.3 ($SiCH_3$), 12.7 (C-17), 18.0 [C($CH_3$)$_3$], 18.1 [C($CH_3$)$_3$], 18.2 [C($CH_3$)$_3$], 19.0 (C-15), 20.8 (C-14), 25.9 [C($CH_3$)$_3$], 26.1 [C($CH_3$)$_3$], 28.1, 28.8, 30.1, 30.2, 30.3, 32.0 (C-9), 35.1 (C-1''), 39.8 (C-4''), 42.2 (C-8), 43.0 (C-12), 43.1 (C-4), 45.8 (C-6''), 55.5 (C-10), 56.3 (C-5''), 59.0 (C-11), 65. (C-16), 70.4 (C-6), 70.9 (C-7), 73.5 (C-13), 74.4 (C-5), 113.0 (C-2), 121.1 (C-4'), 145.6 (C-5'), 154.9 (C-3), 160.5 (C-1); m/z (FAB 3-NOBA/Na) 856 [(MNa)$^+$, 30%], 834 [(MH)$^+$, 37%], 159-(100%).

i) 5-[trans-4-(1,2-Epoxyethyl)cyclohexyl]-2-(1-normon-2-yl)oxazole

The above epoxide (0.100g, 0.12mmol) in THF (30ml) was treated with tetra-n-butylammonium fluoride trihydrate (0.24g, 0.76mmol) for 40h at room temperature. Evaporation to dryness under reduced pressure and purification by flash chromatography using methyl acetate as eluent gave the title compound (0.043g, 73%) as a white foam; $\nu$max (KBr) 3426, 2928, 2867, 1655, 1449, 1111, 857cm$^{-1}$; $\lambda_{max}$ (EtOH) 226nm ($\epsilon_m$ 17,955); $\delta_H$ ($CDCl_3$) 0.93 (3H, d, J 7.1Hz, 17-$H_3$), 1.21 (3H, d, J 6.1Hz, 14-$H_3$), 2.21 (3H, s, 15-$H_3$), 2.36 (1H, dd, J 14.5 and 8.7Hz, 4''-H), 3.44-3.62 (2H, m, 6 and 16-H), 3.72-3.95 (4H, m, 5,7,13 and 16-H), 6.16 (1H, s, 2-H), 6.70 (1H, s, 4'-H); $\delta_C$ ($CDCl_3$) 12.7 (C-17), 19.4 (C-15), 20.8 (C-14), 28.1, 28.8, 30.2, 30.2, 31.7 (C-9), 35.1 (C-1''), 39.5 (C-4'), 39.8 (C-8), 42.8 (C-12), 42.8 (C-4), 45.9 (C-6''), 55.6 (C-5''), 56.3 (C-10), 61.3 (C-11), 65.5 (C-16), 68.9 (C-6), 70.4 (C-7), 71.2 (C-13), 75.2 (C-5), 113.4 (C-2), 120.9 (C-4'), 145.3 (C-5'), 155.5 (C-3), 160.5 (C-1); m/z 491 (M$^+$, 14%), 247(100%). (Found: 491.2894. $C_{27}H_{41}NO_7$ requires 491.2884).

Example 9

5-[trans-4-(2-Azido-1-hydroxyethyl)cyclohexyl]-2-(1-normon-2-yl)oxazole

a) 5-[trans-4-(2-Azido-1-hydroxyethyl)cyclohexyl]-2-(1-nor-6,7,13-tris-t-butyldimethylsilyloxy-mon-2-yl)-oxazole

Example 8 (0.150g, 0.18mmol) and tetramethylguanidinium azide (0.068g, 0.44mmol) in chloroform (5ml) were heated to reflux for 60h. Evaporation to dryness under reduced pressure and purification by flash chromatography using ether:hexane (3:2) as eluent gave the title compound (0.115g, 73%) as a white foam; $\nu_{max}$ (KBr) 3436, 2954, 2930, 2857, 2100, 1471, 1256, 1123, 1084, 837, 775cm$^{-1}$; $\delta_H$ ($CDCl_3$) 0.04-0.10 (18H, m, 6 x $SiCH_3$), 0.89-0.93 (30H, m, 17-$H_3$ and 3 x $^t$Bu), 1.19 (3H, d, J 6.4Hz, 14-$H_3$), 2.20 (3H, s, 15-$H_3$), 2.52-2.74, (4H, m, 4-H, 10-H, 11-H and 1''-H), 3.31-3.62 (5H, m, 6-H, 16-H, 6''-$H_2$, 5''-H), 3.83-3.93 (4H, m, 5-H, 7-H, 13-H, 16-H), 6.14 (1H, s, 2-H), 6.70 (1H, s, 4'-H); $\delta_C$ ($CDCl_3$) -4.9 ($SiCH_3$), -4.6 ($SiCH_3$), -4.5 ($SiCH_3$), -4.2 ($SiCH_3$), -3.2 ($SiCH_3$), 12.7 (C-1), 18.1 [C($CH_3$)$_3$], 18.1 [C($CH_3$)$_3$], 18.2 [C($CH_3$)$_3$], 190 (C-15), 20.8 (C-14), 25.9 [C($CH_3$)$_3$], 26.1 [C($CH_3$)$_3$], 27.7, 28.4, 30.5, 30.5, 30.6, 30.6, 32.0 (C-9), 35.1 (C-1''), 40.8

(C-4'), 42.2 (C-8), 43.0 (C-12), 43.2 (C-4), 55.2 (C-6"), 55.5 (C-10), 59.1 (C-11), 65.5 (C-16), 70.4 (C-6), 70.9 (C-7), 73.5 (C-13), 74.4 (C-5), 74.6 (C-5"), 113.0 (C-2), 121.1 (C-4), 145.7 (C-5'), 155.0 (C-3), 200.6 (C-1); m/z (FAB 3-NOBA/Na) 900 [(MNa)$^+$, 7%) 877 [(MH)$^+$, 13%], 115 (100%).

b) 5-[trans-4-(2-Azido-1-hydroxyethyl)cyclohexyl]-2-(1-normon-2-yl)oxazole

The above oxazole (0.150g, 0.17mmol) and tetra-n-butylammonium fluoride trihydrate (0.33g, 1.06mmol) in THF (10ml) were stirred at room temperature for 48h. Evaporation to dryness under reduced pressure and purification by flash chromatography using methyl acetate as eluent gave the title compound (0.061g, 67%), as a white foam; $\nu_{max}$ (KBr) 3421, 2927, 2860, 2100, 1654, 1597, 1449, 1274, 1111, 1051cm$^{-1}$; $\lambda_{max}$ - (EtOH) 265nm ($\epsilon_m$ 17,400); $\delta_H$ ($CDCl_3$), 0.93 (3H, d, J 7.0Hz, 17-$H_3$), 1.22 (3H, d, J 6.3Hz, 14-$H_3$), 2.23 (3H, s, 15-$H_3$), 2.28 (1H, dd, J 6.6 and 14.5Hz, 4-H), 2.54-2.64 (2H, m, 4-H and 1"-H), 2.71 (1H, dd, J 2.2 and 7.9Hz, 11-H), 2.78-2.82 (1H, m, 10-H), 3.32-3.54 (5H, m, 6-H, 16-H, 6"-$H_2$ and 5"-H), 3.74-3.91 (4H, m, 5-H, 7-H, 13-H and 16-H), 6.17 (1H, s, 2-H), 6.70 (1H, 4'-H); $\delta_C$ ($CDCl_3$) 12.7 (C-17), 19.4 (C-15), 20.8 (C-14), 27.4, 28.3, 30.5, 30.5, 31.7 (C-9), 35.1 (C-1"), 39.5 (C-4"), 40.8 (C-8), 42.7 (C-4), 42.8 (C-12), 55.1 (C-6"), 55.6 (C-10), 61.3 (C-11), 65.5 (C-16), 68.9 (C-6), 70.4 (C-7), 71.2 (C-7), 71.2 (C-13), 74.6 (C-5"), 75.2 (C-5), 113.3 (C-2), 120.8 (C-4'), 145.5 (C-5'), 155.5 (C-3), 160.5 (C-1); m/z (FAB 3-NOBA/Na) 557 [(MNa)$^+$, 43%], 535 [(MH$^+$), 13%] 176 (100%).

Example 10

5-[trans-4-(1,3-dioxolan-2-yl)cyclohexyl]-2-(1-normon-2-yl)oxazole

a) 5-[trans-4-Hydroxymethylcyclohexyl]-2-(1-nor-6,7,13-tris-t-butyldimethylsilyloxy-mon-2-yl)oxazole

5-[trans-4-Methoxycarbonylcyclohexyl]-2-(1-nor-6,7,13-tris-t-butyldimethylsilyloxy-mon-2-yl)oxazole (example 7a) (1.08g, 1.20mmol) in dichloromethane at 0°C was treated with diisobutylaluminiumhydride (1M in toluene) (2.42ml, 2.42mmol). After 2h at 0°C methanol (5ml) and saturated sodium sulphate solution (2ml) were added. After 20min the precipitate was removed by filtration, the filtrate dried ($MgSO_4$) and evaporated to dryness under reduced pressure. Purification by flash chromatography using ether:hexane (3:2) as eluent gave the title compound(0.68g, 65%) as a white foam; $\nu_{max}$ ($CH_2Cl_2$) 3420, 2950, 2860, 1660, 1470, 1460, 1255, 1084, 965, 900, 837, 774cm$^{-1}$; $\delta_H$ ($CDCl_3$) 0.04-0.10 (18H, m, 6 x $SiCH_3$), 0.89-0.93 (30H, m, 17-$H_3$ and 3 x $^t$Bu), 1.19 (3H, d, J 6.3Hz, 14-$H_3$), 2.20 (3H, s, 15-$H_3$), 3.37-3.40 (1H, m, 6-H), 6.14 (1H, s, 2-H), 6.76 (1H, s, 4'-H); $\delta_C$ ($CDCl_3$) -4.9 ($SiCH_3$), -4.6 ($SiCH_3$), -4.5 ($SiCH_3$), -4.3 ($SiCH_3$), -4.2 ($SiCH_3$), -3.2 ($SiCH_3$), 12.7 (C-17), 18.0 [$C(CH_3)_3$], 18.1 [$C(CH_3)_3$], 18.2 [$C(CH_3)_3$], 19.0 (C-15), 20.8 (C-14), 25.7 [$C(CH_3)_3$], 26.1 [$C(CH_3)_3$], 28.7 (C-2", 30.5 (C-3"), 32.0 (C-9), 35.4 (C-1'), 40.0 (C-4"), 42.2 (C-8), 43.0 (C-12), 43.1 (C-4), 55.5 (C-10), 59.1 (C-11), 65.5 (C-16), 68.2 (C-5"), 70.4 (C-6), 70.9 (C-7), 73.5 (C-13), 74.4 (C-5), 113.1 (C-2), 121.0 (C-4'), 145.6 (C-5'), 155.3 (C-3), 160.5 (C-1); m/z (FAB 3-NOBA/Na) 844 [(MNa)$^+$, 18%], 822 [-(MH)$^+$, 20%], 159 (100%).

b) 5-[trans-4-Formylcyclohexyl]-2-(1-nor-6,7,13-tris-t-butyldimethylsilyloxy-mon-2-yl)oxazole

The above alcohol (1.09g, 1.33mmol) in dichloromethane (10ml) was added to a rapidly stirring solution of pyridinium chlorochromate (0.57g, 2.66mmol) and powdered 3A molecular sieves (0.67g) in dichloromethane (10ml). After 0.5h at room temperature the products were poured into ether (500ml). The precipitated solids were removed by filtration and the filtrate evaporated to dryness under reduced pressure. Purification by flash chromatography using ether:hexane (2:3) as eluent gave the title compound (0.56g, 52%) as a white foam; $\nu_{max}$ (KBr) 2928, 2857, 2708, 1726, 1656, 1460, 1255, 1123, 1084, 964, 936, 775cm$^{-1}$; $\delta_H$ ($CDCl_3$) 0.04-0.10 (18H, m, 6 x $SiCH_3$), 0.89-0.91 (30H, m, 17-$H_3$ and 3 x $^t$Bu), 1.19 (3H, d, J 6.3Hz, 14-$H_3$), 2.21 (3H, s, 15-$H_3$), 2.54-2.72 (4H, m, 4-H, 10-H, 11-H and 1"-H), 3.40 (1H, dd, J 9.1 and 1.8Hz, 6-H), 2.56 (1H, d, J 11.2Hz, 10-H), 3.82-3.94 (4-H, m, 5-H, 7-H, 13-H and 16-H), 6.15 (1H, s, 2-H), 6.69 (1H, s, 4-H), 9.67 (1H, d,m J 1.0Hz, 5"-H); $\delta_C$ ($CDCl_3$) -4.9 ($SiCH_3$), -4.6 ($SiCH_3$), 4.5 ($SiCH_3$), -4.3 ($SiCH_3$), -4.2 ($SiCH_3$), -3.2 ($SiCH_3$), 12.7 (C-17), 18.1 [$C(CH_3)_3$], 18.1 [$C(CH_3)_3$], 18.2 [$C(CH_3)_3$], 19.0 (C-15),

20.8 (C-14), 25.4 (C-3″), 25.9 [$C(CH_3)_3$], 26.1 [$C(CH_3)_3$], 29.8 (C-2″), 32.0 (C-9), 34.7 (C-1″), 42.2 (C-8), 43.0 (C-12), 43.1 (C-4), 49.6 (C-4″), 55.5 (C-10), 59.0 (C-11), 65.5 (C-16), 70.4 (C-6), 70.9 (C-7), 73.5 (C-13), 74.4 (C-5), 113.0 (C-2), 121.4 (C-4′), 145.9 (C-5′), 154.3 (C-3), 160.7 (C-1), 203.8 (C-5″); m/z (FAB 3-NOBA/Na) 842 [$(MNa)^+$, 12%], 820 [$(MH)^+$, 9%], 159 (100%).

c) 5-[trans-4-(1,3-Dioxolan-2-yl)cyclohexyl]-2-(1-nor-6,7,13-tris-t-butyldimethylsilyloxy-mon-2-yl)oxazole

The above aldehyde (0.150g, 0.183mmol), ethylene glycol (0.057g, 0.915mmol) and pyridinium p-toluenesulphonate (0.013g, 0.052mmol) in benzene (3ml) were heated to reflux using a Dean and Stark apparatus for 16h. Evaporation to dryness under reduced pressure followed by purification by flash chromatography using hexane:ether (3:2) as eluent gave the title compound (0.051g, 32%) as a viscous colourless oil; $\nu_{max}$ (liquid film) 2930, 2875, 1655, 1450, 1050$cm^{-1}$; $\delta_H$ ($CDCl_3$) 0.04-0.10 (18H, m, 6 x $SiCH_3$), 0.86-0.92 (30H, m, 3 x $^tBu$ and 17-$H_3$), 1.19 (3H, d, J 6.3Hz, 14-$H_3$), 2.20 (3H, s, 15-$H_3$), 2.52-2.71 (4H, m, 10-H, 11-H, 4-H and 1″-H), 3.39 (1H, dd, J 9.1 and 1.8Hz, 6-H), 3.55 (1H, d, J 11.3Hz, 16-H), 3.83-4.00 (8H, m, 5-H, 7-H, 13-H, 6-H and 2 x 6″-$H_2$), 4.65 (1H, d, J 4.8Hz, 5″-H), 6.14 (1H, s, 2-H), 6.70 (1H, s, 4′-H); $\delta_C$ ($CDCl_3$) -4.9 ($SiCH_3$), -4.6 ($SiCH_3$), -4.5 ($SiCH_3$), -4.3 ($SiCH_3$), -4.2 ($SiCH_3$), -3.2 ($SiCH_3$), 12.7 (C-17), 18.1 [$C(CH_3)_3$], 18.1 [$C(CH_3)_3$], 18.2 [$C(CH_3)_3$], 19.0 (C-15), 20.8 (C-14), 25.9 [$C(CH_3)_3$], 26.1 [$C(CH_3)_3$], 26.5 (C-2″), 30.3 (C-3″), 30.3 (C-3″), 31.9 (C-9), 35.2 (C-1″), 41.2 (C-4″), 42.2 (C-8), 43.0 (C-12, 43.1 (C-4), 55.5 (C-10), 59.1 (C-11), 65.0 (C-6″), 65.5 (C-16), 70.4 (C-6), 70.9 (C-7), 73.5 (C-13), 74.4 (C-5), 107.2 (C-5″), 113.1 (C-2), 121.1 (C-4′), 145.6 (C-5′), 155.2 (C-3), 160.5 (C-1); m/z (FAB 3-NOBA/Na) 864 ($MH^+$, 49%), 886 ($MNa^+$, 32%).

d) 5-[trans-4-(1,3-Dioxolan-2-yl)cyclohexyl]-2-(1-normon-2-yl)oxazole

The above oxazole (0.130g, 0.15mmol) and tetra-n-butylammonium fluoride trihydrate (0.28g, 0.88mmol) in THF (10ml) were stirred at room temperature for 40h. Evaporation to dryness under reduced pressure and purification by flash chromatography using 0-10% methanol in dichloromethane as eluent, and recolumning using methyl acetate as eluent gave the title compound (0.051g, 63%) as a white foam; $\nu_{max}$ (KBr) 3425, 2932, 2875, 1654, 1450, 1111, 1052, 900$cm^{-1}$; $\lambda_{max}$ (EtOH) 165nm ($\epsilon_m$ 15,740); $\delta_H$ ($CDCl_3$) 0.93 (3H, d, J 7.0Hz, 17-$H_3$), 1.22 (3H, d, J 6.4Hz, 14-$H_3$), 2.23 (3H, s, 15-$H_3$), 2.71 (1H, dd, J 1.1 and 7.8Hz, 11-H), 2.81 (1H, tt, J 2.2 and 4.7Hz, 10-H), 3.50-3.58 (2H, m, 6-H and 16-H), 3.74-3.99 (8H, m, 3-H, 7-H, 13-H, 6-H and 2 x 6″-$H_2$), 4.66 (1H, d, J 4.8Hz, 5″-H), 6.19 (1H, s, 2-H), 6.71 (1H, s, 4′-H); $\delta_C$ ($CDCl_3$), 12.6 (C-17), 19.3 (C-15), 20.8 (C-14), 26.4 (C-3″), 30.2 (C-2″), 30.3 (C-9), 35.2 (C-1″), 39.5 (C-4″), 41.1 (C-8), 42.7 (C-12), 4.7 (C-4), 55.6 (C-10), 61.2 (C-11), 65.0 (C-6″), 65.5 (C-16), 68.8 (C-6), 70.3 (C-7), 71.1 (C-13), 75.2 (C-5), 107.2 (C-5″), 113.3 (C-2), 120.7 (C-4′), 145.4 (C-5′), 155.7 (C-3), 160.4 (C-1); m/z 521 ($M^+$, 15%), 277 (100%) (Found: $M^+$, 521.2998. $C_{28}H_{43}NO_8$ requires M, 521.2989).

Example 11

5-[4-Oxocyclohexyl]-2-(1-normon-2-yl)oxazole

a) cis- and trans-Methyl-5-(4-t-butyldiphenylsilyloxycyclohexyl)oxazole-4-carboxylate

A solution of 4-t-butyldiphenylsilyloxycyclohexane carboxylic acid (10.92g, 28.6mmol) in N,N-dimethylformamide (50ml) under argon at -15°C was sequentially treated with diethyl cyanophosphonate (5.35g, 4.98ml, 32.9mmol) and triethylamine (3.05g, 4.20ml, 30.2mmol). After 40min a solution of methyl isocyanoacetate (2.83g, 2.60ml, 28.6mmol) and triethylamine (9.94g, 11.93ml, 85.8mmol) in N,N-dimethylformamide (10ml) was added dropwise. After 2h at -15°C the reaction mixture was allowed to warm to room temperature over 1h. Water (100ml) and ethyl acetate (100ml) were added, the organic phase separated and the aqueous phase extracted with ethyl acetate (2 x 150ml). The combined organic phase was dried ($MgSO_4$) and evaporated to dryness under reduced pressure. Purification by flash chromatogaphy using hexane:ethyl acetate (4:1) as eluent and rechromatography using 0-5% methanol in dichloromethane as eluent gave the title compound (3.12g, 31%) as a colourless oil; $\delta_H$ ($CDCl_3$) 1.11 (9H, s, $^tBu$), 3.34-3.72 [m,

4-H (trans-) and 1-H (cis- and trans-)], 3.90 (3H, s, $OCH_3$), 4.04-4.13 [m, 4-H (cis-)], 7.32-7.48 (6H, m, aromatic), 7.62-7.71 (5H, m, 2-H and 4 x aromatic-H); $\delta_C$ ($CDCl_3$), 19.1 [$C(CH_3)_3$], 19.4 [C-2′ (cis-)], 27.0 [C-($CH_3)_3$], 27.1 [$C(CH_3)_3$], 28.9 [C-2′ (trans-)], 33.7 [C-3′ (cis-)], 34.4 (C-1′), 35.2 (C-3′ (trans-)], 51.9 ($OCH_3$), 66.6 [C-4 (cis-)], 71.4 [C-4 (trans-)], 127.5, 127.6, 129.6, 129.7, 134.5 (q), 134.6 (q), 135.5, 168.7 (C-2), 148.8 (C-2), 162.4 (C-5), 162.6 (C-5), 163.1 (C-4), 163.9 (C-4), 180.7 (C=O); m/z (Cl. isobutane) 466 [$(M+H)^+$, 5%], 465 ($M^+$, 12%) and 208 (100%). (Found: $M^+$, 406.1493. $C_{23}H_{24}NO_4Si$ (M-$^t$Bu) requires, 406.1475).

b) cis- and trans-N-[2-Oxo-2-(4-hydroxycyclohexyl-1-yl)ethyl] (6,7,13-tris-t-butyldimethylsilyloxy)monamide

The above oxazole (4.00g, 8.39mmol) in water (4ml) and concentrated hydrochloric acid (4ml) was heated to 100°C for 5h. Water (200ml) was added and the solution washed with ether (2 x 100ml). The aqueous phase was evaporated to dryness under reduced pressure to give the crude amine hydrochloride salt. 6,7-13-tris-t-Butyldimethylsilyloxymonic acid (6.33g, 9.23mmol) in THF (125ml) was treated sequentially with isobutyl chloroformate (1.26g, 120ml, 9.22mmol) and triethylamine (1.02g, 1.40ml, 10.1mmol) at 0°C. After 1h at 0-5°C (the crude amine hydrochloride salt from above in water (10ml), THF (15ml) and triethylamine (0.85g, 1.17ml, 8.39mmol) were added. After 2h at 0°C, ethyl acetate (200ml) and water (100ml) were added, the organic layer separated, the aqueous phase extracted with ethyl acetate (2 x 100ml) and the combined organic extracts dried ($MgSO_4$). Evaporation to dryness under reduced pressure and purification by flash chromatography using ethyl acetate/hexane (3:1) as eluent gave the title compound (3.84g, 55%) as a white foam containing an unidentifiable impurity; $\nu_{max}$ (KBr), 3417, 2954, 2929, 2857, 1713, 1638, 1471, 1460, 1255, 837, 775cm$^{-1}$; $\delta_H$ ($CDCl_3$) 0.04-0.10 (18H, m, 6 x $SiCH_3$), 0.89-0.92 (30H, m, 3 x $^t$Bu and 17-$H_3$), 1.22 (3H, d, J 6.3Hz, 14-$H_3$), 2.16 (s, 15-$H_3$), 2.19 (s, 15-$H_3$), 4.13 (d, J 4.8Hz, 1″-$H_2$), 4.22 (d, J 4.5Hz, 1″-$H_2$), 5.69 (s, 2-H), 5.77 (s, 2-H), 6.13-6.18 (1H, m, N-H); $\delta_C$ ($CDCl_3$) -4.9 ($SiCH_3$), -4.6 ($SiCH_3$), -4.5 ($SiCH_3$), -4.3 ($SiCH_3$), -4.2 ($SiCH_3$), -3.3 ($SiCH_3$), 12.7 (C-17), 18.0 [$C(CH_3)_3$], 18.1 [$C(CH_3)_3$], 18.3 (C-15), 20.5 (C-14), 22.7 [C-2″ (cis-)], 25.9 [$C(CH_3)_3$], 26.0 [$C(CH_3)_3$], 26.3 [C-2″ (trans-)], 31.9 [C-9 and C-3″ (cis)], 34.4 [C-3″ (trans-)], 42.2 (C-8), 4.0 (C-12), 43.1 (C-4), 47.3 (C-1″), 47.4 (C-1), 55.5 (C-10), 59.0 (C-11), 65.4 [C-4″ (cis-)], 69.6 [C-4″ (trans-)], 70.4 (C-6), 70.8 (C-7), 73.4 (C-13), 74.0 (C-5), 119.3 (C-2), 153.5 (C-3), 167.0 (C-1), 207.2 (C-7′); m/z (FAB 3-NOBA/Na) 848 (MNa, 52%), 826 ($MH^+$, 3%) and 159 (100%).

c) 5-[4-Oxocyclohexyl]-2-(1-nor-6,7)13-tris-t-butyldimethylsilyloxy-mon-2-yl)oxazole

Pyridine (2.00g, 2.05ml, 25.3mmol) and trichloroacetylchloride (3.60g, 2.21ml, 19.9mmol) were added sequentially to a solution of the above monamide (3.84g, 4.65mmol) and N,N-dimethylaminopyridine (catalytic amount) in dichloromethane (30ml) at 0°C. After 2h at 0°C pH 7 buffer solution (50ml) was added, the organic phase separated, the aqueous phase extracted with dichloromethane (2 x 50ml) and the combined organic extracts dried ($MgSO_4$). The products were evaporated to dryness under reduced pressure and dissolved in methanol (25ml) and potassium carbonate (0.64g, 4.65mmol) added at 0°C. After 1h at 0°C the solution was warmed to room temperature for 2h. Ethyl acetate (100ml) and pH 7 buffer solution (100ml) were added, the organic phase separated, the aqueous phase extracted with ethyl acetate (3 x 100ml) and the combined organic extracts dried ($MgSO_4$). Evaporation to dryness under reduced pressure and purification by flash chromatography using ether/hexane (3:1) as eluent gave the impure 4-hydroxycyclohexyloxazole (1.93g, 51%). The crude oxazole (1.89g, 2.34mmol) was dissolved in dichloromethane (10ml) and added to a rapidly stirring suspension of pyridinium chlorochromate (1.01g, 4.68mmol) and 3A molecular sieves (1.00g) in dichloromethane (10ml). After 1h at room temperature the products were poured into ether (500ml), and filtered. Evaporation to dryness under reduced pressure and purification by flash chromatography using ether/hexane (2:1) as eluent gave the title compound (1.134g, 60%) as a viscous oil; $\delta_H$ ($CDCl_3$) 0.04-0.10 (18H, m, $SiCH_3$), 0.86-0.92 (30H, m, 3 x $^t$Bu and 17-$H_3$), 1.19 (3H, d, J 6.3Hz, 14-$H_3$), 2.20 (3H, s, 15-$H_3$), 3.16 (1H, tt, J 10.7 and 3.7Hz. 1″-H), 3.40 (1H, dd, J 2.0 and 9.1Hz, 6-H), 3.56 (1H, d, J 11.2Hz, 16-H), 3.82-4.01 (4H, m, 5-H, 7-H, 13-H and 16-H), 6.17 (1H, s, 2-H), 6.81 (1H, s, 4′-H); $\delta_C$ ($CDCl_3$) -4.9 ($SiCH_3$), -4.6 ($SiCH_3$), -4.5 ($SiCH_3$), -4.3 ($SiCH_3$), -4.2 ($SiCH_3$), -3.2 ($SiCH_3$), 12.7 (C-17), 18.1 [$C(CH_3)_3$], 18.1 [$C(CH_3)_3$], 18.2 [$C(CH_3)_3$], 19.1 (C-15), 20.8 (C-14), 25.9 [$C(CH_3)_3$], 26.1 [$C(CH_3)_3$], 30.5 (C-2″), 32.0 (C-9), 3.3 (C-1″), 40.1 (C-3″), 42.2 (C-8), 43.0 (C-12), 43.1 (C-4), 55.5 (C-10), 59.0 (C-11), 65.5 (C-16), 70.4 (C-6), 70.9 (C-7), 73.5 (C-13), 74.4 (C-5), 112.8 (C-2), 122.2 (C-4′), 146.5 (C-5′), 152.5 (C-3), 170.1 (C-1), 209.9 (C-4″); m/z (FAB 3-NOBA/Na) 82.8 ($MNa^+$, 59%), 806 ($MH^+$, 23%), 159 (100%).

d) 5-[4-Oxocyclohexyl]-2-(1-normon-2-yl)oxazole

The above oxazole (0.09g, 0.11mmol) and tetra-n-butylammonium fluoride trihydrate (0.44g, 1.4mmol) in THF (15ml) were stirred at room temperature for 24h. Evaporation to dryness under reduced pressure and purification by flash chromatography using methyl acetate as eluent gave the title compound (0.017g, 33%) as a white foam; $\delta_H$ ($CDCl_3$) 0.93 (3H, d, J 7.0Hz, 17-$H_3$), 1.21 (3H, d, J 6.3Hz, 14-$H_3$), 1.71-1.75 (2H, m, 9-$H_2$), 2.23 (3H, s, 15-$H_3$), 3.16 (1H, tt, J 10.4 and 3.6Hz, 1″-H), 3.50 (1H, dd, J 8.4 and 3.0Hz, 6-H), 3.56 (1H, dd, J 2.2 and 11.7Hz, 16-H), 3.86-3.97 (4H, m, 5-H, 7-H, 13-H and 16-H), 6.18 (1H, s, 2-H), 6.80 (1H, s, 4′-H); $\delta_C$ ($CDCl_3$) 12.7 (C-17), 19.4 (C-15), 20.8 (C-14), 30.5 (C-3″), 31.7 (C-9), 33.3 (C-1″), 39.5 (C-8), 40.1 (C-2″), 42.7 (C-12), 42.8 (C-4), 55.6 (C-10), 61.3 (C-11), 65.4 (C-16), 69.0 (C-6), 70.4 (C-7), 71.4 (C-13), 75.1 (C-5), 113.2 (C-2), 122.1 (C-4′), 145.9 (C-5′), 152.9 (C-3), 160.8 (C-1), 210.1 (C-4″); m/z (463, 12%), 219 (100%). (Found: $M^+$, 463.2575. $C_{25}H_{37}NO_7$ requires M, 463.2570).

Example 12

5-[trans-4-(2-Ethoxy-1-hydroxyethyl)cyclohexyl]-2-(1-normon-2-yl)oxazole

a) 5-[trans-4-(2-Ethoxy-1-hydroxyethyl)cyclohexyl)-2-(1-nor-6,7,13,-tris-t-butyldimethylsilyloxy-mon-2-yl)-oxazole

The epoxide (example 8) (0.51g. 0.61mmol) and boron trifluoride etherate (0.25ml, 2.0mmol) in ethanol (2ml) and chloroform (10ml) were stirred at room temperature for 2h. pH 7 Buffer solution (100ml) was added and the aqueous phase extracted with dichloromethane (2 x 50ml). Drying ($MgSO_4$), evaporation to dryness under reduced pressure and purification by flash chromatography using ether/hexane (3:2) as eluent gave the title compound (0.24g, 45%) as a colourless oil; $\nu_{max}$ (liquid film) 3430, 2950, 2850, 1655, 1255, 1108, 335, 755cm$^{-1}$; $\delta_H$ ($CDCl_3$) 0.06-0.10 (18H, m, 6 x $SiCH_3$), 0.89-0.91 (30H, m, 3 x $^t$Bu and 17-$H_3$), 1.18-1.25 (6H, m, $OCH_2CH_3$ and 14-$H_3$), 2.20 (3H, s, 15-$H_3$), 2.55-2.73 (4H, m, 4-H, 10-H, 11-H and 4″-H), 3.33-3.41 (2H, m, 6″-H and 6-H), 3.49-3.60 (5H, m, 6″-H, 16-H, $OCH_2CH_3$ and 5″-H), 3.83-3.92 (4H, m, 5-H, 7-H, 13-H and 16-H), 6.15 (1H, s, 2-H), 6.64 (1H, s, 4′-H); $\delta_C$ ($CDCl_3$) -4.9 ($SiCH_3$), -4.6 ($SiCH_3$), -4.5 ($SiCH_3$), -4.3 ($SiCH_3$), -4.2 ($SiCH_3$), -3.2 ($SiCH_3$), 12.7 (C-17), 15.1 ($OCH_2CH_3$), 18.1 [$C(CH_3)_3$], 18.2 [$C(CH_3)_3$], 19.0 (C-15), 20.8 (C-14), 25.9 [$C(CH_3)_3$], 26.1 [$C(CH_3)_3$], 27.8, 28.3, 30.6, 30.7, 30.7, 30.7, 32.0 (C-9), 35.2 (C-1″), 40.2 (C-4″), 42.2 (C-8), 43.0 (C-12), 43.1 (C-4), 55.5 (C-10), 59.1 (C-11). 65.5 (C-16), 66.7 ($OCH_2CH_3$), 70.4 (C-6), 71.0 (C-7), 72.9 (C-6″), 73.5 (C-13), 74.1 (C-5), 74.4 (C-5″), 113.1 (C-2), 121.0 (C-4′), 145.6 (C-5′), 155.2 (C-3), 160.5 (C-1); m/z (FAB 3-NOBA/Na) 902 ($MNa^+$, 58%), 800 ($MH^+$, 34%), 159 (100%).

b) 5-[trans-4-(2-Ethoxy-1-hydroxyethyl)cyclohexyl]2-(1-normon-2-yl)oxazole

The above oxazole (0.29g, 0.33mmol) and tetra-n-butylammonium fluoride trihydrate (0.62g, 1.97mmol) in THF (10ml) were stirred at room temperature for 40h. Evaporation to dryness under reduced pressure and purification by flash chromatography using methyl acetate/acetone (2:1) as eluent gave the title compound (0.093g, 53%) as a white foam; $\nu_{max}$ (KBr) 3425, 2926, 1655, 1513, 1449, 1377, 1111, 1052cm$^{-1}$; $\lambda_{max}$ (EtOH) 265nm ($\epsilon_m$ 18,309); $\delta_H$ ($CDCl_3$) 0.93 (3H, d, J 7.0Hz, 17-$H_3$), 1.19-1.25 (6H, m, 14-$H_3$ and $OCH_2CH_3$), 2.22 (3H, s, 15-$H_3$), 2.54-2.66 (1H, m, 4″-H), 2.71 (1H, dd, J 7.9 and 2.0Hz, 11-H), 2.78-2.84 (1H, m, 10-H), 3.33-3.40 (1H, m, 6″-H), 3.45 (1H, dd, J 8.3 and 7.9Hz, 6-H), 3.47-3.60 (5H, 16-H, 5″-H, 6″-H and $OCH_2CH_3$), 3.74-3.83 (2H, m, 5-H and 13-H), 3.85-3.93 (2H, m, 16-H and 7-H), 6.16 (1H, s, 2-H), 6.68 (1H, s, 4′-H); $\delta_C$ ($CDCl_3$) 12.6 (C-17), 15.1 ($CH_2CH_3$), 19.3 (C-15), 20.8 (C-14), 27.7, 28.2, 30.6, 30.6, 30.7, 30.7 31.7 (C-9), 35.2 (C-1″), 39.5 (C-8), 40.2 (C-4″), 42.7 (C-4), 4.7 (C-12), 55.9 (C-10), 61.2 (C-11), 65.5 (C-16), 66.8 ($OCH_2CH_3$), 68.6 (C-6), 70.4 (C-7), 71.1 (C-13), 72.8 (C-6″), 74.1 (C-5″), 75.2 (C-5), 113.3 (C-2), 120.7 (C-4′), 145.3 (C-5′), 155.7 (C-3), 160.4 (C-1); m/z 537 ($M^+$, 22%), 293 (100%). (Found: $M^+$, 537.3291. $C_{29}H_{47}NO_8$ requires M, 537.3302).

Example 13

5-[trans-(4-Ethoxycarbonylmethylene)cyclohexyl]-2-(1-normon-2-yl)oxazole

a) 5-[trans-(4-Ethoxycarboylmethylene)cyclohexyl-2-(1-nor-6,7,13-tris-t-butyldimethylsilyloxy-mon-2-yl)-oxazole

Sodium hydride (80% dispersion in mineral oil) (0.0175g, 0.58mmol) was added to diethyl ethoxycarbonylmethylphosphonate (0.122g, 0.50mmol) in 1,2-dimethoxyethane (2ml) at 0°C. After 0.5h at 0°C, ketone (example 11c) (0.20g, 0.25mmol) in 1,2-dimethyoxyethane (2ml) was added. The mixture was warmed to room temperature and stirred at that temperature for 3h. Saturated ammonium chloride solution (10ml) was added and the aqueous phase extracted with ethyl acetate (3 x 10ml). Drying ($MgSO_4$), evaporation to dryness under reduced pressure and purification by flash chromatography using ether/hexane (2:1) as eluent gave the title compound (0.154g, 70%) as a viscous oil; $\nu_{max}$ (liquid film) 2930, 1710, 1650, 1450, 1145, 1110cm$^{-1}$; $\delta_H$ ($CDCl_3$) 0.05-0.11 (18H, m, 6 x $5SiCH_3$), 0.90-0.93 (30H, m, 17-$H_3$ and 3 x $^tBu$), 1.20 (3H, d, J 6.4Hz, 14-$H_3$), 1.29 (3H, t, J 7Hz, $CH_2CH_3$), 2.21 (3H, s, 15-$H_3$), 2.94 (1H, tt, J 7.9 and 4.3Hz, 1''-H), 3.41 (1H, dd, J 1.8 and 9.0Hz, 6-H), 3.56 (1H, d, J 11.1Hz, 16-H), 4.16 (2H, q, J 7.1Hz, $CH_2CH_3$), 5.68 (1H, s, 5''-H), 6.17 (1H, s, 2-H), 6.74 (1H, s, 4'-H); $\delta_C$ ($CDCl_3$) -4.9 ($SiCH_3$), -4.6 ($SiCH_3$), -4.5 ($SiCH_3$), -4.3 ($SiCH_3$), -4.2 ($SiCH_3$), -3.2 ($SiCH_3$), 12.7 (C-17), 14.3 ($CH_2CH_3$), 18.1 [$C(CH_3)_3$], 18.1 [$C(CH_3)_3$], 18.2 [$C-(CH_3)_3$], 19.0 (C-15), 20.9 (C-14), 25.9 [$C(CH_3)_3$], 26.1 ($C(CH_3)_3$], 28.2 (C-3''), 31.4 (C-2''), 32.0 (C-9), 32.2 (C-2''), 32.2 (C-2''), 34.6 (C-1''), 36.3 (C-3), 42.1 (C-8), 43.0 (C-12), 43.1 (C-4), 55.5 (C-10), 59.0 (C-11), 59.6 ($CH_2CH_3$), 65.5 (C-16), 70.4 (C-6), 70.4 (C-6), 70.9 (C-7), 73.5 (C-13), 74.4 (C-5), 113.0 (C-2), 114.2 (C-5''), 121.7 (C-4'), 145.9 (C-5'), 153.7 (c-3), 160.6 (C-1), 166.5 (C-6''); m/z (FAB 3-NOBA/Na), 898 ($MNa^+$, 31%), 876 ($MH^+$, 27%), 159 (100%).

b) 5-[trans-(4-Ethoxycarbonyl-methylene)cyclohexyl]-2-(1-normon-2-yl)oxazole

The above oxazole (0.100g, 0.114mmol) and tetra-n-butylammmonium fluoride trihydrate (0.43g, 1.37mmol) in THF (2ml) were stirred at room temperature for 24h. Evaporation to dryness under reduced pressure and purification by flash chromatography using methyl acetate as eluent gave the title compound (0.038g, 62%) as a white foam; $\nu_{max}$ (KBr) 3433, 2926, 1711, 1652, 1450, 1146, 1111, 1046cm$^{-1}$; $\lambda_{max}$ - (EtOH) 765nm ($\epsilon_m$ 17,835); $\delta_H$ ($CDCl_3$) 0.92 (3H, d, J 7.0Hz, 17-$H_3$), 1.21 (3H, d, J 6.3Hz, 14-$H_3$), 1.24-1.30 (3H, m, $CH_2CH_3$), 1.70-1.74 (2H, m, 9-$H_2$), 2.21 (3H, s, 15-$H_3$), 2.71 (1H, dd, J 7.8 and 2.1Hz, 11-H), 2.79-2.82 (1H, m, 10-H), 3.48-3.50 (1H, m, 6-H), 3.55 (1H, dd, J 11.5 and 1.9Hz, 16-H), 3.75-3.93 (4H, m, 5-H, 7H, 13-H, 16-H), 4.12-4.18 (2H, m, $CH_2CH_3$), [5.61 (br.s) and 5.67 (s)] (1H, 5''-H), 5.17 (1H, s, 2-H), [6.72 (s) and 6.74 (s)] (1H, 4'-H); $\delta_C$ ($CDCl_3$) 12.7 (C-17), 14.3 ($CH_2CH_3$), 19.4 (C-15), 20.8 (C-14), 27.1, 27.8, 28.2, 29.7, 30.9 (C-1''), 31.7 (C-9), 32.1, 34.6 (C-1''), 36.3 (C-3''), 39.5 (C-8), 42.7 (C-4), 4.8 (C-12), 43.2 (C-4), 55.6 (C-10), 59.7 ($CH_2CH_3$), 60.6 ($CH_2CH_3$), 61.3 (C-11), 68.9 (C-6), 70.4 (C-7), 71.3 (C-13), 75.2 (C-5), 113.3 (C-2), 113.4 (C-2), 114.3 (C-5''), 121.4 (C-4'), 123.8 (C-4'), 131.3 (C-4''), 145.5 (C-5'), 145.7 (C-5'), 154.3 (C-3), 155.1 (C-3), 160.5 (C-1), 160.6 (C-1), 166.8 (C-6''), 171.8 (C-6''): m/z 533 ($M^+$, 20%), 289 (100%). (Found: $M^+$, 533.3001. $C_{29}H_{43}NO_8$ requires M, 533.2989).

Example 14

5-[trans-(4-Hydroxyethylidene)cyclohexyl-2-(1-normon-2-yl)oxazole

a) 5-[trans-(4-Hydroxyethylidene)cyclohexyl]-2-(1-nor-6,7,13-tris-t-butyldimethylsilyloxy-mon-2-yl)oxazole

Di-isobutylaluminium hydride (1.5M in toluene) (0.64ml, 0.96mmol) was added dropwise at -78 °C to the ester (example 13a) (0.28g, 0.319mmol) in toluene (6ml). After 2h, methanol (2ml) and saturated sodium sulphate solution (2ml) were added and the reaction mixture allowed to warm to room temperature over 0.5h. The precipitated solids were removed by filtration and washed with ethyl acetate (50ml). Drying ($MgSO_4$), evaporation to dryness under reduced pressure and purification by flash chromatography using ether/hexane (3:2) as eluent gave the title compound (0.23g, 86%) as a white foam; $\nu_{max}$ (KBr) 3416, 2931, 2858, 1660, 1472, 1256, 1124, 1084, 965, 837, 774cm$^{-1}$; $\delta_H$ ($CDCl_3$) 0.04-0.10 (18H, m, 6 x $SiCH_3$), 0.86-

0.92 (30H, m, 3 x $^{t}$Bu and 17-$H_3$), 1.19 (3H, d, J 6.3Hz, 14-$H_3$), 2.20 (3H, s, 15-$H_3$), 3.39-3.41 (1H, m, 6-H), 3.55 (1H, d, J 11.4Hz, 16-H), 4.16 (2H, d, J 7.0Hz, 6″-$H_2$), 5.44 (1H, t, J 7.0Hz, 6″-$H_2$), 5.44 (1H, t, J 7.0Hz, 5″-H), 6.14 (1H, s, 2-H), 6.71 (1H, s, 4′-H); $\delta_C$ ($CDCl_3$) -4.7($SiCH_3$), -4.5 ($SiCH_3$), -4.4 ($SiCH_3$), -4.3 ($SiCH_3$), -4.2 ($SiCH_3$), -3.2 ($SiCH_3$), 12.7 (C-17), 18.1 [$C(CH_3)_3$], 18.1 [$C(CH_3)_3$], 18.2 [$C(CH_3)_3$], 19.0 (C-15), 20.7 (C-4), 25.9 [$C(CH_3)_3$], 26.1 [$C(CH_3)_3$], 27.4 (C-3″), 31.7, 31.7, 32.0 (C-9), 32.2, 32.2, 35.1 (C-1″), 35.5 (C-3″), 42.2 (C-8), 43.0 (C-12), 43.2 (C-4), 55.5 (C-10), 58.5 (C-6″), 59.1 (C-11). 65.5 (C-16), 70.4 (C-6), 70.9 (C-7), 73.5 (C-13), 74.4 (C-5), 113.0 (C-2), 121.4 (C-4′), 121.8 (C-5″), 141.8 (C-5′), 145.8 (C-4″), 154.4 (C-3), 160.6 (C-1); m/z 833 ($M^+$, 1%), 73 (100%).

b) 5-[trans-(4-Hydroxyethylidene)cyclohexyl]-2-(1-nor-mon2-yl)oxazole

The above oxazole (0.100g, 0.12mmol) and tetra-n-butylammonium fluoride trihydrate (0.43g, 1.6mmol) in THF (2ml) were stirred at room temperature for 60h. Evaporation to dryness under reduced pressure and purification by flash chromatography using methyl acetate/hexane 1:1 as eluent gave the title compound (0.038g. 64%) as a white foam; $\nu_{max}$ (KBr) 3409, 2967, 2931, 1658, 1246, 1111, 1050, 1003cm 1; $\lambda_{max}$ - (EtOH) 265nm ($\epsilon_m$ 16,755); $\delta_H$ ($CDCl_3$) 0.89 (3H. d, J 7.0Hz, 17-$H_3$), 1.20 (3H, d, J 6.3Hz, 14-$H_3$), 1.70-1.73 (2H, m, 9-$H_2$), 2.20 (3H, s, 15-$H_3$), 3.47-3.49 (1H, m, 6-H), 3.54 (1H, d, J 10.0Hz, 16-H), 4.10-4.20 (2H, m, 6″-$H_2$), 5.42 (1H, t, J 6.9Hz, 5″-H), 6.16 (1H, s, 2-H), 6.69 (1H, s, 4′-H); $\delta_C$ ($CDCl_3$) 12.6 (C-17), 19.4 (C-15), 20.8 (C-14), 27.4 (C-3″), 31.6, 31.6, 31.7 (C-9), 32.1, 32.1, 35.0 (C-1″), 35.4 (C-3″), 39.5 (C-8), 42.7 (C-4), 42.7 (C-12), 55.6 (C-10), 58.3 (C-6″), 61.2 (C-11), 65.4 (C-16), 68.8 (C-6), 70.3 (C-7), 71.1 (C-13), 76.2 (C-5), 113.3 (C-2), 121.1 (C-4′), 121.8 (C-5″), 141.6 (C-5′), 145.5 (C-4″), 154.9 (C-3), 160.5 (C-1); m/z 491 ($M^+$, 6%).

Example 15

5-[cis-(1-(R,S),3(S,R))-3-Hydroxycyclohexyl]-2-(1normon-2-yl)oxazole

a) cis-1-t-Butyldiphenylsilyloxy-3-isopropoxycarbonylcyclohexane

cis-3-Isopropoxycarbonylcyclohexanol (2.00g, 10.8mmol), t-butylchlorodiphenylsilane (4.43g, 4.19ml, 16.1mmol) and imidazole (2.19g, 32.2mmol) in DMF (25ml) were stirred at 60° C for 16h. The products were partitioned between ethyl acetate (150ml) and water (250ml). The organic layer was separated and the aqueous phase extracted with ethyl acetate. The combined organic extracts were dried ($MgSO_4$), evaporated to dryness under reduced pressure and purified by flash chromatography using dichloromethane/hexane (1:1) as eluent to give the title compound (4.14g, 91%) as a colourless liquid; $\nu_{max}$ - (liquid film) 2960, 2840, 1730, 1595, 1110, 1040, 720cm$^{-1}$; δH ($CDCl_3$) 1.02 (9H, s, $^{t}$Bu), 1.18 (6H, d, J 6.3Hz, $CH(CH_3)_2$], 1.15-1.85 (7H, m, cyclohexyl), 2.02-2.16 (2H, m, 1-H and cyclohexyl), 3.54 (1H, tt, J 4.2 and 10.7Hz, 3-H), 4.95 [1H, s, J 6.3Hz, $CH(CH_3)_2$], 7.32-7.46 (6H, m, aromatic), 7.65-7.69 (4H, m, aromatic); $\delta_C$ ($CDCl_3$) 19.1 [$C(CH_3)_3$], 21.8 [$CH(CH_3)_2$], 23.5 (C-5), 27.0 [$C(CH_3)_3$], 28.1 (C-4), 35.4 (C-6), 38.0 (C-2), 42.3 (C-3), 67.3 [$CH(CH_3)_2$], 71.5 (C-1), 127.5, 127.5, 129.5, 129.5, 134.5(q), 134.6(q), 135.8, 174.5 (C=O); m/z (FAB 3-NOBA/Na) 447 ($MNa^+$, 18%), 425 ($MH^+$, 7%), 176 (100%).

b) cis and trans-1-Bromoacetyl-3-t-butyldiphenylsilyloxycyclohexane

The isopropyl ester (Example 15a) (4.14g, 9.76mmol) in methanol (150ml), THF (50ml) and 1M lithium hydroxide (49ml, 49mmol) was stirred at room temperature for 60h. The solution was adjusted to pH 7 with Amberlite IR 120$H^+$ resin and filtered, the filtered resin washed with water and dichloromethane, and the filtrate evaporated to dryness under reduced pressure to give the crude acid. Oxalyl chloride (0.95ml, 10.9mmol) in dichloromethane (10ml) was added dropwise over 30min to the crude acid in dichloromethane (100ml) and DMF (1 drop) at room temperature. After 1h the products were evaporated to dryness under reduced pressure to give the crude acid chloride. The crude acid chloride in ether (50ml) was added dropwise to diazomethane (ca.28mmol) in ether (100ml) at 0°C. After 1h concentrated hydrobromic acid (48%) (2.95ml) was added and the solution stirred at 0°C for 2h. The solution was neutralised with sodium

hydrogen carbonate solution and extracted with ethyl acetate. Drying ($MgSO_4$), evaporation to dryness under reduced pressure and purification by flash chromatography using dichloro methane/hexane (1:2 to 1:1) as eluent gave firstly the trans-title compound (1.35g, 30%) as a colourless liquid containing the chloro analogue as an impurity (10%); $\nu_{max}$ (liquid film) 2940, 2860, 1715, 1110, 700cm$^{-1}$; $\delta_H$ ($CDCl_3$) 1.09 (9H, s, $^tBu$), 1.31-1.50 (4H, m, cyclohexyl), 1.52-1.98 (4H, m, cyclohexyl), 3.31 (1H, tt, J 3.4 and 11.4Hz, 1-H), 3.83 (2H, s, $CH_2Br$), 4.12-4.18 (1H, m, 3-H), 7.34-7.48 (6H, m, aromatic), 7.65-7.69 (4H, m, aromatic); $\delta_C$ ($CDCl_3$) 19.3 [$C(CH_3)_3$], 19.7 (C-5), 27.1 [$C(CH_3)_3$], 28.0 (C-6), 32.6 (C-4), 33.1 ($CH_2Br$), 35.4 (C-2), 42.4 (C-1), 67.3 (C-3), 127.6, 127.7, 129.7, 129.8, 134.1(q), 134.4(q), 135.7, 204.8 (C=O); m/z (C.I.$NH_3$) 459 ($MH^+$, 2%), 203 (100%); [Found: 401.0557. $C_{20}H_{22}O_2SiBr$(M-$^tBu$) requires 401.0574]; followed by the cis-title compound (1.40g, 31%) as a colourless liquid; $\nu_{max}$ (liquid film) 2940, 2860, 1715, 1110, 700cm$^{-1}$; $\delta_H$ ($CDCl_3$) 1.05 (9H, s, $^tBu$), 1.05-1.52 (4H, m, cyclohexyl), 1.65-1.96 (4H, m, cyclohexyl), 2.54 (1H, tt, J 3.2 and 11.5Hz, 1-H), 3.56-3.68 (1H, m, 3-H), 7.34-7.48 (6H, m, aromatic), 7.65-7.69 (4H, m, aromatic); $\delta_C$ ($CDCl_3$) 19.1 [$C(CH_3)_3$], 23.3 (C-5), 27.1 [$C(CH_3)_3$], 27.5 (C-6), 33.0 ($CH_2Br$), 35.1 (C-4), 37.5 (C-2), 46.5 (C-1), 171.4 (C-3), 127.6, 127.6, 129.6, 129.7, 134.4(g), 135.8, 203.2 (C=O); m/z (C.I.$NH_3$) 459 ($MH^+$, 100%); [Found: 401.0588. $C_{20}H_{22}O_2SiBr$(M-$^tBu$) requires 401.0574].

c) cis-3-Bromoacetylcyclohexanol

The cis-isomer silyl ether (Example 15b) (1.36g, 2.96mmol) was dissolved in 6% methanolic hydrogen chloride (28ml) and stirred at room temperature for 16h. The solution was neutralised with pH 7 buffer solution (100ml) and the aqueous phase extracted with dichloromethane (3 x 150ml). Drying ($MgSO_4$), evapora tion to dryness under reduced pressure and purification by flash chromatography using dichloromethane/ethyl acetate (3:1) as eluent gave the title compound (0.48g, 76%) as a colourless liquid containing the chloro analogue (ca.10%); $\nu_{max}$ (liquid film) 3400 (br.s), 2940, 2860, 1720, 1060cm$^{-1}$; $\delta_H$ - ($CDCl_3$) 1.25-1.49 (4H, m, cyclohexyl), 1.58 (1H, br.s, OH), 1.81-2.04 (3H, m cyclohexyl), 2.09-2.17 (1H, m, cyclohexyl), 2.75 (1H, tt, J 3.5 and 11.3Hz, 3-H), 3.68 (1H, tt, J 4.0 and 10.7Hz, 1-H), 4.18 (2H, s, $CH_2Br$); $\delta_C$ ($CDCl_3$) 23.2 (C-5), 27.4 (C-4), 34.9 ($CH_2Br$), 36.8 (C-6), 46.2 (C-3), 47.1 (C-2), 69.6 (C-3), 204.0 (C=O); m/z (C.I.$NH_3$) 223 ($MH^+$, 3%), 221 ($MH^+$, 3%), 177 [$MH^+(Cl^{37})$, 100%].

d) cis-3-Azidoacetylcyclohexanol

cis-3-Bromoacetylcyclohexanol (0.50g, 2.26mmol) in chloroform (25ml) was treated with tetramethylguanidinium azide (0.42g, 2.66mmol) for 16h at room temperature. Evaporation to dryness under reduced pressure and purification by flash chromatography using dichloromethane/ethyl acetate (3:1) as eluent gave the title compound (0.40g, 98%) as a colourless oil which discoloured on standing; $\nu_{max}$ (liquid film) 3420-(brs), 1960, 2100, 1725, 1270cm$^{-1}$; $\delta_H$ ($CDCl_3$) 1.15-1.47 (4H, m, cyclohexyl), 1.66 (1H, br.s, OH), 1.75-2.06 (3H, m, cyclohexyl), 2.06-2.14 (1H, m, cyclohexyl), 2.50 (1H, tt, J 3.8 and 11.4Hz, 3-H), 3.66 (1H, tt, J 4.2 and 10.3Hz, 1-H), 4.02 (2H, s, $CH_2N_3$); $\delta_C$ ($CDCl_3$) 23.2, 27.3, 34.8, 36.5, 46.8 (C-3), 55.9 ($CH_2N_3$), 69.5 (C-1), 206.3 (C=O); m/z (L.C.M.S. thermospray) 201 ($MNH_4^+$, 100%), 184 ($MH^+$, 22%).

e) N-[2-Oxo-2-(cis-(1-(R,S),3(S,R))-3-hydroxycyclo hexyl)ethyl]monamide

The cis-azide (Example 15d) (0.40g, 2.18mmol) in methanol (30ml), water (20ml) and concentrated hydrochloric acid (1.75ml) was hydrogenated over 5% Pd on C (0.077g) at 1 atm. for 1h. The reaction mixture was filtered and the filtrate evaporated to dryness under reduced pressure to give the crude cis-3-aminoacetylcyclohexanol hydrochloride.

The monamide was prepared according to the general method from the crude amine hydrochloride in THF/$H_2O$ (1:1, 25ml) and isolated after chromatography using 0-20% methanol in dichloromethane as a white foam (0.16g, 15%) as a 4:1 mixture with the trans-isomer; $\nu_{max}$ (KBr) 3407, 2933, 1718, 1660, 1629, 1052cm$^{-1}$; $\delta_H$ ($CD_3OD$) 0.94 (3H, d, J 7.0Hz, 17-$H_3$), 1.20 (3H, d, J 6.5Hz, 14-$H_3$), 2.14 (3H, s, 15-$H_3$), 2.43-2.67 (2H, m, 1″-H and 4-H), 2.70 (1H, dd, J 7.5 and 2.1Hz, 11-H), 2.75-2.83 (1H, m, 10-H), 3.35 (1H, dd, J 8.9 and 3.1Hz, 6-H), 3.51-3.64 (2H, m, 3″-H and 16-H), 3.71-3.92 (4H, m, 5, 7, 13 and 16-H), 4.12 (2H, s, 1′-$H_2$), 5.82 (1H, s, 2-H); $\delta_C$ ($CD_3OD$) 12.3 (C-17), 19.0 (C-15), 26.4 (C-14), 24.7, 28.7, 33.0 (C-9), 35.8, 37.9, 41.6 (C-8), 43.7 (C-4), 43.9 (C-12), 47.9 (C-1″), 48.2 (C-1′), 56.9 (C-10), 61.2 (C-11), 66.3 (C-16), 70.0 (C-6), 70.6 (C-3″), 70.7 (C-7), 71.6 (C-13), 76.2 (C-5), 120.6 (C-2), 153.1 (C-3), 169.7 (C-1), 209.5 (C-2′); m/z (E.I.)

483 ($M^+$, 2%), 139 (100%); (Found: $M^+$, 483.2832. $C_{25}H_{41}NO_8$ requires M, 483.2832).

f) 5-[cis-(1-(R,S),3-(S,R))-3-Hydroxycyclohexyl-1-2-(1-normon-2-yl)oxazole

The monamide (15e) (0.40g, 2.18mmol) was cyclised according to the general procedure to give the oxazole (0.16g, 15%) as a white foam containing a 4:1 mixture of cis to trans isomers; $\nu_{max}$ (KBr) 3409, 2934, 2861, 1712, 1449, 1111, 1000cm$^{-1}$; $\delta_H$ ($CD_3OD$) 0.94 (3H, d, J 7.0Hz, 17-$H_3$), 1.19 (3H, d, J 6.4Hz, 14-$H_3$), 2.19 (3H, s, 15-$H_3$), 2.29 (1H, dd, J 8.5 and 14.5Hz, 4-H), 2.67-2.83 (4H, m, 4, 10, 11 and 1″-H), 3.40 (1H, dd, J 8.8 and 3.0Hz, 6-H), 3.56 (1H, d, J 11.2Hz, 16-H), 3.66 (1H, tt, J 11.0 and 4.2Hz, 3″-H), 3.72-3.90 (4H, m, 5, 7, 13, 16-H), 6.12 (1H, s, 2-H), 6.78 (1H, s, 4′-H); $\delta_C$ ($CD_3OD$), 12.2 (C-17), 19.5 (C-15), 20.3 (C-14), 24.7, 23.4 (C-1″), 31.3, 33.0 (C-9), 35.9, 40.7, 41.6 (C-8), 43.7 (C-12), 43.7 (C-4), 56.8 (C-10), 61.2 (C-11), 66.3 (C-16), 70.0 (C-6), 70.5 (C-3″), 70.7 (C-7), 71.6 (C-13), 176.4 (C-5), 113.7 (C-2), 121.6 (C-4′), 147.8 (C-5′), 157.7 (C-3), 162.0 (C-1); m/z (E.I.) 465 ($M^+$, 17%), 221 (100%).

Example 16

5-[trans-(1-(R,S),3-(R,S))-3-Hydroxycyclohexyl]-2-(1-normon-2-yl)oxazole

a) trans-3-Azidoacetylcyclohexanol

trans-3-Bromoacetylcyclohexanol (0.40g, 1.80mmol) in chloroform (25ml) was treated with tetramethylguanidinium azide (0.36g, 2.15mmol) for 16h at 0-5°C in a fridge. Evaporation to dryness under reduced pressure and purification by flash chromatography using dichloromethane/ethyl acetate (3:1) as eluent gave the title compound (0.27g, 85%) as a colourless oil; $\nu_{max}$ (liquid film) 3420 (br.s), 2960, 2100, 1725, 1270cm$^{-1}$; $\delta_H$ ($CDCl_3$) 1.3-1.89 (9H, m, OH and 8 x cyclohexyl), 2.92 (1H, tt, J 3.7 and 10.6Hz, 1-H), 4.00 (2H, s, $CH_2N_3$), 4.16-4.21 (1H, m, 3-H); $\delta_C$ ($CDCl_3$) 19.5, 27.7, 32.4, 34.3, 42.7 (C-1), 55.9 ($CH_2N_3$), 65.6 (C-3), 207.6 (C=O); m/z (C.I.$NH_3$) 201 ($MNH_4^+$, 67%), 158 (100%).

(b) N-[2-Oxo-1-(trans-(1-(R,S),3-(R,S))-3-Hydroxycyclohexyl)ethyl]monamide

The trans-azide (Example 16d) (0.27g, 1.47mmol) in methanol (20ml), water (10ml) and concentrated hydrochloric acid (1.2ml) was hydrogenated over 5% Pd on C (0.077g) at 1 atm. for 1h. The reaction mixture was filtered and the filtrate evaporated to dryness under reduced pressure to give the crude trans-3-aminoacetylcyclohexanol hydrochloride.

The monamide was prepared according to the general method from the crude amine hydrochloride in THF/$H_2O$ (1:1, 25ml) and isolated after chromatography using 0-20% methanol in dichloromethane as a white foam (0.27g, 38%) containing a 9:1 mixture of trans and cis-isomers; $\nu_{max}$ (KBr) 3404, 2932, 1719, 1660, 1629, 1111cm$^{-1}$; $\delta_H$ ($CD_3OD$) 0.94 (3H, d, J 7.0Hz, 17-$H_3$), 1.20 (3H, d, J 6.5Hz, 14-$H_3$), 2.14 (3H, s, 15-$H_3$), 2.61 (1H, d, J 14.3Hz, 4-H), 2.70 (1H, dd, J 7.5 and 2.1Hz, 6-H), 2.78-2.83 (1H, m, 10-H), 2.94 (1H, tt, J 10.1 and 3.7Hz, 1″-H), 4.12 (2H, s, 2″-$H_2$), 5.82 (1H, s, 2-H); $\delta_C$ ($CD_3OD$) 12.3 (C-17), 19.0 (C-15), 20.4 (C-14), 20.8, 28.8, 33.0 (C-9), 33.4, 35.6, 41.7 (C-8), 43.7 (C-4), 43.8 (C-1″), 43.9 (C-12), 48.2 (C-1′), 56.9 (C-10), 61.3 (C-11), 66.3 (C-3″), 66.5 (C-16), 70.1 (C-6), 70.7 (C-7), 71.7 (C-13), 76.3 (C-5), 120.7 (C-2), 153.0 (C-3), 169.7 (C-1), 210.7 (C-2′); m/z (E.I.) 483 ($M^+$, 5%), 139 (100%).

c) 5-[trans-(1-(R,S),3-(R,S))-3-Hydroxycyclohexyl]-2-(1-normon-2-yl)oxazole

The monamide (16b) (0.20g, 0.4mmol) was cyclised according to the general procedure to give the oxazole (0.048g, 25%) as a white foam containing a 9:1 mixture of trans to cis isomers; $\nu_{max}$ (KBr) 3420, 2932, 1656, 1598, 1449, 1378, 1113, 1051cm$^{-1}$; $\lambda_{max}$ (EtOH) 265nm ($\epsilon_m$ 16,300); $\delta_H$ ($CD_3OD$) 0.94 (3H, d, J 7.0Hz, 17-$H_3$), 1.19 (3H, d, J 6.4Hz, 14-$H_3$), 1.35-1.43 (1H, m, 12-h), 1.43-1.63 (3H, m, 3 x cyclohexyl), 1.64-1.90 (5H, m, 9-Hz and 3 x cyclohexyl), 1.90-2.02 (3H, m, 8-H and 2 x cyclohexyl), 2.29 (1H, dd, J 8.5 and 14.5Hz, 4-H), 2.67-2.72 (2H, m, 4 and 11-H), 2.78-2.83 (1H, m, 10-H), 3.40 (1H, dd, J 8.8 and 3.0Hz, 6-H),

3.56 (1H, dd, J 1.5 and 11.5Hz, 16-H), 3.75-3.80 (2H, m, 5 and 13-H), 3.86-3.89 (2H, m, 16 and 17-H), 4.01-4.08 (1H, m, 3″-H), 6.13 (1H, s, 2-H), 6.77 (1H, s, 4′-H); $\delta_C$ ($CD_3OD$) 12.3 (C-17), 19.6 (C-15), 20.4 (C-14), 20.9, 31.0 (C-1″), 31.3, 33.0 (C-9) 33.5, 38.2, 41.7 (C-8), 43.8 (C-12), 43.8 (C-4), 56.9 (C-10), 61.3 (C-11), 66.4 (C-16), 66.6 (C-3″), 70.1 (C-6), 70.8 (C-7), 71.6 (C-13), 76.5 (C-5), 113.6 (C-2), 122.0 (C-4′), 147.8 (C-5′), 157.4 (C-3), 162.1 (C-2); m/z (E.I.) 465 ($M^+$, 18%), 221 (100%).

Biological Data

Human Bacteria

The activity of the compounds of the invention against various bacteria important in diseases in humans was assayed in vitro using a standard serial dilution technique in nutrient agar with 5% chocolated horse blood. The M.I.C. values (µg/ml) were determined after incubation for 18 h at 37°C and are shown in the table below:

| Organism | Example No. | | | | | | |
|---|---|---|---|---|---|---|---|
| | 1 | 2 | 3 | 4 | 5 | 6 | 7 |
| H.influenzae Q1 | 2 | 0.25 | 2 | 1 | 0.5 | 0.5 | 4 |
| B.catarrhalis 1502 | 8 | 4 | 32 | 64 | 4 | 8 | - |
| Strep.pyogenes CN10 | 0.13 | 0.25 | 32 | 32 | 1 | 0.25 | 0.5 |
| Strep.pneumoniae PU7 | 0.13 | 0.13 | - | - | 4 | 0.5 | 4 |
| Staph.aureus Oxford | 2 | 1 | 16 | 32 | 8 | 1 | 1 |
| **Organism** | **Example No.** | | | | | | |
| | 8 | 9 | 10 | 11 | 12 | 13 | 14 |
| H.influenzae Q1 | 2 | 2 | 4 | 0.5 | 4 | 1 | 1 |
| B.catarrhalis 1502 | - | - | 8 | 8 | 16 | 1 | 8 |
| S.pyogenes CN10 | - | 2 | 2 | 1 | 2 | 1 | 0.5 |
| S.pneumoniae PU7 | 2 | 0.5 | 4 | - | 4 | 2 | 2 |
| S.aureus Oxford | 2 | 2 | 4 | 8 | 2 | 2 | 2 |

## Claims

1. A compound of formula (I):

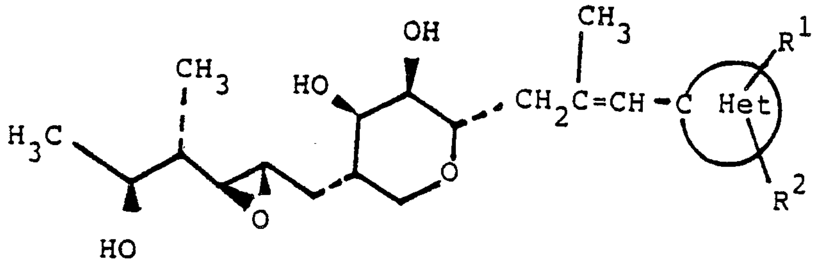

(I)

wherein

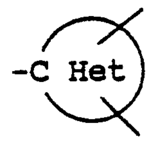

is a divalent or a trivalent, 5-membered heterocyclic group having a 6-$\pi$ electron system, and containing from 1 to 4 heteroatoms, each selected from oxygen, nitrogen and sulphur;
$R^1$ is a substituted $C_{3-7}$ cycloalkyl group attached to a carbon or nitrogen of

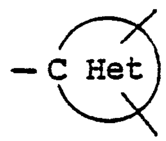 ;

and, where appropriate, $R^2$ is a group attached to a carbon or nitrogen of

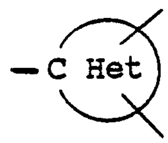 ,

and, when present, is selected from optionally substituted $C_{1-20}$ alkyl, optionally substituted $C_{3-7}$ cycloalkyl, optionally substituted $C_{2-8}$ alkenyl, aryl, aralkyl, heterocyclyl and hydrogen.

2. A compound as claimed in claim 1 having the formula (I-E):

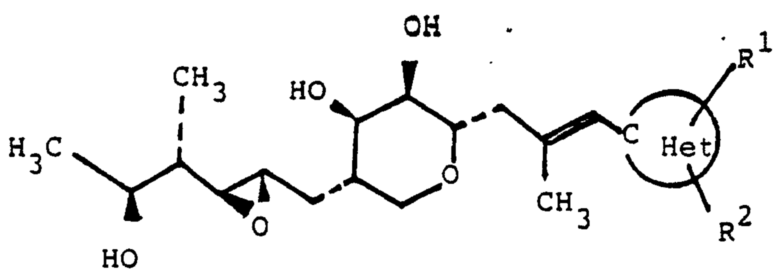

(I-E)

wherein

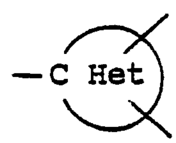 ,

$R^1$ and $R^2$ are as defined in claim 1.

3. A compound as claimed in claim 1 having the formula (IB):

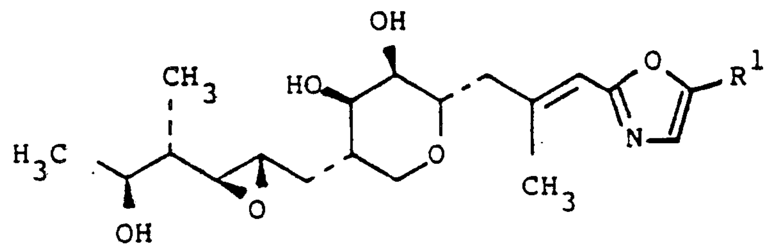

(IB)

4. A compound of formula (I) as defined in claim 1 selected from the group consisting of:
5-[trans-(4-Methoxycarbonyl)cyclohexyl]-2-(1-normon-2-yl)oxazole;
5-[trans-4-(Hydroxymethylcyclohexyl)]-2-normon-2-yl)oxazole;
sodium 5-(4-Carboxylatocyclohexyl)-2-(1-normon-2-yl)oxazole;
5-(cis-4-Hydroxycyclohexyl)-2-(1-normon-2-yl)oxazole;
5-[trans-(4-Hydroxycyclohexyl)]-2-(1-normon-2-yl)oxazole;
5-[trans-4-Cyanocyclohexyl]-2-(1-normon-2-yl)oxazole;
5-[trans-4-(2-Hydroxyprop-2-yl)cyclohexyl]-2-(1-normon-2-yl)oxazole;
5-[trans-4-(1,2-epoxyethyl)cyclohexyl]-2-(1-normon-2-yl)oxazole;
5-[trans-4-(2-Azido-1-hydroxyethyl)cyclohexyl]-2-(1-normon-2-yl)oxazole;
5-[trans-4-(1,3-dioxolan-2-yl)cyclohexyl]-2-(1-normon-2-yl)oxazole;
5-[4-Oxocyclohexyl]-2-(1-normon-2-yl)oxazole;
5-[trans-4-(2-Ethoxy-1-hydroxyethyl)cyclohexyl]-2-(1-normon-2-yl)oxazole;
5-[trans-(4-Ethoxycarbonylmethylene)cyclohexyl]-2-(1-normon-2-yl)oxazole;
5-[trans-(4-Hydroxyethylidene)cyclohexyl]-2-(1-normon-2-yl)oxazole;
5-[cis-(1-(R,S),3(S,R))-3-Hydroxycyclohexyl]-2-(1-normon-2-yl)oxazole; and
5-[trans-(1-(R,S),3-(R,S))-3-Hydroxycyclohexyl]-2-(1-normon-2-yl)oxazole.

5. A process for producing a compound of formula (I) as defined in claim 1 which process comprises reacting a compound of formula (II):

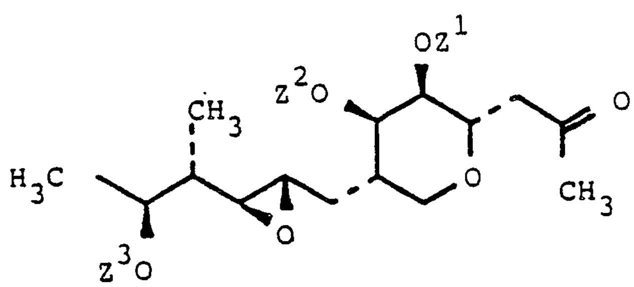

(II)

wherein $Z^1$, $Z^2$ and $Z^3$ are the same or different and each is hydrogen or a hydroxyl-protecting group, with a compound of formula (III):

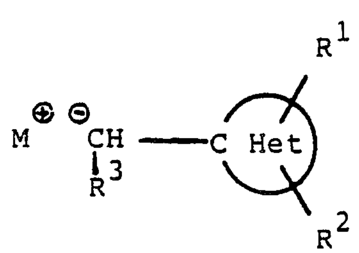

(III)

wherein:

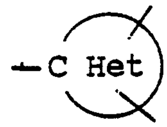

,

$R^1$ and $R^2$ are as defined in claim 1;
$M^+$ is a metal cation; and
$R^3$ is an anion-stabilising group which will spontaneously eliminate with a β-hydroxyl group to produce an olefin;
and,
where necessary, removing any hydroxyl-protecting groups, and, if desired, converting one compound of formula (I) into a further compound of formula (I).

6. A process for producing a compound of formula (IA):

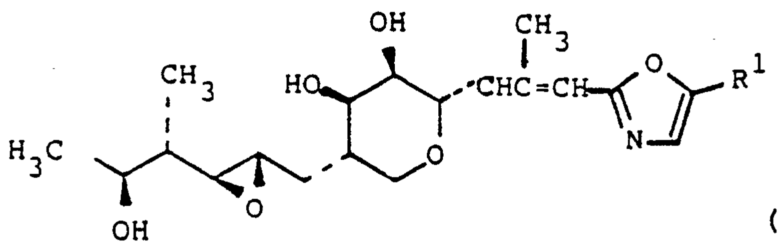

(IA)

wherein $R^1$ is as defined in claim 1, which process comprises cyclising a compound of formula (VI):

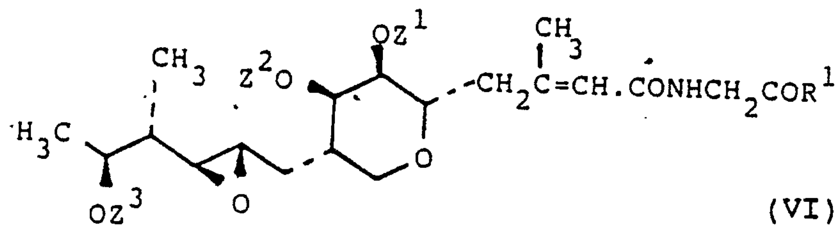

(VI)

wherein:
$R^1$ is as defined in claim 1; and
$Z^1$, $Z^2$ and $Z^3$ are the same or different and each is hydrogen or a hydroxyl-protecting group;
to form a compound of formula (IA) and, where necessary, removing any hydroxyl-protecting groups, and, if desired, converting one compound of formula (IA) into a further compound of formula (IA).

7. A compound of formula (VI):

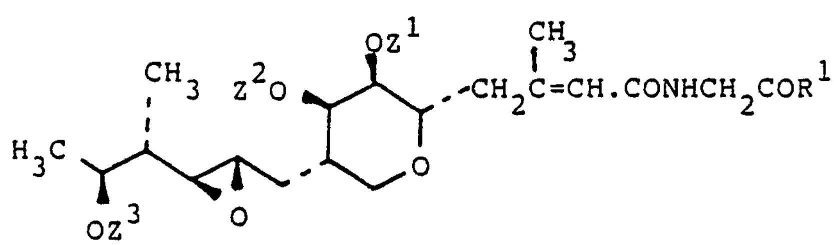

(VI)

wherein:
$R^1$ is as defined in claim 1; and $Z^1$, $Z^2$ and $Z^3$ are the same or different and each is hydrogen or a hydroxyl-protecting group.

8. A compound of formula (VI) as defined in claim 7 selected from the group consisting of:
N-[2-Oxo-2-(trans-4-methoxycarbonylcyclohexyl)ethyl]monamide;
N-[2-Oxo-2-(cis-4-hydroxycyclohexyl)ethyl]monamide;
N-[2-Oxo-2-(trans-4-hydroxycyclohexyl)ethyl]monamide;
N-(2-Oxo-2-(trans-4-cyanocyclohexyl)ethyl]monamide;
N-[2-Oxo-2-[(trans-4-(2-bromo-1-hydroxy)ethyl)cyclohexyl]ethyl]monamide;
N-[2-oxo-2[(trans-4-(2-chloro-1-hydroxy)ethyl)cyclohexyl]ethyl]monamide;
N-[2-Oxo-2-(cis-4-hydroxycyclohexyl-1-yl)ethyl]monamide;
N-[2-Oxo-2-(trans-4-hydroxycyclohexyl-1-yl)ethyl] monamide;
N-[2-Oxo-2-(cis-(1-(R,S),3(S,R))-3-hydroxycyclohexyl) ethyl]monamide;
N-[2-Oxo-1-(trans-(1-(R,S),3-(R,S))-3-hydroxycyclohexyl)ethyl]monamide; and
derivatives thereof wherein each or any of the hydroxyl groups is protected by a hydroxyl-protecting group.

9. A process for producing a compound of formula (IA) as defined in claim 6, which process comprises reacting monic acid or isomonic acid with a compound or formula (V):

$R^1COCH_2NH_3Cl^-$ (V)

wherein $R^1$ is as defined in claim 1, with triphenylphosphine and carbon tetrachloride in the presence of a tertiary amine.

10. A compound of formula (I) as defined in claim 1 for use in the treatment of bacterial infections.

11. A compound of formula (I) as defined in claim 1 for use as a therapeutic substance.

12. A pharmaceutical or a veterinary composition which comprises a compound of formula (I) as defined in claim 1 together with a pharmaceutically or veterinarily acceptable carrier or excipient.

13. The use of a compound of formula (I) as defined in claim 1 for the manufacture of a medicament.

Claims for the following Contracting State: ES

1. A process for producing a compound of formula (I):

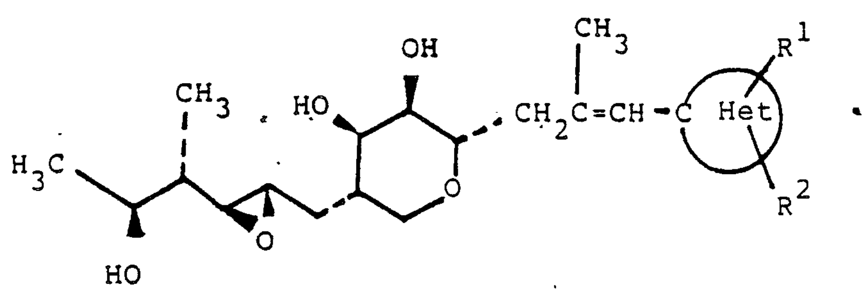

(I)

wherein

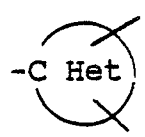

is a divalent or a trivalent, 5-membered heterocyclic group having a 6-$\pi$ electron system, and containing from 1 to 4 heteroatoms, each selected from oxygen, nitrogen and sulphur;
$R^1$ is a substituted $C_{3-7}$ cycloalkyl group attached to a carbon or nitrogen of

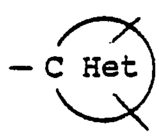

;

and, where appropriate, $R^2$ is a group attached to a carbon or nitrogen of

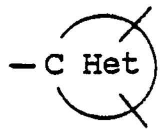 ,

and, when present, is selected from optionally substituted $C_{1-20}$ alkyl, optionally substituted $C_{3-7}$ cycloalkyl, optionally substituted $C_{2-8}$ alkenyl, aryl, aralkyl, heterocyclyl and hydrogen. which process comprises either:

(a) reacting a compound of formula (II):

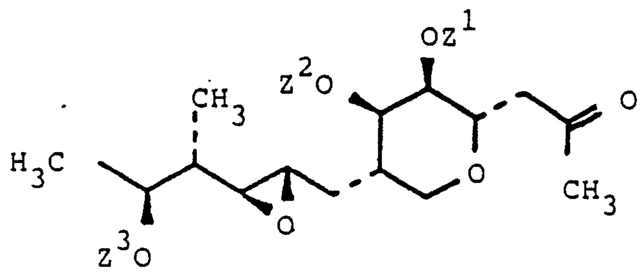

(II)

wherein $Z^1$, $Z^2$ and $Z^3$ are the same or different and each is hydrogen or a hydroxyl-protecting group, with a compound of formula (III):

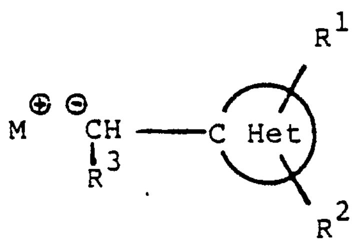

, (III)

wherein:

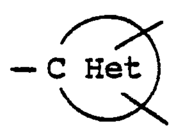 ,

$R^1$ and $R^2$ are as herein before defined;

$M^+$ is a metal cation; and

$R^3$ is an anion-stabilising group which will spontaneously eliminate with a $\beta$-hydroxyl group to produce an olefin; or

(b) cyclising a compound of formula (VI):

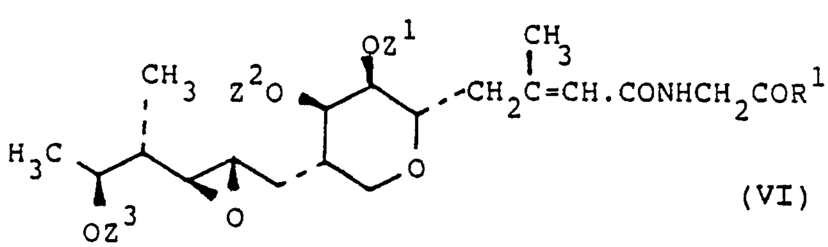

(VI)

wherein:

$R^1$ is as hereinbefore defined; and
$Z^1$, $Z^2$ and $Z^3$ are the same or different and each is hydrogen or a hydroxyl-protecting group;
to form a compound of formula (I) wherein

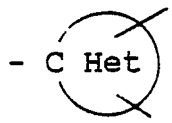

is 5-substituted-1,3-oxazol-2-yl; or
(c) reacting monic acid or isomonic acid with a compound of formula (V):
$R^1COCH_2NH_3Cl^-$ (V)
wherein $R^1$ is as hereinbefore defined;
with triphenylphosphine and carbon tetrachloride in the presence of a tertiary amine to form a compound of formula (I) wherein

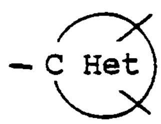

is 5-substituted-1,3-oxazol-2-yl;
and
where necessary, removing any hydroxyl-protecting groups, and, if desired, converting one compound of formula (I) into a further compound of formula (I).

2. A process as claimed in claim 1 to form a compound of the formula (I-E):

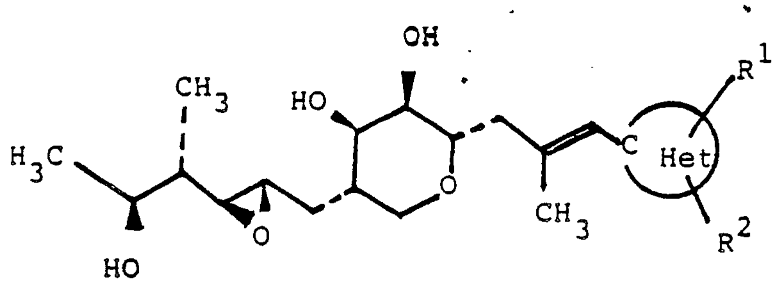

(I-E)

wherein

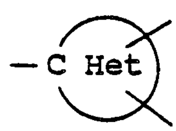 ,

$R^1$ and $R^2$ are as defined in claim 1.

3. A process as claimed in claim 1 or claim 2 wherein $R^3$ is a trialkylsilyl or a dialkylphosphonate group.

4. A process as claimed in any one of claims 1 to 3 wherein $R^3$ is as trimethylsilyl or a diethylphosphonate group.

5. A process as claimed in any one of claims 1 to 4 wherein $M^+$ is an alkali metal cation.

6. A process as claimed in claim 1 or claim 2 wherein cyclising the compound of formula (VI) is effected using a chlorinating agent in the presence of a tertiary amine; a carboxylic anhydride; mixed anhydride; or an acid chloride.

7. A process as claimed in claim 6 wherein the chlorinating agent is selected from the group consisting of triphenylphosphine/carbon tetrachloride, phosphorus oxychloride, phosgene, thionyl chloride and phosphorus pentachloride.

8. A process as claimed in claim 6 wherein cyclising the compound of formula (VI) is effected using

trichloroacetyl chloride.

9. A process as claimed in any one of claims 1 to 8 for producing a compound selected from the group consisting of:
5-[trans-(4-Methoxycarbonyl)cyclohexyl]-2-(1-normon-2-yl)oxazole;
5-[trans-4-(Hydroxymethylcyclohexyl)]-2-normon-2-yl)oxazole; sodium 5-(4-Carboxylatocyclohexyl)-2-(1-normon-2-yl)-oxazole;
5-(cis-4-Hydroxycyclohexyl)-2-(1-normon-2-yl)oxazole;
5-[trans-(4-Hydroxycyclohexyl)]-2-(1-normon-2-yl)oxazole;
5-[trans-4-Cyanocyclohexyl]-2-(1-normon-2-yl)oxazole;
5-[trans-4-(2-Hydroxyprop-2-yl)cyclohexyl]-2-(1-normon-2-yl)oxazole;
5-[trans-4-(1,2-epoxyethyl)cyclohexyl]-2-(1-normon-2-yl)oxazole;
5-[trans-4-(2-Azido-1-hydroxyethyl)cyclohexyl]-2-(1-normon-2-yl)oxazole;
5-[trans-4-(1,3-dioxolan-2-yl)cyclohexyl]-2-(1-normon-2-yl)oxazole;
5-[4-Oxocyclohexyl]-2-(1-normon-2-yl)oxazole;
5-[trans-4-(2-Ethoxy-1-hydroxyethyl)cyclohexyl]-2-(1-normon-2-yl)oxazole;
5-[trans-(4-Ethoxycarbonylmethylene)cyclohexyl]-2-(1-normon-2-yl)oxazole;
5-[trans-(4-Hydroxyethylidene)cyclohexyl]-2-(1-normon-2-yl)oxazole;
5-[cis-(1-(R,S),3(S,R))-3-Hydroxycyclohexyl]-2-(1-normon-2-yl)oxazole; and
5-[trans-(1-(R,S),3-(R,S))-3-Hydroxycyclohexyl]-2-(1-normon-2-yl)oxazole.

10. A process for the preparation of a pharmaceutical or a veterinary composition which comprises admixing a compound of formula (I) as defined in claim 1 together with a pharmaceutically or a veterinarily acceptable carrier or excipient.

11. The use of a compound of formula (I) as defined in claim 1 for the manufacture of a medicament.